(19) Europäisches Patentamt
European Patent Office
Office européen des brevets

(11) Veröffentlichungsnummer: **0 103 323**
**B1**

(12) # EUROPÄISCHE PATENTSCHRIFT

(45) Veröffentlichungstag der Patentschrift: **16.12.87**

(21) Anmeldenummer: **83201188.6**

(22) Anmeldetag: **12.08.83**

(51) Int. Cl.⁴: **C 07 C 127/00,** C 07 C 118/00, C 08 G 18/80, C 08 G 18/70, C 08 G 18/08, C 08 G 18/14

(54) **Verfahren zur Herstellung von stabilisierten Polyisocyanaten, stabilisierte Polyisocyanate retardierter Reaktivität und ihre Verwendung zur Polyurethanherstellung.**

(30) Priorität: **18.08.82 DE 3230757**

(43) Veröffentlichungstag der Anmeldung: **21.03.84 Patentblatt 84/12**

(45) Bekanntmachung des Hinweises auf die Patenterteilung: **16.12.87 Patentblatt 87/51**

(84) Benannte Vertragsstaaten: **AT BE CH DE FR GB IT LI NL SE**

(56) Entgegenhaltungen:
**EP-A-0 062 780**
**US-A-2 858 298**

(73) Patentinhaber: **BAYER AG, Konzernverwaltung RP Patentabteilung, D-5090 Leverkusen 1 Bayerwerk (DE)**

(72) Erfinder: **Grögler, Gerhard, Dr., von Diergardtstrasse 46, D-5090 Leverkusen (DE)**
Erfinder: **Hess, Heinrich, Dr., Auf der Griesse 48, D-5090 Leverkusen (DE)**
Erfinder: **Kopp, Richard, Dr., Wolfskaul 12, D-5000 Koeln 80 (DE)**

**EP 0 103 323 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Gegenstand der Erfindung ist ein Verfahren zur Herstellung von durch Polymerumhüllung stabilisierten, festen Polyisocyanaten mit retardierter Reaktivität durch Umsetzung fester, feinteiliger polyisocyanate mit zwei- und/oder mehrfunktionellen, nieder- und/oder höhermolekularen Verbindungen mit aliphatisch gebundenen Aminogruppen und/oder $-CO.NH.NH_2$-Endgruppen und/oder Hydrazinen in einer Menge von 0,1 bis 25 Äquivalentprozent "Amin" pro Äquivalent NCO in einem flüssigen Medium aus Mono- und/oder Polyolen und/oder Polyaminen und/oder Weichmachern (gegebenenfalls in Gegenwart von unpolaren oder wenig polaren Lösungsmitteln und/oder Wasser) zu einer Suspension von Polyaddukt-umhüllten, stabilisierten Polyisocyanaten in dem flüssigen Medium, wobei die stabilisierten Polyisocyanate, gegebenenfalls durch Filtration, isoliert werden können. Sie lassen sich dann nachträglich in Polyolen und/oder Polyaminen suspendieren.

Erfindungsgegenstand sind auch Polyaddukt-umhüllte Polyisocyanate nach obigem Verfahren in feinteiliger Form, vorzugsweise suspendiert in nieder- und/oder höhermolekularen Polyolen und/oder nieder- und/oder höhermolekularen aromatischen und/oder höhermolekularen aliphatischen Polyaminen, einer 0,1- bis 25-%-igen Umsetzung der NCO-Gruppen und mit einer gegenüber den nicht stabilisierten Polyisocyanaten erhöhten "Aufdickungstemperatur".

Weiterer Erfindungsgegenstand ist die Verwendung der stabilisierten, gegebenenfalls in Polyolen und/oder (bevorzugt) Polyaminen suspendierten, Polyaddukt-umhüllten Polyisocyanate als Reaktionskomponenten, bevorzugt unter Mitverwendung von aromatischen Polyaminen als Kettenverlängerungsmittel bei Polyurethansynthesen, insbesondere zur Verwendung in härtbaren, langzeitlagerstabilen PU-Reaktivsystemen.

Die Einkomponenten-Reaktivmischung wird, gegebenenfalls unter Verwendung von Blei- und/oder Zinn-Katalysatoren, vorzugsweise durch Hitzehärtung, in Polyurethane überführt.

Lagerstabile Einkomponentensysteme auf der Basis von bei Raumtemperatur festen Polyisocyanaten mit einer hohen Lagerstabilität auch gegenüber (flüssigen) aromatischen Polyaminen sind bis jetzt noch nicht beschrieben worden. Auch sind nur wenige Veröffentlichungen zur Oberflächenmodifizierung von bei Raumtemperatur festen Polyisocyanaten bekannt.

Die Anmelderin hat sich unter Bezugnahme auf die ältere nicht vorveröffentlichte europäische Anmeldung Nr. 82 102 272.0, spätere europäische Patentanmeldung Nr. 62 780 mit der Benennung der Vertragsstaaten AT, BE, DE, FR, GB, IT, NL und SE freiwillig für die vorstehend genannten Vertragsstaaten eingeschränkt und gesonderte Patentansprüche für diese Vertragsstaaten vorgelegt.

Für die in der älteren Patentanmeldung 82 102 272.0 nicht benannten Vertragsstaaten CH und LI wurden die Patentansprüche nicht eingeschränkt.

In der DE-OS-25 57 407 wird ein Verfahren beschrieben, bei dem die Lösung eines Polyisocyanates in einem niedrig siedenden Lösungsmittel in einen Reaktionsraum mit einem gasförmigen Di- und/oder Polyamin gedüst wird, wobei durch die Reaktion des Polyisocyanates mit dem Amin und durch die Verdampfung des Lösungsmittels Polyurethanpolyharnstoff-Hohlperlen erhalten werden, die vorzugsweise als Füllstoffe eingesetzt werden. Die Reaktion wird so geführt, daß die NCO-Gruppen mit dem Amin und gegebenenfalls weiteren zugesetzten NCO-reaktiven Komponenten möglichst vollständig abreagieren. Es wird nicht beschrieben, die Reaktion so zu führen, daß die Polyharnstoffhülle nur einen geringen Teil des Feststoffes ausmacht und im Inneren noch nicht abreagierte NCO-Gruppen für weitere Reaktionen im Sinne eines PU-Einkomponentensystems zur Verfügung stehen.

US-PS-3 409 461 beschreibt die Umhüllung von Polyisocyanaten mit einer Schutzsubstanz, vorzugsweise einem Polymeren, wodurch die Polyisocyanatteilchen an der Oberfläche desaktiviert sind. Hierzu wird das Isocyanat in eine Lösung des Polymers in einem niedrig siedenden, das Isocyanat praktisch nicht lösenden Lösungsmittel dispergiert und die Dispersion sprühgetrocknet. Vorzugsweise wird fein gemahlenes (1 bis 10 µm Teilchengröße) Naphthylen-1,5-diisocyanat mit einer 1- bis 2,5-%-igen Lösung von Polystyrol, Polyvinylbutylether, chloriertem Kautschuk und ähnlichen in Tetrachlormethan sprühgetrocknet. Es werden freifließende Pulver mit einer Teilchengröße von ca. 1 bis 50 µm erhalten. Diese werden vorzugsweise zur Verbesserung der Haftung von Polyesterprodukten (Geweben, Fasern, Folien) an Kautschukelastomeren eingesetzt.

Bei diesem Verfahren zur Umhüllung von Isocyanaten mittels zugesetzter Polymerer aus Lösung müssen erhebliche Mengen von teilweise giftigen Lösungsmitteln eingesetzt werden, z. B. 4 kg Tetrachlormethan für 50 g Naphthylen-1,5-diisocyanat, die dann in einem energetisch aufwendigen Verfahren wieder entfernt werden müssen. Als besonderer Nachteil des Verfahrens muß der erhebliche Anteil des Hüllenmaterials an dem Gesamtgewicht des umhüllten Isocyanats angesehen werden. Dieser beträgt 9 bis 91 Gew.-%, bei den Beispielen liegt er im allgemeinen in der Größenordnung von 50 Gew.-%. Dadurch würde bei der Herstellung von qualitativ hochwertigen Polyurethanen ein zu hoher Anteil an störender Fremdsubstanz eingebracht werden.

Die US-PS-3 551 346 beschreibt eine Verkapselung von flüssigen Diisocyanaten durch Grenzflächenreaktionen von im Diisocyanat gelösten $CH_3$-Si-$(OCH_3)_3$ mit in der Wasserphase gelöstem $(CH_3)_3$.Si-O-Na unter Filmbildung. Diese, durch Silikonpolymerbildung vorverkapselten Tröpfchen werden anschließend durch Coacervation (z. B. mit entgegengesetzt geladenen Polymeren nach US-PS-2 800 457) endgültig "verkapselt".

In der DE-OS-1 570 548 wird ein längere Zeit lagerfähiges Einkomponentensystem beschrieben, welches aus

einem Gemisch von 1 Mol eines Polyesters, Polyethers oder Polythioethers, mindestens 1,5 Mol eines Uretdiongruppen enthaltenden festen Isocyanates mit einem Schmelzpunkt ≧ 100°C und mindestens 0,3 Mol eines festen Kettenverlängerungsmittels mit OH- und/oder $NH_2$-Gruppen mit einem Schmelzpunkt ≧ 80°C besteht. Dabei müssen mindestens 80 % der festen Gemischbestandteile mit einer Teilchengröße ≧ 38 µm vorliegen. Die Lagerbeständigkeit beträgt bei Raumtemperatur einige Tage bis einige Wochen, bei 50°C nur einige Stunden. Ein Nachteil des Verfahrens liegt darin, daß von drei Reaktionspartnern mindestens zwei in fester Form vorliegen müssen, um die nötige Lagerstabilität zu gewährleisten. Das führt dazu, daß im allgemeinen sehr hochviskose Gemische erhalten werden, deren Viskosität langsam weiter ansteigt, weil keine der Verbindungen in ihrer Reaktivität modifiziert wurde.

Die aus der ständigen Viskositätszunahme ersichtliche Reaktion an der Oberfläche der festen Teilchen erfolgt unkontrolliert und praktisch zu langsam und ergibt keine ausreichende Retardierung der Reaktivität der Polyisocyanate unter Selbststabilisierung des Systems.

Auch dürften beim Aushärten der Mischung wegen der hohen Anteile an festen Bestandteilen Inhomogenitäten im ausgeheizten Produkt zu erwarten sein. Auch ist die Verarbeitung der hochviskosen bis festen Gemische schwieriger, weil diese im Gegensatz zu flüssigen Gemischen zuerst einmal durch Temperaturerhöhung oder Druckanwendung in einen verformbaren Zustand gebracht werden müssen.

Wie in Vergleichsversuchen gezeigt wird, tritt beim Verweilen von hochschmelzenden Polyisocyanaten in Gemischen aus höher- und niedermolekularen Polyolen eine ständige und relativ schnelle Weiterreaktion unter starker Viskositätserhöhung ein, d.h. die Oberflächenreaktion an den festen Polyisocyanatteilchen bildet keine zur Retardierung ausreichende, d.h. hinreichend stabilisierende Hülle um das Polyisocyanat.

In der GB-PS-1 134 285 wird ein Verfahren zur Herstellung von dimeren Diisocyanaten in einem wäßrigen Reaktionsmedium beschrieben. Derartig hergestellte Dimere in wäßriger Suspension reagieren nach den Angaben in dieser Patentschrift mit polyfunktionellen Verbindungen mit aktiven Wasserstoffatomen nicht bei Raumtemperatur, können jedoch thermisch zu Polyurethanen vernetzt werden. Möglicherweise wird die Stabilität durch eine eingetretene, langsame Oberflächenreaktion von Isocyanatgruppen mit Wasser hervorgerufen. Eine Vernetzung wird anschließend bei hohen Temperaturen, z. B. 150 bis 200°C, durch Spaltung des Uretdionrings herbeigeführt.

Demgegenüber sollen erfindungsgemäß durch Reaktion mit den "Amin-Stabilisatoren" Poly-Aminen, Hydrazin(en) oder Hydrazid-Verbindungen mit $-CO.NH.NH_2$-Endgruppen gebildete, mittels Umhüllung mit Polyadditionsprodukten ("Polyaddukten") stabilisierte, feste Polyisocyanate mit einer retardierten Reaktivität hergestellt werden, wobei die Umhüllung im wesentlichen aus Polyadditionsprodukten in dünner Schicht und in nur untergeordneter Abreaktion der NCO-Gruppen an der Oberfläche der festen Isocyanatteilchen gebildet wird. Durch die Stabilisierungsreaktion mittels Polyadduktumhüllung werden Polyisocyanatteilchen erhalten, welche in den Einkomponentensystemen erst oberhalb einer noch näher zu definierenden "Aufdickungstemperatur", z. B. durch Aufsprengen der Polyadduktumhüllung, durch Zerstörung der Umhüllung durch Scherkräfte, Diffusionserleichterung der Polyisocyanate durch die Polyadduktschicht oder gar Auflösung der Polyadduktschicht durch polare Lösungsmittel, als Polyisocyanate in Reaktion treten.

Die "Polyaddukt"-Hülle auf den festen Polyisocyanatteilchen ist je nach der Art "Amin-Stabilisatoren" etwas unterschiedlich. Mit Polyaminen bilden sich Polyharnstoff-Umhüllungen, mit Hydrazin(en) bilden sich Polyhydrazodicarbonamid-Umhüllungen, mit Hydrazidverbindungen mit $CO-NH-NH_2$-Endgruppen, wie Dihydraziden, Bis-semicarbaziden, Bis-carbazinestern, Semicarbazidhydraziden oder Aminohydraziden, bilden sich noch komplexere Polyaddukte mit einer Vielzahl von unterschiedlich angeordneten -NH- und -CO-Gruppen in der Polymerkette.

Die stabilisierten Polyisocyanate werden vorzugsweise direkt in Suspension in Polyolen und/oder Polyaminen, vorzugsweise in höhermolekularen Polyolen, gegebenenfalls unter Zusatz von niedermolekularen Polyolen oder aromatischen Polyaminen als Kettenverlängerungsmitteln, bevorzugt aber in höhermolekularen Polyaminen mit aromatischen und/oder aliphatischen Aminogruppen, gegebenenfalls unter Zusatz von niedermolekularen aromatischen Polyaminen und/oder niedermolekularen Polyolen als Kettenverlängerungsmittel hergestellt und liegen somit in einer direkt weiterverwendbaren Suspensionsform für Einkomponentenpolyurethane vor.

Neben dieser in situ-Herstellung in den Polyolen oder aromatischen Polyaminoverbindungen können jedoch die stabilisierten Polyisocyanate auch durch Umsetzung in Monoalkoholen, Lösungsmitteln, Weichmachern oder Wasser hergestellt und die gebildeten stabilisierten, umhüllten Polyisocyanate, z. B. durch Filtration, abgetrennt, isoliert und erst anschließend in (höhermolekularen) Polyolen und/oder Polyaminen suspendiert werden. Die stabilisierten Polyisocyanate enthalten noch mindestens 75 %, vorzugsweise mehr als 92 % und besonders bevorzugt noch mehr als 97 %; max. jedoch 99,9 %, bevorzugt max. 99,7 %, der Isocyanatgruppen der unmodifizierten Isocyanate.

Es wurde gefunden, daß die Polyadduktumhüllung mit den "Amin-Stabilisatoren" (zwei- und/oder mehrfunktionellen, nieder- und/oder höhermolekularen aliphatischen Polyaminen, Poly-hydrazidverbindungen mit $CO-NH-NH_2$-End-gruppen und/oder Hydrazin(en)) in sehr geringen Äquivalentprozenten erzielt werden kann, wobei jedoch zur Erzielung nicht-poröser, "elastischer" Polyadduktumhüllungen vorzugsweise in Gegenwart von Mono- und/oder Polyolen und/oder (höhermolekularen) Polyaminen und/oder Weichmachern und/oder Wasser gearbeitet wird, wenn die Amin-Stabilisatoren nicht selbst eine gewisse elastifizierende Wirkung aufweisen, wie z. B. höhermolekulare Polyetherpolyamine mit aliphatischen Amino- oder $-CO-NH-NH_2$-Endgruppen.

Die stabilisierten Polyisocyanate besitzen in den suspendierenden Polyolen und/oder auch höhermolekularen Polyaminen eine außerordentlich gute Lagerstabilität, auch bei erhöhten Temperaturen, selbst in Gegenwart hochwirksamer Polyurethankatalysatoren. Bei Verwendung der erfindungsgemäßen, umhüllten Polyisocyanate sind auch aromatische Diamine als Kettenverlängerer enthaltende PU-Reaktivmischungen ausgezeichnet lagerstabil oder zeigen in Gießsystemen eine stark verlängerte Topfzeit, selbst wenn es sich um lösliche (flüssige) aromatische Polyamine handelt.

Die Aushärtung von Einkomponenten-Reaktivmischungen kann durch einfaches Erhitzen erfolgen, wobei oberhalb einer bestimmten Temperatur ("Aufdickungstemperatur") eine schnelle Polyadditionsreaktion erfolgt. Durch einfache Variation der Reaktionsbedingungen, z. B. Temperatur bei der Umhüllungsreaktion, Auswahl des Reaktionsmediums oder der Art und Menge der zur Stabilisierung herangezogenen "Amin-Stabilisatoren", läßt sich diese "Aufdickungstemperatur" variieren und eine hohe Lagerstabilität auch bei höheren Lagertemperaturen einstellen.

Die mit den stabilisierten Polyisocyanaten gebildeten langzeitlagerbeständigen, gut fließfähigen, gegebenenfalls leicht aufschmelzbaren, heterogenen Einkomponentensysteme lassen sich aber auch durch Zugabe von polaren Lösungsmitteln (z. B. Dimethylformamid) zur Aushärtung bringen, in manchen Fällen genügt auch schon eine Einwirkung von starken Scherkräften.

Bei Hitzehärtung können die erfindungsgemäßen Einkomponenten-PU-Reaktivsysteme bereits in einem relativ niederen Temperaturbereich (oberhalb der Aufdickungstemperatur, bevorzugt $\geqslant$ 55°C, besonders bevorzugt 100 bis 135°C) zur Umsetzung gebracht werden, wobei je nach Wahl der Reaktionspartner hochwertige Polyurethankunststoffe hergestellt werden können.

Die erfindungsgemäßen Befunde sind überraschend, waren der veröffentlichten Literatur nicht zu entnehmen und vom Fachmann nicht vorauszusehen. Insbesondere die aus den erfindungsgemäßen Suspensionen auf Basis von höhermolekularen Polyaminen und/oder aromatischen niedermolekularen Polyaminen als Kettenverlängerungsmitteln hergestellten Polyurethane (eigentlich Polyurethan-"Harnstoffe") zeigen verbesserte Eigenschaften, z. B. höhere Moduli und höhere Erweichungsbereiche, als entsprechende, auf Basis von Polyolen hergestellte Polyurethane.

Gegenstand der Erfindung sind somit Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb 30°C, vorzugsweise oberhalb 80°C,

in feinteiliger Form mit einer Teilchengröße von 0,5 bis 20 µm, vorzugsweise 1 bis 50 µm.

mit zwei- oder mehrfunktionellen, vorzugsweise zwei- bis dreifunktionellen, besonders bevorzugt zweifunktionellen, nieder- und/oder höhermolekularen Verbindungen mit aliphatisch gebundenen, primären und/oder sekundären Aminogruppen und/oder -CO-NH-$NH_2$-Endgruppen und/oder Hydrazin(en) und einem Molekulargewicht von 32 bis 6000, vorzugsweise von 60 bis 3000, oder ihren Mischungen, als "Amin-Stabilisatoren"

in einer Menge von 0,1 bis 25 Äquivalentprozent Amin pro NCO, vorzugsweise 0,1 bis 8 Äquivalentprozent, besonders bevorzugt 0,3 bis 3 Äquivalentprozent,

in einem flüssigen Medium

aus nieder- und/oder höhermolekularen Mono- und/oder Polyolen und/oder nieder- und/oder höhermolekularen aromatischen Polyaminen und/oder höhermolekularen aliphatischen Polyaminen und/oder Weichmachern und gegebenenfalls Wasser, vorzugsweise aus höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen, besonders Di- und Triolen, und/oder aromatischen Polyaminen, bevorzugt Diaminen, mit Molekulargewichten von 60 bis 399,

gegebenenfalls in Gegenwart von unpolaren oder wenig polaren Lösungsmitteln, vorzugsweise auf der Basis von aliphatischen, cycloaliphatischen oder aromatischen Kohlenwasserstoffen, Halogenkohlenwasserstoffen, Ethern, Ketonen oder Estern,

bei Temperaturen unterhalb der Schmelztemperatur der Polyisocyanate, bevorzugt bis etwa 70°C, besonders bevorzugt 0 bis 50°C,

zu einer Suspension von Polyaddukt-umhüllten, stabilisierten Polyisocyanaten in dem flüssigen Medium umsetzt und gegebenenfalls die stabilisierten Polyisocyanate aus den Monoalkoholen, Weichmachern, Wasser und/oder Lösungsmitteln isoliert und in Polyolen und/oder Polyaminen suspendiert.

Gegenstand der Erfindung ist weiterhin ein Verfahren zur Herstellung von durch Polyadduktumhüllung stabilisierten Polyisocyanaten nach der geschilderten Verfahrensweise, dadurch gekennzeichnet, daß man die festen Polyisocyanate mit Hydrazin, Alkylhydrazinen, N,N'-Dialkyl-hydrazinen mit $C_1$-$C_6$-Alkylgruppen und/oder mit zwei- oder mehrfunktionellen, nieder- und/oder höhermolekularen Verbindungen mit -CO.NH.$NH_2$-Endgruppen und einem Molekulargewicht von 32 bis 6000, als "Amin-Stabilisatoren"

in einer Menge von 0,1 bis 25 Äquivalentprozent, vorzugsweise 0,1 bis 8 Äquivalentprozent, einer Hydrazin- oder Hydrazid-Endgruppe pro NCO-Gruppe

in einem flüssigen Medium wie beim vorstehend geschilderten Verfahren umsetzt.

Gegenstand der Erfindung sind insbesondere Verfahren zur Herstellung von durch Polymerumhüllung stabilisierten Polyisocyanaten nach den bereits beschriebenen Verfahrensweisen, dadurch gekennzeichnet, daß man die festen Polyisocyanate mit den "Amin-Stabilisatoren" in den angegebenen Mengen in einem flüssigen Medium aus höhermolekularen aromatischen und/oder aliphatischen Polyaminen mit Molekulargewichten von 400 bis 6000, und gegebenenfalls niedermolekularen aromatischen Polyaminen, sowie

gegebenenfalls nieder- und/oder höhermolekularen Polyolen und/oder gegebenenfalls Weichmachern oder Wasser, gegebenenfalls in Gegenwart von unpolaren oder wenig polaren Lösungsmitteln, bei Temperaturen unterhalb der Schmelztemperatur der Polyisocyanate

zu einer Suspension von Polyaddukt-umhüllten, stabilisierten Polyisocyanaten in den höhermolekularen Polyaminen umsetzt und gegebenenfalls (sofern die Stabilisierungsreaktion nur in Weichmachern, Wasser und/oder wenigpolaren Lösungsmitteln erfolgt), die stabilisierten Polyisocyanate aus den Weichmachern, Wasser und/oder wenig polaren Lösungsmitteln isoliert und anschließend in den höhermolekularen Polyaminen suspendiert.

Bevorzugt ist das Verfahren, die Umsetzung der Polyisocyanate mit den "Amin-Stabilisatoren" direkt in den höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen als Kettenverlängerungsmittel mit Molekulargewichten von 60 bis 399, unter Bildung der Suspensionen, durchzuführen, wobei vorzugsweise die Komponenten in solchen Mengen umgesetzt werden, wie dies einer Rezeptur der Einkomponenten-PU-Reaktivsysteme entspricht.

Ein weiterer Erfindungsgegenstand sind Polyaddukt-umhüllte, stabilisierte, feste, feinteilige Polyisocyanate retardierter Reaktivität, hergestellt nach den erfindungsgemäßen Verfahren, lagerstabil suspendiert in nieder- und/oder höhermolekularen Polyolen und/oder nieder- und/oder höhermolekularen aromatischen Polyaminen und/oder höhermolekularen aliphatischen Polyaminen, mit einem NCO-Restgehalt von mindestens 75 %, vorzugsweise 92 %, besonders bevorzugt 97 %, und weniger als 99,9 %, vorzugsweise weniger als 99,7 %, der ursprünglich vorhandenen NCO-Gruppen der festen Ausgangspolyisocyanate

und einer Aufdickungstemperatur der Suspension von oberhalb 55°C bevorzugt 80 bis 140°C und besonders bevorzugt 100 bis 135°C.

Bevorzugt ist die Suspension in einem Medium aus höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen mit Molekulargewichten von 60 bis 399.

Besonders bevorzugt sind die Polyadduktumhüllten Polyisocyanate, suspendiert in höhermolekularen aromatischen und/oder aliphatischen Polyaminen mit Molekulargewichten von 400 bis 6000, vorzugsweise 400 bis 3000, gegebenenfalls unter Zusatz von niedermolekularen Polyolen (vorzugsweise Di- und Triolen) und/oder aromatischen Polyaminen (vorzugsweise Diaminen), des Molekulargewichts 62 bis 399 und gegebenenfalls untergeordneten Mengen, bezogen auf höhermolekulare Polyamine, an höhermolekularen Polyolen.

Weiterhin sind bevorzugt Polyaddukt-umhüllte, stabilisierte Polyisocyanate besonders unter Verwendung von aliphatischen Polyaminen als Amin-Stabilisatoren, suspendiert in höhermolekularen Polyolen der Molekulargewichte 400 bis 6000 unter Zusatz von niedermolekularen aromatischen Diaminen und gegebenenfalls niedermolekularen Polyolen mit Molekulargewichten bis 399.

Ein weiterer Erfindungsgegenstand ist auch die Verwendung von nach den beschriebenen Verfahren erhaltenen, Polyaddukt-umhüllten, stabilisierten festen Polyisocyanaten retardierter Reaktivität, suspendiert in nieder- und/oder höhermolekularen Polyol- und/oder nieder- und/oder höhermolekularen aromatischen Polyaminoverbindungen und/oder höhermolekularen aliphatischen Polyaminoverbindungen entsprechend den vorgenannten Herstellungsverfahren und den genannten Zusammensetzungen der Suspensionen,

als Polyisocyanatkomponente A) und höhermolekulare Polyol- und/oder Polyaminoverbindungen B) und gegebenenfalls niedermolekulare Kettenverlängerungsmittel C)

zur Herstellung von Polyurethanen, vorzugsweise zur Herstellung von Polyurethanen über lagerstabile Einkomponentensysteme, auf der Basis von

A) Polyisocyanaten
B) höhermolekularen Polyhydroxyl- und/oder Polyaminoverbindungen,
C) gegebenenfalls niedermolekularen Kettenverlängerungsmitteln,
D) gegebenenfalls Polyurethankatalysatoren und
E) gegebenenfalls üblichen Hilfs- und Zusatzstoffen.

Die retardiert reagierenden Polyisocyanate lassen dabei sowohl Polyurethanherstellungen zu, wo die Reaktionszeit (Topfzeit) verlängert wird (z. B. bei Gießsystemen); sie gestatten jedoch auch, bei niedrigen Temperaturen langzeitlagerstabile Einkomponentensysteme aufzubauen, welche erst z. B. durch Temperaturerhöhung oder polare Lösungsmittel zur Reaktion gebracht werden.

Erfindungsgegenstand ist besonders die Verwendung von Polyaddukt-umhüllten Polyisocyanaten nach den vorbeschriebenen Verfahren bzw. Suspensionszusammensetzungen zur Herstellung von Polyurethanen aus

A) Polyisocyanaten
B) höhermolekularen Polyhydroxyl- und/oder Polyaminoverbindungen,
C) gegebenenfalls niedermolekularen Kettenverlängerungsmitteln,
D) gegebenenfalls Polyurethankatalysatoren und
E) gegebenenfalls üblichen Hilfs- und Zusatzstoffen,

dadurch gekennzeichnet, daß man die nach den beschriebenen Verfahren erhaltenen, Polyaddukt-umhüllten, suspendierten Polyisocyanate nach dem beschriebenen Verfahren als Polyisocyanatkomponente A) in höhermolekularen Komponenten B) und gegebenenfalls Kettenverlängerungsmitteln C)

gegebenenfalls unter Zusatz von weiteren, nicht-stabilisierten Polyisocyanaten A), höhermolekularen Verbindungen B) und Kettenverlängerungsmitteln C), gegebenenfalls unter Zusatz von tert.-Amin- und/oder

Metallkatalysatoren D) und Hilfsstoffen E)

zur Herstellung von fließfähigen oder leicht aufschmelzenden PU-Reaktivsystemen mit einer Aufdickungstemperatur von ≧ 55°C einsetzt und diesen durch Hitze, Scherkräfte und/oder polare Lösungsmittel zu massiven oder zelligen Polyurethanen aushärtet.

Eine bevorzugte Durchführungsform betrifft die Verwendung von erfindungsgemäß mit Hydrazin, Alkylhydrazinen, N,N'-Dialkylhydrazinen (mit $C_1$-$C_6$-Alkylgruppen) und/oder mit zwei- oder mehrfunktionellen, nieder- und/oder höherfunktionellen Verbindungen mit $-CO-NH-NH_2$-Endgruppen und einem Molekulargewicht von 32 bis 6000 oder ihren Mischungen, als "Amin-Stabilisatoren" in einer Menge von 0,1 bis 25 Äquivalentprozent an Aminogruppen, bezogen auf die NCO-Gruppen,

in Suspension in höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen des Molekulargewichts 60 bis 399,

vorliegenden, stabilisierten Polyisocyanate zur Herstellung von Polyurethanen unter den genannten Bedingungen.

Eine besonders bevorzugte Verwendungsform zur Polyurethanherstellung in der Verwendung der durch die angegebenen "Amin-Stabilisatoren" stabilisierten Polyisocyanate, welche in höhermolekularen aromatischen und/oder aliphatischen Polyaminen, gegebenenfalls unter Zusatz von niedermolekularen aromatischen Polyaminen, vorzugsweise Diaminen, und gegebenenfalls niedermolekularen Polyolen mit Molekulargewichten von 62 bis 399, suspendiert sind. Hierbei sind keine Katalysatoren zur Durchhärtung zum Polyurethan(harnstoff) erforderlich und die Temperaturen, bzw. die Aushärtungszeiten, liegen niedriger als bei Polyolsystemen; ebenso werden sehr günstige Elastomereigenschaften erhalten.

Eine weitere bevorzugte Verwendungsform zur Polyurethanherstellung liegt in der Verwendung von mit "Amin-Stabilisatoren", insbesondere von mit zwei- oder mehrfunktionellen, vorzugsweise zwei- bis dreifunktionellen, nieder- und/oder höhermolekularen Verbindungen mit aliphatisch gebundenen primären und/oder sekundären Aminogruppen und Molekulargewichten von 60 bis 3000 stabilisierten Polyisocyanaten nach obigen Verfahren, suspendiert in höhermolekularen Polyolen B) und unter Mitverwendung von aromatischen Polyaminen als Kettenverlängerungsmittel C), wobei diese bereits bei der Herstellung der Suspension mitverwendet sind oder der Polyurethan-bildenden Reaktivmischung erst später als Kettenverlängerungsmittel zugesetzt werden. Eine derartige Mischung kann auch gegebenenfalls niedermolekulare Polyole als Kettenverlängerungsmittel zusätzlich enthalten. Dabei werden mindestens 10 Mol-%, bevorzugt mindestens 50 Mol-%, der Kettenverlängerungsmittel C) als aromatische Polyamine (bevorzugt Diamine) neben gegebenenfalls niedermolekularen Polyolen eingesetzt. Auch diese Systeme zeichnen sich durch ein günstiges Härtungsverhalten und gegenüber nur Polyol-Kettenverlängerungsmittel enthaltenden Systemen durch bessere Elastomereigenschaften, z. B. Festigkeiten, mechanische Eigenschaften und Erweichungsbereiche, aus.

Wegen der älteren europäischen Anmeldung 82 102 272.0 wurden die für CH und LI geltenden Patentansprüche für die Vertragsstaaten AT, BE, DE, FR, GB, IT, NL und SE auf die gegenüber der obenangegebenen Anmeldung neuen Gegenstände eingegrenzt.

In diesem Anspruchssatz (Serie a) wurden die nach Art. 54/3 neuheitsschädlichen Anspruchsteile herausgenommen. Dies betrifft die Verfahren zur Herstellung und Dispergierung der Polyisocyanate in Polyolen, wobei der "Amin-Stabilisator" entweder schon im Polyol enthalten ist oder nachträglich zugeführt wird; er betrifft auch desaktivierte, stabilisierte Polyisocyanate als Stoffmischung in Polyolen.

Bevorzugt werden die Polyaddukt-umhüllten, stabilisierten Polyisocyanate retardierter Aktivität als alleinige Polyisocyanate A) beim Aufbau von Polyurethanen eingesetzt. Es ist jedoch auch möglich, Kombinationen aus den erfindungsgemäß stabilisierten Polyisocyanaten und nichtstabilisierten Polyisocyanaten, z. B. Toluylendiisocyanaten, Diphenylmethandiisocyanaten, Naphthylen-1,5-diisocyanat oder dimerem Toluylendiisocyanat, zu verwenden. Bei diesen Kombinationen werden jedoch bevorzugt mindestens 50 Äquivalentprozent erfindungsgemäß stabilisierter Polyisocyanate eingesetzt.

Als nicht-stabilisierte Polyisocyanate sind alle Polyisocyanate geeignet, wie sie in der DE-OS-2 920 501 (Seiten 12-16) aufgeführt sind.

Verwendet man Suspensionen der stabilisierten Polyisocyanate in den höhermolekularen Polyhydroxy- und/oder höhermolekularen Polyamino-Verbindungen B), so können die Mengen an B) bevorzugt so gewählt sein, daß die Zusammensetzung bereits der gewünschten, Polyurethan-bildenden Komponentenmischung entspricht ("Einkomponenten-Reaktivmischung"). Es können jedoch bei abweichender Zusammensetzung zur Polyurethanbildung gleiche oder verschiedene, weitere höhermolekulare Komponenten B) zur Polyurethanherstellung mitverwendet werden.

Ebenso können gegebenenfalls auch bereits die Polyol- oder aromatischen Polyamin-Kettenverlängerungsmittel C) der Stabilisierungsreaktion in für Einkomponentensysteme geeigneten Mengen vorliegen, andernfalls kann C) als weitere Kettenverlängerungskomponente(n) zugefügt werden.

Eine Ausführungsform besteht beispielsweise darin, daß man die Suspension stabilisierter Polyisocyanate in den höhermolekularen Verbindungen B) und gegebenenfalls den niedermolekularen Verbindungen C) so herstellt, daß ein Überschuß an Hydroxyl- und/oder Aminogruppen in der Suspension vorliegt und diese dann mit einem üblichen (nicht umhüllten) Polyisocyanat so umsetzt, daß alle Hydroxyl- und/oder Aminogruppen mit etwa äquivalenten Mengen in Form von Isocyanatgruppen aus stabilisierten und nicht-stabilisierten Polyisocyanaten reagieren können.

Enthält die Suspension stabilisierter Polyisocyanate in den höhermolekularen Verbindungen B) und gegebenenfalls den Kettenverlängerungsmitteln C) einen Überschuß an Isocyanatgruppen (aus den erfindungsgemäßen umhüllten Polyisocyanaten), so können der Reaktionsmischung weitere höhermolekulare (B) bzw. niedermolekulare Verbindungen (C) zugemischt und zum Polyurethan umgesetzt werden.

Dies kann in den beiden geschilderten Fällen etwa dadurch erreicht werden, daß man die Polyisocyanatsuspension einerseits und die anderen Komponenten in getrennten Linien dosiert, vermischt und anschließend, z. B. in einer Form, thermisch aushärtet.

Eine Vorentscheidung darüber, ob eine bestimmte erfindungsgemäße Kombination eines festen Diisocyanats mit einem aliphatischen "Amin-Stabilisator" zur Herstellung lagerstabiler Einkomponenten-PU-Reaktivsysteme grundsätzlich geeignet ist, liefert der folgende Test unter Festlegung der "Aufdickungstemperatur":

"DETA-Test" für die Charakterisierung der umhüllten Polyisocyanate ("Aufdickungstemperatur"):

1 Mol des mit dem "Aminstabilisator" modifizierten, festen Diisocyanats werden in 1000 g eines linearen Polyoxypropylenetherdiols des Molekulargewichts 2000 suspendiert. Nach Zusatz von 0,5 Mol 2,4-/2,6- (65/35) Diamino-3,5-diethyltoluol-Isomerengemisch (DETA) wird die Einkomponenten-Reaktivmischungssuspension mit einer Geschwindigkeit von etwa 10°C/min aufgeheizt. Die Temperatur, bei der die Mischung rasch pastenartige Konsistenz unter Verfestigung annimmt, wird als "Aufdickungstemperatur" bezeichnet.

Eine Aufdickungstemperatur von weniger als 55°C zeigt erfahrungsgemäß an, daß eine betreffende Kombination aus festem Polyisocyanat und Aminstabilisator in der vorliegenden Konzentration für eine Verwendung bei langzeitlagerstabilen Einkomponenten-PU-Reaktivsystemen wenig geeignet ist (jedoch bei z. B. Gießsystemen evtl. noch zu einer erwünschten Verlängerung der Gießzeit führen kann).

Durch Aminstabilisierung erhaltene, Polyharnstoff-umhüllte Polyisocyanate retardierter Reaktivität, welche im DETA-Test unter Verwendung von höhermolekularen Polyetherpolyolen plus aromatischem Kettenverlängerungsmittel DETA bereits Aufdickungstemperaturen unter 55°C zeigen, sind für die erfindungsgemäßen Suspensionen in höhermolekularen Polyaminen erfindungsgemäßen wenig geeignet.

A)

Als Ausgangskomponenten für die erfindungsgemäß stabilisierten, festen Polyisocyanate sind

1.

alle Di- oder Polyisocyanate oder deren beliebige Gemische geeignet, sofern sie einen Schmelzpunkt oberhalb 30°C, vorzugsweise oberhalb 40°C, besonders bevorzugt oberhalb 80°C, aufweisen.

Es können dies aliphatische, cycloaliphatische, araliphatische, bevorzugt aber aromatische und heterocyclische Polyisocyanate sein, ferner Polyphenyl-polymethylen-polyisocyanate, erhalten durch Anilin-Formaldehyd-Kondensation und anschließende Phosgenierung nach den britischen Patentschriften 874 430 und 848 671, weiterhin perchlorierte Arylpolyisocyanate, Carbodiimidgruppen aufweisende Polyisocyanate, Allophanatgruppen aufweisende Polyisocyanate, Isocyanuratgruppen aufweisende Polyisocyanate, Urethan- oder Harnstoffgruppen aufweisende Polyisocyanate, acylierte Harnstoffgruppen aufweisende Polyisocyanate, Biuretgruppen aufweisende Polyisocyanate, durch Telomerisationsreaktion hergestellte Polyisocyanate, Estergruppen aufweisende Polyisocyanate, ferner bevorzugt Uretdiongruppen aufweisende Diisocyanate und Harnstoffgruppen aufweisende Diisocyanate. Als Beispiele für die einsetzbaren Polyisocyanate seien genannt:

| | |
|---|---|
| p-Xylylendiisocyanat | Fp: 45-46°C |
| 1,5-Diisocyanatomethylnaphthalin | 88-89°C |
| 1,3-Phenylendiisocyanat | 51°C |
| 1,4-Phenylendiisocyanat | 94-96°C |
| 1-Methylbenzol-2,5-diisocyanat | 39°C |
| 1,3-Dimethylbenzol-4,6-diisocyanat | 70-71°C |
| 1,4-Dimethylbenzol-2,5-diisocyanat | 76°C |
| 1-Nitrobenzol-2,5-diisocyanat | 59-61°C |
| 1,4-Dichlorbenzol-2,5-diisocyanat | 134-137°C |
| 1-Methoxybenzol-2,4-diisocyanat | 75°C |
| 1-Methoxybenzol-2,5-diisocyanat | 89°C |
| 1,3-Dimethoxybenzol-4,6-diisocyanat | 125°C |
| Azobenzol-4,4'-diisocyanat | 158-161°C |
| Diphenylether-4,4'-diisocyanat | 66-68°C |
| Diphenylmethan-4,4'-diisocyanat | 42°C |
| Diphenyl-dimethylmethan-4,4'-diisocyanat | 92°C |
| Naphthalin-1,5-diisocyanat | 130-132°C |
| 3,3'-Dimethylbiphenyl-4,4'-diisocyanat | 68-69°C |
| Diphenyldisulfid-4,4'-diisocyanat | 58-60°C |
| Diphenylsulfon-4,4'-diisocyanat | 154°C |
| 1-Methylbenzol-2,4,6-triisocyanat | 75°C |
| 1,3,5-Trimethylbenzol-2,4,6-triisocyanat | 93°C |
| Triphenylmethan-4,4'4''-triisocyanat | 89-90°C |
| 4,4'-Diisocyanato-(1,2)-diphenyl-ethan | 88-90°C |
| dimeres 1-Methyl-2,4-phenylendiisocyanat | 156°C |
| dimeres-1-Isopropyl-2,4-phenylendiisocyanat | 125°C |
| dimeres 1-Chlor-2,4-phenylendiisocyanat | 177°C |
| dimeres 2,4'-Diisocyanato-diphenylsulfid | 178-180°C |
| dimeres Diphenylmethan-4,4'-diisocyanat | |
| 3,3'-Diisocyanato-4,4'-dimethyl-N,N'-diphenylharnstoff | |
| N,N'-Bis[4(4-Isocyanatophenylmethyl)phenyl]harnstoff | |
| N,N'-Bis[4(2-Isocyanatophenylmethyl)phenyl]harnstoff. | |

Erfindungsgemäß werden besonders bevorzugt 1,5-Naphthalin-diisocyanat, 3,3'-Diisocyanato-4,4'-dimethyl-N,N'-diphenylharnstoff, dimeres 1-Methyl-2,4-diisocyanatobenzol, dimeres 4,4'-Diisocyanato-diphenylmethan und 3,3'-Dimethyl-4,4'-diisocyanato-diphenyl eingesetzt. Die dimeren Diisocyanate können auch durch "in-situ"-Dimerisierung, in z. B. Weichmachern, Lösungsmitteln, Wasser oder Polyolen feinverteilt hergestellt und gegebenenfalls in dieser Form der Aminstabilisierungsreaktion unterworfen werden.

Als "Amin-Stabilisatoren" für die genannten Polyisocyanate werden z. B. zwei- oder mehrfunktionelle, nieder- oder höhermolekulare Verbindungen mit aliphatisch gebundenen, primären und/oder sekundären Aminogruppen und einem Molekulargewicht von 60 bis etwa 6000, vorzugsweise 60 bis 3000, eingesetzt. Es sind dies niedermolekulare und/oder höhermolekulare primäre und/oder sekundäre Polyamine, vorzugsweise Diamine. Die Aminogruppen sind dabei an aliphatische Gruppen (einschließlich cycloaliphatische, oder an den aliphatischen Rest von araliphatischen Gruppen) gebunden. Die aliphatischen bzw. cycloaliphatischen Di- und Polyamine können gegebenenfalls neben den Aminogruppen auch noch OH-Gruppen tertiäre Aminogruppen, Ethergruppen, Thioethergruppen, Urethangruppen, Harnstoffgruppen, Carboxylgruppen oder Carbonsäurealkylestergruppen aufweisen.

Erfindungsgemäß einsetzbare Di- und Polyamine sind z. B. Ethylendiamin, 1,2- und 1,3-Propandiamin, 1,4-Butandiamin, 1,6-Hexandiamin, Neopentandiamin, 2,2,4- und 2,4,4-Trimethyl-1,6-diaminohexan, 2,5-Dimethyl-2,5-diaminohexan, 1,10-Decandiamin, 1,11-Undecandiamin, 1,12-Dodecandiamin, Bis-aminomethyl-hexahydro-4,7-methano-indan (TCD-Diamin), 1,3-Cyclohexandiamin, 1,4-Cyclohexandiamin, 1-Amino-3,3,5-trimethyl-5-aminomethyl-cyclohexan (Isophorondiamin), 2,4- und/oder 2,6-Hexahydrotoluylendiamin, 2,4'- und/oder 4,4'-Diaminodicyclohexylmethan, m- oder p-Xylylendiamin, Bis-(3-aminopropyl)-methylamin, Bis-N,N'-(3-aminopropyl)-piperazin, Diaminoperhydroanthrazene und 1-Amino-2-aminomethyl-3,3,5-(3,5,5)-trimethylcyclopentan, 2,2-Dialkylpentan-1,5-diamine oder Triamine wie 1,5,11-Triaminoundecan, 4-Aminomethyl-1,8-diaminooctan, Lysinmethylester, cycloaliphatische Triamine gemäß DE-OS-2 614 244, 4,7-Dioxadecan-1,10-diamin, 2,4- und 2,6-Diamino-3,5-diethyl-1-methylcyclohexan und deren Gemische, alkylierte Diaminodicyclohexylmethane, z. B. 3,3'-Dimethyl-4,4'-diamino-dicyclohexylmethan oder 3,5-Diisopropyl-3',5'-diethyl-4,4'-diaminodicyclohexylmethan, perhydrierte Diaminonaphthaline, perhydrierte Diaminoanthrazene, oder höherwertige Amine wie Diethylentriamin, Triethylentetramin, Pentaethylenhexamin, Dipropylentriamin, Tripropylentetramin, oder N,N'-Dimethyl-ethylendiamin, 2,5-Dimethylpiperazin, 2-Methylpiperazin, Piperazin(hydrat) und 2-Hydroxyethylpiperazin.

Außer diesen niedermolekularen aliphatischen Diaminen oder im Gemisch hiermit können auch höhermolekulare aliphatische Di- und Polyamine verwendet werden, wie sie z. B. durch reduktive Aminierung von Polyoxyalkylenglykolen mit Ammoniak nach BE-PS-634 741 oder US-PS-3 654 370 erhalten werden können. Weitere höhermolekulare Polyoxyalkylen-Polyamine können nach Methoden, wie sie in der Firmenschrift

"Jeffamine, Polyoxypropylene Amines" von Texaco Chemical Co., 1978, aufgezählt werden, hhergestellt werden, beispielsweise durch Hydrierung von cyanethylierten Polyoxypropylenglykolen (DE-OS-1 193 671), durch Aminierung von Polypropylenglykolsulfonsäureestern (US-PS-3 236 895), durch Behandlung eines Polyoxyalkylenglykols mit Epichlorhydrin und einem primären Amin (FR-PS-1 466 708) oder durch Umsetzung von NCO-Prepolymeren mit Hydroxylgruppen aufweisenden Enaminen, Aldiminen oder Ketiminen und anschließende Hydrolyse gemäß DE-A-2 546 536. Geeignete höhermolekulare aliphatische Di- und Polyamine sind auch die nach DE-OS-29 48 419 und DE-OS-3 039 600 durch alkalische Hydrolyse von NCO-Prepolymeren (mit aliphatischen Diisocyanaten) mit Basen über die Carbamatstufe zugänglichen Polyamine. Diese höhermolekularen Polyamine besitzen Molekulargewichte von etwa 400 bis 6000, vorzugsweise 400 bis 3000 und besonders bevorzugt von 1000 bis 3000. Infolge ihres Aufbaus sind derartige höhermolekulare Polyamine zur Ausbildung einer nicht-brüchigen, "elastischen" Polyharnstoffumhüllung besonders geeignet. Sie werden daher, vorzugsweise in Mischung mit den niedermolekularen Di- und Polyaminoverbindungen, zur Aminstabilisierung der Polyisocyanatteilchen eingesetzt. Im Falle der Verwendung dieser höhermolekularen Aminoverbindungen kann bei der Stabilisierungsreaktion ein Zusatz von Polyolen (zur "Elastifizierung" der Umhüllungshaut der Isocyanatteilchen) unterbleiben.

Als "Stabilisatoren" für die genannten Polyisocyanate werden aber auch Hydrazin, Alkylhydrazine sowie N,N'-Dialkylhydrazine, vorzugsweise mit $C_1$-$C_6$-Alkylgruppen, die auch als weitere Substituenten Chlor- oder OH-Gruppen tragen können (mit Molekulargewichten von vorzugsweise 32 bis 198) und/oder zwei- oder mehrfunktionelle, nieder- oder höhermolekulare Verbindungen mit $-CO.NH.NH_2$-Endgruppen, und einem Molekulargewicht von 90 bis etwa 6000, vorzugsweise 90 bis 3000, eingesetzt. Es sind dies z. B. Hydrazin, zumeist in Form von Hydrazinhydrat, alkylsubstituierte Hydrazine, z. B. Methylhydrazin, Ethylhydrazin, Hydroxyethyl-hydrazin oder N,N'-Dimethylhydrazin. Weiter geeignete "Stabilisatoren" sind Verbindungen mit Hydrazid-Endgruppen, z. B. Di- oder Polyhydrazide wie Carbodihydrazid, Hydracrylsäurehydrazid, Oxalsäuredihydrazid, Adipinsäuredihydrazid, Terephthalsäuredihydrazid, Isophthalsäurehydrazid oder Verbindungen mit Hydrazid- und Semicarbazid-, Carbazinester- oder Amino-Gruppen, z. B. β-Semicarbazidopropionsäurehydrazid, 2-Semicarbazidoethylencarbazinester, Aminoessigsäurehydrazid, β-Aminopropionsäurehydrazid oder Biscarbazinester oder Bissemicarbazide wie Ethylen-bis-carbazinester bzw. Ethylen-bis-semicarbazid oder Isophoron-bis-semicarbazid. Bevorzugt sind Hydrazin und niedermolekulare Verbindungen mit $-CO-NH-NH_2$-Gruppen mit Molekulargewichten von 32 bis 399. Besonders bevorzugt ist Hydrazinhydrat und β-Semicarbazido-propionsäurehydrazid sowie Alkylenbissemicarbazide.

Selbstverständlich können beliebige Kombinationen der genannten "Amin-Stabilisatoren" verwendet werden, um z. B. nachteilige Nebeneffekte eines Amins durch entsprechende Vorteile anderer Amine auszugleichen (z. B. nieder- und höhermolekulare Diamine in gemeinsamer Anwendung) oder um möglichst viele vorteilhafte Nebeneffekte zu vereinigen. In Frage kommen z. B. Kombinationen von schnell reagierenden Aminen wie z. B. Ethylendiamin mit durch sterische Hinderung verlangsamten Aminen oder von niedermolekularen Aminen mit hochmolekularen Aminen wie z. B. aliphatischen Aminopolyethern, oder von Polyaminen mit Hydrazinen oder Hydrazid-Derivaten. Vorzugsweise verwendet man neben Hydrazin- oder Hydrazid-Derivaten höchstens bis 50 Mol-% an Polyaminen, bezogen auf die Gesamtmenge der eingesetzten "Amin-Stabilisatoren".

Die "Amin-Stabilisatoren" werden in einer Menge von 0,1 bis 25 Äquivalentprozent $NH_2$ pro Äquivalent NCO im Polyisocyanat eingesetzt, vorzugsweise 0,1 bis 8 Äquivalentprozent, besonders bevorzugt 0,3 bis 3 Äquivalentprozent. Die Polyadduktumhüllung kann zwar auch mit noch höheren Anteilen, z. B. 30 Äquivalentprozent $NH_2$ pro NCO, durchgeführt werden, doch werden durch solch hohe Umsetzungen die Anteile der reaktiven Isocyanatgruppen bei der Verwendung der stabilisierten Isocyanate in Polyurethan-Einkomponenten-Reaktivsystemen zu stark vermindert und impraktikabel.

Als $NH_2$-Äquivalent gilt bei Hydrazin jeweils eine $-NH_2$-Gruppe (bzw. eine -NH-Alkylgruppe bei Alkylhydrazinderivaten), bei "Hydrazid"-Verbindungen gilt eine $-CO.NH.NH_2$-Gruppe als ein $NH_2$-Äquivalent.

Als Temperatur der Umhüllungsreaktionen werden Temperaturen unter den jeweiligen Schmelztemperaturen des Polyisocyanats gewählt. Sie liegen im allgemeinen unter 70°C, bevorzugt bei 0 bis 50°C.

Die Stabilisierung des bei Raumtemperatur festen Isocyanats gegenüber H-aktiven Verbindungen erfolgt in der Regel innerhalb weniger Minuten, so daß auch eine kontinuierliche Arbeitsweise bei der Herstellung der Einkomponentensysteme gewährleistet wird.

Bei Einkomponenten-PU-Reaktivsystemen unter Mitverwendung der stabilisierten, erfindungsgemäßen Polyisocyanate wird einerseits die Forderung nach einer möglichst unbegrenzten Lagerstabilität bei Raumtemperatur oder wenig erhöhten Temperaturen, z. B. 50 bis 60°C, gestellt und andererseits die rasche Vernetzung der Einkomponentensysteme bei Temperaturen oberhalb von etwa 100°C gefordert. Die von der Praxis an die stabilisierten Polyisocyanate gestellten Bedingungen können sowohl durch die Menge als auch durch die chemische Konstitution der Amin-Stabilisatoren und die Reaktionsbedingungen bei der Umhüllungsreaktion (Konzentration, Temperatur) in hohem Maße gesteuert werden. Wird eine bestimmte Aminmenge bei der Stabilisierungsreaktion überschritten, die bei etwa $\geqslant$ 25 Äquivalentprozent $NH_2$, bezogen auf freies NCO, liegt, so tritt bei Verwendung in den Polyurethan-Reaktivsystemen bei üblichen Ausheizbedingungen (110 bis 140°C) - wenn überhaupt - nur unbefriedigende Vernetzung ein. Es empfiehlt sich daher, bei der gewünschten Rezeptur zunächst die optimale Zusatzmenge des jeweiligen "Amin-Stabilisators" zu bestimmen, wobei dann bei einer ausreichenden Lagerstabilität (z. B. 14 Tage bei 50°C) der Einkomponentensysteme die Aushärtungstemperatur bzw. Aushärtungszeit festgelegt sind. Im allgemeinen ist

der stabilisierende Effekt von niedermolekularen Verbindungen mit primären $NH_2$-Gruppen stärker als bei Verbindungen mit sekundären Aminogruppen. Der stabilisierende Effekt steigt auch mit zunehmender Funktionalität der Amine. Triamine zeigen im allgemeinen stärkere Wirkung als Diamine. Schwächeren Effekt zeigen dagegen höhermolekulare Aminoverbindungen, können jedoch mit niedermolekularen Diaminen mit gutem Wirkungseffekt kombiniert werden.

Die "Amin-Stabilisierung" der festen Polyisocyanate durch Polyaddukt-Umhüllung wird im erfindungsgemäßen Verfahren in einem flüssigen Medium, das kein (gutes) Lösungsmittel für die festen Polyisocyanate darstellt, durchgeführt.

Das flüssige Medium kann aus niedermolekularen und/oder bevorzugt höhermolekularen Mono- und/oder (bevorzugt) Polyolen und/oder aromatischen Polyaminen mit Molekulargewichten von 62 bis 6000 und/oder höhermolekularen aromatischen und höhermolekularen aliphatischen Polyaminen mit Molekulargewichten von 400 bis 6000 bestehen. Vorzugsweise werden jedoch höhermolekulare Polyole und/oder aromatische und/oder aliphatische höhermolekulare Polyamine des Molekulargewichtsbereichs 400 bis 6000, vorzugsweise 400 bis 3000, besonders bevorzugt 1000 bis 3000, eingesetzt, gegebenenfalls unter Zusatz von niedermolekularen Polyolen und/oder aromatischen Polyaminen.

Beispiele für die Monoalkohole sind längerkettige Alkohole wie Isohexadecanol, ferner Propoxylierungsprodukte von einwertigen Alkoholen mit Molekulargewichten von vorzugsweise 400 bis 6000, z. B. Propoxylierungsprodukte von n-Butanol. Diese Monoalkohole sind jedoch weniger bevorzugt, weil sie nicht direkt als Suspendierungsmittel für Polyisocyanate bei der Synthese von Polyurethanen weiter eingesetzt werden können, da sie Abbrecher darstellen. Sie müssen daher in einem zusätzlichen Schritt erst entfernt werden, bevor man die polymerumhüllten Polyisocyanate in den polyurethanbildenden Reaktivsystemen einsetzt.

Geeignete niedermolekulare Polyole sind beispielsweise Butandiol-1,4, Decandiol-1,10, Tetra-(hydroxypropyl)-ethylendiamin oder Ricinusöl.

B)

1.

Die ferner bevorzugten höhermolekularen Polyole sind z. B. Polyoxyalkylen-polyole, z. B. Polyoxytetramethylenglykole oder Ethoxylierungs- und/oder Propoxylierungsprodukte von niedermolekularen Di- und Polyolen oder Di- und Polyaminen, z. B. propoxyliertes Trimethylolpropan, propoxyliertes Ethylendiamin oder lineare oder verzweigte Polypropylenglykolether, die anteilsweise in statistischer, blockartiger oder endständiger Form Ethylenoxid enthalten können und insgesamt Molekulargewichte von 400 bis 6000 aufweisen. In einer Ausführungsform werden beispielsweise zwei- oder mehrfunktionelle höhermolekulare Polyole B), gegebenenfalls unter Zusatz niedermolekularer Polyole C) als flüssiges Medium zur Suspendierung der stabilisierten Polyisocyanate verwendet, welche bei der Verwendung zum Aufbau von Polyurethanen direkt als Hydroxylgruppen tragende Reaktionsteilnehmer benutzt werden. Es können daher alle, üblicherweise für die Synthese von Polyurethanen eingesetzten höhermolekularen Verbindungen (B) mit endständigen OH-Gruppen, wie Polyether, Polyacetale, Polythioether und auch Polyester verwendet werden, wie sie beispielsweise in der DE-OS-2 920 501 aufgeführt sind. Beim direkten Einsatz der Suspension der stabilisierten Polyisocyanate in den Polyolen für Einkomponenten-Polyurethansysteme können die (höhermolekularen) Polyole B) auch entsprechende Mengen an niedermolekularen Polyolen, vorzugsweise Diolen und/oder aromatischen Polyaminen, vorzugsweise Diaminen, enthalten, welche Molekulargewichte zwischen 60 und 399 aufweisen. Oft werden diese Kettenverlängerungsmittel C) den Suspensionen der Polyharnstoff-umhüllten Polyisocyanate jedoch erst anschließend zugesetzt. Dem System können besonders bevorzugt auch die niedermolekularen aromatischen Polyamine (als Kettenverlängerungsmittel C)) zugefügt werden, wenn entsprechende Einkomponenten-PU-Reaktivsysteme hergestellt werden. Bevorzugt ist eine Umsetzung der Komponenten in solchen Mengen, wie sie der Rezeptur der Einkomponenten-PU-Reaktivsysteme entsprechen.

Als höhermolekulare Polyhydroxylverbindungen B), die sowohl als Suspendierungsmedium für die Polyisocyanate, wie auch als weitere Reaktivkomponenten für die Polyurethanherstellung eingesetzt werden können, sind zwei- oder höherwertige Polyhydroxylverbindungen mit 2 bis 8, vorzugsweise 2 bis 4, Hydroxylgruppen und einem Molekulargewicht von 400 bis 6000 geeignet. Dies sind mindestens zwei Hydroxylgruppen aufweisende Polyester, Polyether, Polythioether, Polyacetale, Polycarbonate, Polylactone oder Polyesteramide, sowie auch Polybutadienverbindungen oder ihre Mischungen, wie sie für die Herstellung von homogenen, gegebenenfalls zellförmigen oder schaumstoffartigen Polyurethanen an sich bekannt sind. Besonders bevorzugt sind Polyether und Polyester.

Die in Frage kommenden Polyether sind solche der an sich bekannten Art und werden z. B. durch Polymerisation von Tetrahydrofuran oder von Epoxiden wie Ethylenoxid, Propylenoxid, Butylenoxid, Styroloxid oder Epichlorhydrin bzw. durch Anlagerung dieser Epoxidverbindungen, vorzugsweise von Ethylenoxid oder -propylenoxid, gegebenenfalls im Gemisch oder nacheinander, an Startkomponenten mit reaktionsfähigen Wasserstoffatomen wie Wasser, mehrwertige Alkohole, Ammoniak oder mehrwertige Amine oder Zucker hergestellt.

Die in Frage kommenden, Hydroxylgruppen aufweisenden Polyester sind z. B. Umsetzungsprodukte von mehrwertigen, vorzugsweise zweiwertigen, und gegebenenfalls zusätzlich drei- und mehrwertigen Alkoholen mit mehrwertigen, vorzugsweise zweiwertigen, Polycarbonsäuren oder deren Anhydriden oder entsprechenden Polycarbonsäureestern von niedrigen Alkoholen.

Die Polycarbonsäuren können aliphatischer, cycloaliphatischer, aromatischer, araliphatischer und/oder heterocyclischer Natur und gegebenenfalls, z. B. durch Halogenatome, substituiert, sie können aber auch ungesättigt sein.

Als Beispiele für solche Carbonsäuren und deren Derivate seien genannt: Adipinsäure, Sebacinsäure, Azelainsäure, Dodecandisäure, Phthalsäure, Isophthalsäure, Tetrahydrophthalsäureanhydrid, Tetrachlorphthalsäureanhydrid, Endomethylentetrahydrophthalsäureanhydrid, Glutarsäureanhydrid, Maleinsäureanhydrid, Fumarsäure, dimerisierte und trimerisierte ungesättigte Fettsäuren, Terephthalsäuredimethylester und Terephthalsäure-bis-glykolester.

Als mehrwertige Alkohole kommen z. B. Ethylenglykol, Propandiol-1,2 und -1,3, Butandiol-1,4 und -2,3, 2,3-Dibrombutendiol, Hexandiol-1,6, Decandiol-1,10, Neopentylglykol, 1,4-Bis-hydroxymethylcyclohexan, 2-Methyl-1,3-propandiol, Glycerin, Trimethylolpropan, Hexantriol-1,2,6, Pentaerythrit, Chinit, Mannit und Sorbit, Formit oder Formose, Methylglykosid, ferner Di-, Tri-, Tetra-ethylenglykole, -propylenglykole sowie -butylenglykole in Frage.

Auch Polyester aus Lactonen, z. B. ε-Caprolacton, oder aus Hydroxycarbonsäuren, z. B. ω-Hydroxycapronsäure, sind einsetzbar, insbesondere wenn sie zusätzliche Komponenten, wie Diethylenglykol oder 1,4-Butandiol, enthalten, um ihre hohe Kristallinität zu mindern.

Als Polyacetale sind z. B. die aus Glykolen und Formaldehyd herstellbaren Verbindungen geeignet.

Als Hydroxylgruppen aufweisende Polycarbonate kommen solche der an sich bekannten Art in Betracht, z. B. die durch Umsetzung von Propandiol-1,3, Butandiol-1,4 und/oder Hexandiol-1,6, Di-, Tri- oder Tetraethylenglykol oder Thiodiglykol, mit Diarylcarbonaten, z. B. Diphenylcarbonat, oder Phosgen, hergestellt werden können.

Auch Hydroxylendgruppen aufweisende Polybutadiene sind erfindungsgemäß geeignet, da sie besonders elastische und hydrolysenstabile Produkte ergeben. Es können gegebenenfalls auch Polyhydroxylverbindungen eingesetzt werden, in welchen hochmolekulare Polyaddukte bzw. Polykondensate oder Polymerisate in feindisperser oder auch gelöster Form enthalten sind.

Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen in situ in o.g., Hydroxylgruppen aufweisenden, Verbindungen, ablaufen läßt.

Auch durch Vinylpolymerisate modifizierte Polyhydroxylverbindungen, wie sie z. B. durch Polymerisation von Styrol und Acrylnitril in Gegenwart von Polyethern oder Polycarbonatpolyolen erhalten werden, sind für das erfindungsgemäße Verfahren geeignet.

Weitere Vertreter der genannten zu verwendenden Verbindungen sind z. B. in High Polymers, Vol. XVI, "Polyurethans, Chemistry and Technology", verfaßt von Saunders-Frisch, Interscience Publishers, New York, London, Band I, 1962, Seiten 32 bis 42 und Seiten 44 bis 54 und Band II, 1964, Seiten 5 bis 6 und 198 bis 199, sowie im Kunststoff-Handbuch, Band VII, Vieweg-Höchtlen, Carl-Hanser-Verlag, München, 1966, z. B. auf den Seiten 45 bis 71, sowie in der DE-A-2 854 384 ausführlich beschrieben.

Es ist selbstverständlich möglich, Mischungen der o.g. Polyhydroxylverbindungen einzusetzen.

B)

2.

Als flüssiges Medium werden auch flüssige oder niedrigschmelzende (50°C) nieder- und/oder höhermolekulare aromatische Polyamine und/oder höhermolekulare aliphatische Polyamine verwendet. Niedermolekulare aromatische Polyamine (Molekulargewicht bis 399) sind als alleiniges flüssiges Medium weniger bevorzugt, bevorzugt werden höhermolekulare Polyamine.

Als höhermolekulare Polyaminoverbindungen mit aromatischen Aminogruppen mit einem Molekulargewichtsbereich von 400 bis 6000 werden insbesondere solche Polyaminoverbindungen eingesetzt, wie sie durch Hydrolyse von entsprechenden NCO-Prepolymeren auf der Basis von höhermolekularen Polyhydroxylverbindungen und überschüssigen aromatischen Diisocyanaten durch (vorzugsweise basische) Hydrolyse hergestellt werden können. Beispiele für dieses Verfahren werden in der DE-OS-2 948 419, DE-OS-3 039 600, DE-OS-3 112 118, der EP-A-61 627, EP-A-71 132 und EP-A-71 139 angegeben. In der erstgenannten Patentschrift werden auch weitere Verfahren des Standes der Technik zur Herstellung von aromatischen Aminoverbindungen höhermolekularer Struktur genannt, wie sie für das erfindungsgemäße Verfahren geeignet sind. Es handelt sich bei dem Verfahren nach DE-OS-2 948 419 und den anderen zitierten Patentschriften vorzugsweise um Polyether-Polyamine, aber auch Polyester-, Polyacetal-, Polythioether- oder Polycaprolacton-Polyamine, vorzugsweise 2- oder 3-funktionelle Polyamine, welche Urethangruppen (aus der Reaktion der entsprechenden höhermolekularen Polyhydroxylverbindungen mit den überschüssigen Polyisocyanaten) enthalten und die Aminogruppen an den Rest des (ehemaligen) Polyisocyanats tragen. Die aromatischen, höhermolekularen Polyamine können jedoch auch nach anderen Verfahren, z. B. durch Umsetzung von NCO-Prepolymeren mit überschüssigen Mengen an Hydrazin, Aminophenylethylamin, oder anderen Diaminen entsprechend DE-AS-1 694 152 hergestellt werden; eine andere Synthesemöglichkeit beschreibt die FR-PS-1 415 317 durch Überführung der NCO-Prepolymere mit Ameisensäure in die N-Formylderivate und deren Verseifung. Auch die Umsetzung von NCO-Prepolymeren mit Sulfaminsäure gemäß DE-AS-1 155 907 führt zu höhermolekularen Polyaminen. Neben an aromatische Reste gebundenen Aminogruppen (aus aromatischen Polyisocyanaten) lassen sich auch (über aliphatische Polyisocyanate) an

aliphatische Reste gebundene, höhermolekulare Polyaminoverbindungen herstellen.

Diese höhermolekularen, aliphatischen Polyamine, wie sie bereits auf Seite 25 und 26 als höhermolekulare "Amin-Stabilisatoren" beschrieben wurden, können sowohl als "Amin-Stabilisator", als auch als höhermolekulare Polyaminoverbindung C) ("flüssiges Medium") Verwendung finden, wenn die "Stabilisierungsreaktion" bei niedrigen Temperaturen, z. B. Raumtemperatur, durchgeführt werden. Hier kommt die "Stabilisierungsreaktion" überraschenderweise bei weniger als 25 % Umsatz aller NCO-Gruppen zu einem Stillstand. Wird die Temperatur entsprechend gesteigert, z. B. auf 120°C, so tritt jedoch eine Durchreaktion aller aliphatischen Aminogruppen mit den Isocyanatgruppen ein. Hierbei können direkt Elastomere erhalten werden, wenn das Verhältnis zwischen NCO-Gruppen und NCO-reaktiven Gruppen (OH- und/oder $NH_2$-Gruppen) gleich im polymerbildenden Bereich liegt.

Als flüssiges Medium können auch weichmacherartige Verbindungen bei der Stabilisierung der Isocyanate verwendet oder mitverwendet werden, z. B. Phthalate, wie Dioctyl-, Diisododecyl-, Dibenzyl-, Butylbenzylphthalat, oder auch Phosphate mit $\geqslant$ 8 C-Atomen im Alkylrest, wie Trioctylphosphat. Auch Kohlenwasserstoffe wie sog. Butadienöle, oder Polyether mit höherem Molekulargewicht, können als Reaktionsmedium verwendet werden. Dabei wird im allgemeinen so verfahren, daß das feinpulverisierte feste Isocyanat zu einer Lösung des Amin-Stabilisators im Weichmacher möglichst bei Raumtemperatur eingerührt wird. Wenn man die stabilisierten Isocyanate in dieser Suspension verwenden will, kann die Zugabe der gewünschten weiteren Ausgangskomponenten, wie z. B. höhermolekulare aromatische Polyamine, nach Stabilisierung des Polyisocyanats erfolgen. Man kann jedoch auch diese Weichmacher mit höhermolekularen Polyaminen vermischt als flüssiges Medium zur Bildung der Suspension der stabilisierten Polyisocyanate einsetzen. Auch eine Isolierung der stabilisierten Polyisocyanate durch Filtration und deren nachträgliche Suspendierung in den höhermolekularen Polyol- und/oder Polyaminverbindungen ist möglich, wenn auch nicht bevorzugt.

Als flüssiges Medium ist auch überraschenderweise Wasser geeignet, wobei man dem Wasser die "Amin-Stabilisatoren" zugibt und mit dieser Lösung die festen Polyisocyanate vermischt. Bei Verwendung von insbesondere niedermolekularen aliphatischen Polyaminen als Aminstabilisatoren erreicht man bei diesem Verfahren jedoch oftmals nur dann eine völlig befriedigende Stabilisierung des Isocyanates, wenn in Gegenwart von geringen Mengen (z. B. 2 bis 25 Gew.-%, vorzugsweise 5 bis 15 Gew.-%, bezogen auf Wasser, einer höhermolekularen Polyol- oder höhermolekularen (aromatischen) Polyaminkomponente gearbeitet wird.

Bei Verwendung von Wasser als wesentlichem Reaktionsmedium zur Stabilisierung der Polyisocyanate wird im allgemeinen das stabilisierte Polyisocyanat durch Filtration isoliert, gegebenenfalls vorsichtig getrocknet und in der isolierten, feinpulvrigen Form den gewünschten höhermolekularen Polyolen und/oder Polyaminen und gegebenenfalls weiteren Ausgangskomponenten für die Einkomponenten-Polyurethan-Reaktivmischungen zugemischt. Diese Zwischenstufe einer Isolierung der "stabilisierten" Polyisocyanate ist jedoch nicht bevorzugt.

Den genannten flüssigen Medien (Polyolen, Polyaminen, Weichmachern oder Wasser) können gegebenenfalls Lösungsmittel unpolarer oder wenig polarer Art zugemischt werden, z. B. aliphatische, cycloaliphatische oder aromatische Kohlenwasserstoffe, Halogenkohlenwasserstoffe, Ether, Ketone oder Ester, vorzugsweise mit Siedepunkten unter 146°C. Hierdurch läßt sich gegebenenfalls eine Reaktion in einem niederviskoseren Medium erreichen; die Lösungsmittel werden zweckmäßig anschließend wieder entfernt, z. B. durch Abziehen im Vakuum.

Die geschilderten Stabilisierungsreaktionen führen zu einer Suspension von Polyaddukt-umhüllten, stabilisierten Polyisocyanaten in dem flüssigen Medium.

Die Suspensionen enthalten mindestens 3 Gew.-%, bevorzugt mindestens 5 Gew.-%, und in den meisten Fällen mindestens 7,5 Gew.-% feste stabilisierte Polyisocyanate; die Feststoffgehalte liegen zumeist unter 70 Gew.-%, vorzugsweise unter 50 Gew.-% und in den meisten Fällen unter 40 Gew.-%.

Sind die Polyisocyanate in einem solchen Medium, z. B. höhermolekularen Polyolen oder Polyaminen, suspendiert, welche für die weiteren Reaktionen zu Polyurethanen geeignet sind, so kann diese Suspension direkt weiterverwendet werden. Gegebenenfalls - wenn auch weniger bevorzugt - werden jedoch die stabilisierten Polyisocyanate aus der Suspension, vor allem bei Anwendung von Wasser, Mono-olen oder sehr viel Weichmacher und/oder Lösungsmittel, z. B. durch Filtration, isoliert und als Pulver den gewünschten Reaktionskomponenten (den höhermolekularen Polyolen und/oder Polyaminen B), gegebenenfalls weiteren, gleichartig oder verschieden aufgebauten höhermolekularen Polyolen B), sowie gegebenenfalls niedermolekularen Kettenverlängerungsmitteln C)) zugesetzt.

Besonders wichtig für die Praxis sind solche lagerstabilen Suspensionen der stabilisierten Polyisocyanate in höhermolekularen Polyaminen B), gegebenenfalls unter Zusatz weiterer, höhermolekularer Polyole B) und/oder Kettenverlängerungsmittel C), z. B. niedermolekularen Polyamin- und niedermolekularen Polyolen, wie sie direkt für die Einkomponentenreaktion bzw. für die Formulierung von Einkomponentensystemen verwendet werden können. Bevorzugt ist eine Umsetzung der Komponenten in solchen Mengen- und Äquivalentverhältnissen, wie sie einer Rezeptur von Einkomponenten-PU-Reaktivsystemen direkt entsprechen.

Die langzeitlagerbeständigen Einkomponenten-PU-Reaktivsysteme, die sich zu massiven oder geschäumten Polyurethanen härten lassen, werden bevorzugt durch in situ-Stabilisierung der Polyisocyanate mittels der aliphatischen Aminstabilisatoren, Hydrazin(e) und/oder Hydrazid-Verbindungen in Gegenwart der für das Einkomponenten-Polyurethan-System erwünschten höhermolekularen Polyol- oder Polyaminverbindungen B), gegebenenfalls unter Zusatz von weiteren höhermolekularen Polyhydroxylverbindungen B) und gegebenenfalls

niedermolekularen Polyolen und/oder aromatischen Polyaminen als Kettenverlängerungsmitteln C) hergestellt. Hierbei wird der durch Vorversuche ermittelte, geeignete "Amin-Stabilisator" in der gewünschten Konzentration in höhermolekularen Polyolen oder Polyaminen, z. B. den Polyhydroxy-Polyethern, -polyestern oder -polycarbonaten oder den Aminopolyethern, Aminopolyestern oder Aminopolyacetalen, bei vorzugsweise möglichst geringer Temperatur (Raumtemperatur) zugesetzt. Danach erfolgt die Zumischung des bei Raumtemperatur festen (pulverförmigen) Polyisocyanats, wobei sich - innerhalb weniger Minuten - das stabilisierte, durch Polyharnstoff umhüllte Polyisocyanat bildet. Vorher oder anschließend können noch die gewünschten niedermolekularen Kettenverlängerungsmittel C), vorzugsweise aromatische Polyamine oder niedermolekulare (aliphatische oder cycloaliphatische) Polyolverbindungen, gegebenenfalls weitere höhermolekulare Polyhydroxyl- oder Polyamino-Verbindungen B) und gegebenenfalls der gewünschte Katalysator D) und die üblichen Hilfs- und Zusatzstoffe E) der Suspension zugesetzt werden.

Wie bereits erwähnt, werden die erfindungsgemäßen, langzeitlagerbeständigen Einkomponenten-Reaktivsysteme vorzugsweise unter Mitverwendung von niedermolekularen Kettenverlängerern oder Vernetzern hergestellt (Komponente C)).

C)

Bei diesen niedermolekularen Kettenverlängerern oder Vernetzern (Komponente C)) handelt es sich um zwei- oder mehrfunktionelle Verbindungen, welche an aliphatische und/oder cycloaliphatische Gruppen gebundene Hydroxylgruppen (Polyole) und/oder an aromatische, einschließlich heterocyclische Ringe mit aromatischem Charakter gebundene $NH_2$-Gruppen (Polyamine) und Molekulargewichte zwischen 62 und 399 aufweisen. Bevorzugt sind dabei niedermolekulare Diole mit an aliphatische oder cycloaliphatische Gruppen gebundenen Hydroxylgruppen, sowie aromatische Diamine des genannten Molekulargewichtsbereichs bis 399.

Diese Verbindungen weisen in der Regel 2 bis 8, vorzugsweise 2 bis 4, besonders bevorzugt jedoch 2, gegenüber Isocyanaten reaktionsfähige Wasserstoffatome wie Hydroxyl- und/oder Aminogruppen auf. Es können selbstverständlich auch Mischungen von verschiedenen Verbindungen des Typs C) verwendet werden. Als Beispiele für derartige Verbindungen seien genannt: Ethylenglykol, Trimethylenglykol, Butandiol-2,3 und/oder -1,4, Hexandiol-1,6, Neopentylglykol, 1,4-Bis-hydroxyethyl-cyclohexan, 1,4-Dihydroxycyclohexan, Terephthalsäure-bis(β-hydroxyethyl)ester, 1,4,3,6-Dianhydrohexite, 1,4-Monoanhydrotetrite, sowie weniger bevorzugt Diole mit sekundären Hydroxylgruppen, z. B. Propylenglykol, Butandiol-2,3, oder Pentandiol-2,5. Als mehrwertige Verbindungen seien genannt: Trimethylolpropan, Trimethylolethan, Hexantriol-1,2,6, Glycerin, Pentaerythrit, Chinit, Mannit, Sorbit, Rizinusöl, sowie Di-, Tri- und Tetraethylen-, -propylen-, und -butylenglykole, ferner Bis-(2-hydroxyethyl)-hydrochinon, Bis-(2-hydroxyethyl)-resorcin, Formose oder Formit. Ferner sind geeignet tertiäraminhaltige Di- oder Polyole, z. B. N-Methyldiethanolamin, Triethanolamin oder N,N'-Bis-hydroxyethylpiperazin.

Bevorzugt werden jedoch anstelle von niedermolekularen Polyolen niedermolekulare aromatische Diamine eingesetzt. Unter aromatischen Polyaminen sollen auch solche Amine verstanden werden, welche die Aminogruppe an heterocyclische Reste mit aromatischem Charakter gebunden enthalten. Als aromatische Polyamine sind z. B. geeignet: p-Phenylendiamin, 2,4-/2,6-Toluylendiamine, Diphenylmethan-4,4'- und/oder -2,4'- und/oder -2,2'-diamine, 3,3'-Dichlor-4,4'-diaminodiphenylmethan, 3-($C_1$-$C_8$)-Alkyl-4,4'-diaminodiphenylmethane, die 3,3'-Di-($C_1$-$C_4$)-4,4'-diaminodiphenylmethane sowie die 3,3',5,5'-Tetra-($C_1$-$C_4$)-alkyl-4,4'-diphenylmethane, die 4,4'-Diaminodiphenyl-sulfide, -sulfoxide oder -sulfone, Ethergruppen aufweisende Diamine gemäß DE-A-1 770 525 und 1 809 172 (US-PS-3 654 364 und 3 736 295), gegebenenfalls in 5-Stellung substituierte 2-Halogen-1,3-phenylendiamine (DE-A-2 001 772, 2 025 896 und 2 065 869), Bisanthranilsäureester (DE-A-2 040 644 und 2 160 590), 2,4-Diamino-benzoesäureester nach DE-A-2 025 900, sowie durch eine oder zwei ($C_1$-$C_4$)-Alkylgruppen substituierte Toluylendiamine. Besonders bevorzugt sind 3,5-Diethyl-2,4- und/oder -2,6-diaminotoluol (besonders ihre technischen (80/20)- oder (65/35)-Isomerengemische), unsymmetrisch tetraalkylsubstituierte Diaminodiphenylmethane, z. B. 3,5-Diethyl-3'-5'-diisopropyl-4,4'-diaminodiphenylmethan und ihre Isomerengemische entsprechend DE-A-2 902 090, 4,4'-Diaminobenzanilid, sowie 3,5-Diamino-benzoesäure-($C_1$-$C_4$)-alkylester, 4,4'- und/oder 2,4'-Diamino-diphenylmethan, sowie Naphthylen-1,5-diamin.

Die aromatischen Diamine sind bevorzugt vor den Glykolen. Es sind jedoch auch Diole oder Diamine mit zusätzlichen Gruppen einsetzbar, z. B. Adipinsäure-bis-(2-hydroxyethyl)-ester, Terephthalsäure-bis-(2-hydroxyethyl)-ester, Diol-urethane, Diol-harnstoffe oder Polyole, welche Sulfonat- und/oder Phosphonatgruppen enthalten, z. B. 1,6-Hexamethylen-bis-(2-hydroxyethylurethan), 4,4'-Diphenylmethan-bis-(2-hydroxyethylharnstoff) oder das Addukt von Na-Bisulfit an Butendiol-1,4, bzw. dessen Alkoxylierungsprodukte. Weitere niedermolekulare Verbindungen C) werden ausführlich in der DE-A-2 854 384 beschrieben.

Ferner können in üblicher Weise gegebenenfalls gegenüber Isocyanaten monofunktionelle Verbindungen in Anteilen von 0,01 bis 10 Gew.-% als sogenannter Kettenabbrecher mitverwendet werden. Derartige monofunktionelle Verbindungen sind z. B. Monoamine, wie Butyl- oder Dibutylamin, Stearylamin, Pyrrolidin, Anilin oder Tolylamin, Butanol, 2-Ethylhexanol, Cyclohexanol oder Ethylenglykolmonoethylester.

D)

Als Katalysatoren D) für die erfindungsgemäß langzeitlagerstabilen Einkomponentensysteme können die üblichen Polyurethankatalysatoren, mit besonders gutem Effekt aber organische Blei- und/oder

Zinnverbindungen verwendet werden, gegebenenfalls unter Mitverwendung weiterer, üblicher Polyurethankatalysatoren, insbesondere von tert.-Amin-haltigen Katalysatoren.

Von den Bleiverbindungen werden Verbindungen aus folgender Gruppe bevorzugt:

a) organische Salze des zweiwertigen Bleis mit Carbonsäuren,

b) Dithiocarbamate des zweiwertigen Bleis der Formel

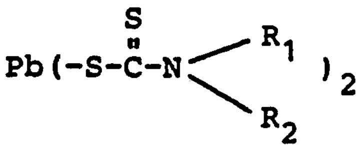

worin

$R_1$ und $R_2$ verschieden sein können und einen $C_1$-$C_{20}$-Alkylrest bedeuten,

c) Tetraorgano-Blei-IV-Verbindungen, wobei der organische Rest einen niederen Alkylrest, wie z. B. Methyl oder Ethyl, bedeutet und

d) Verbindungen aus 1,3-Dicarbonylverbindungen, wie z. B. Acetylaceton, mit zweiwertigem Blei.

Als organische Zinnverbindungen kommen Zinn-(II)-salze von Carbonsäuren wie Zinn-acetat, Zinn-octoat, Zinn-ethylhexanoat und Zinn-laurat, und die Zinn-(IV)-Verbindungen, z. B. Dibutylzinnoxid, Dibutylzinndichlorid, Dibutylzinndiacetat, Dibutylzinndilaurat, Dibutylzinnmaleat oder Dibutylzinndiacetat in Betracht.

Bevorzugt verwendet werden bei den Zinnkatalysatoren jedoch schwefelhaltige Zinnverbindungen der Formel

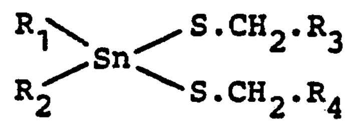

worin

$R_1$ und $R_2$ Alkylreste mit 1 bis 10 C-Atomen,

$R_3$ und $R_4$ Wasserstoff und/oder $C_1$-$C_{18}$-Alkyl und/oder den Rest $COOR_1$ bedeuten.

Als Beispiele seien Di-(octyl)-zinn(IV)-bis-thiomethyl oder Dimethyl-zinn-bis-thiolauryl genannt. Besonders bevorzugt verwendet werden die Verbindungen, in denen $R_3$ und $R_4$ den genannten Esterrest bedeuten, z. B. Dimethyl-zinn-bis-thioglykolsäurehexylester oder Dibutylzinn-bis-thioglykol-octylester. Selbstverständlich können die o.g. Katalysatoren als Gemische eingesetzt werden, insbesondere dann, wenn die niedermolekularen Kettenverlängerer und die höhermolekularen Polyole gleichzeitig primäre und sekundäre OH-Gruppen tragen, bzw. wenn die H-aciden Verbindungen verschiedene Reaktivitäten aufweisen (vgl. Beispiel 13). Von Interesse sind auch Kombinationen der organischen Metallverbindungen mit Amidinen, Aminopyridinen, Hydrazinopyridinen (DE-A-2 434 185, 2 601 082 und 2 603 834), oder 1,4-Diaza-bicyclo-2.2.2-octan und/oder üblichen, tert.-Amin-Katalysatoren, wie sie in der Polyurethanchemie üblicherweise verwendet werden.

Die Bleikatalysatoren sind dabei von besonderer Wirksamkeit und Effektivität, wenn in dem System Polyetherpolyole mit sekundären Hydroxylgruppen, z. B. Polypropylenoxidglykole, verwendet werden.

Bei Einsatz von Uretdiondiisocyanaten kann insbesondere bei Bleikatalysatoren auch eine zusätzliche Vernetzung infolge Spaltung des Uretdionringes erfolgen, wobei jedoch verzweigende Allophanatgruppen oder bei vollständiger Spaltung des Uretdionrings zusätzliche Urethangruppen gebildet werden.

Bei Einsatz von (Polyester)polyolen mit im wesentlichen primären Hydroxylgruppen sind dagegen die Zinnverbindungen, besonders die Zinn/Schwefel-Katalysatoren, von besonderer Wirksamkeit. Bei aromatischen $NH_2$-Gruppen-haltigen Polyethern kann zumeist auf eine Katalyse völlig verzichtet werden. Die Katalysatoren werden in der Regel in einer Menge von 0,001 bis 5 Gew.-%, vorzugsweise von 0,01 bis 2 Gew.-%, bezogen auf A) + B), eingesetzt.

Als gegebenenfalls einzusetzende Hilfs- und Zusatzstoffe E) seien genannt: Farbstoffe oder Pigmente, Füllstoffe wie Silicagel, Gips, Talkum, Aktivkohle, Metallpulver, UV-Absorptionsmittel oder Stabilisatoren wie phenolische Antioxidantien, Lichtschutzmittel, Treibmittel, oberflächenaktive Zusatzstoffe wie Emulgatoren oder Schaumstabilisatoren, gegebenenfalls Zellregler, Antiblockmittel, Silikone, Flammschutzmittel, oder fungistatisch und/oder bakteriostatisch wirkende Substanzen.

Als Füllstoffe sind z. B. Fasermaterialien, d.h. alle an sich bekannten anorganischen und/oder organischen, faserförmigen Verstärkungsmaterialien einsetzbar, z. B. Glasfasern, vorzugsweise in Längen zwischen 20 + 60 mm, Graphitfasern und Asbestfasern oder Fasermaterialien, die von einem organischen Polymeren stammen, z. B. von einem Polyester wie Polyethylenterephthalat, oder vorzugsweise aromatischen Polyamiden, wie dem m-Phenylen/Isophthalsäurepolyamid, oder dem Poly-p-phenylen-terephthalamid, oder auch Polycaprolactam. Diese Fasermaterialien können auch als Matte, Band, durchgehende Fasern, Vlies, Stoff oder als Stapelfaserwirrgemisch vorliegen. Bevorzugt sind Glasfasern, die mit Schlichten ausgerüstet sind, um den Fasern eine Affinität zu Polyurethanen zu verleihen. Die einzubauende Füllstoffmenge hängt von der

gewünschten Verbesserung der mechanischen Eigenschaften ab, im allgemeinen werden 5 bis 60 Gew.-% Fasermaterial angewendet.

Das $NCO/(NH_2 + OH)$-Verhältnis (NCO aus reaktivem, stabilisiertem Polyisocyanat und gegebenenfalls weiterem, freiem Polyisocyanat zu Amino- und/oder OH-Gruppen aus höhermolekularen Polyolen und/oder Polyaminen B) und/oder Kettenverlängerungsmitteln C)) beträgt bei der Polyurethanbildung 0,5 : 1 bis 1,5 : 1, bevorzugt 0,8 : 1 bis 1,5 : 1, besonders bevorzugt 0,95 : 1 bis 1,2 : 1 (Angaben in Äquivalenten).

Pro $(OH + NH_2)$-Äquivalent an höhermolekularen Polyolen und/oder Polyaminen B) werden dabei 0,3 bis 10, bevorzugt 0,5 bis 8 und besonders bevorzugt 0,75 bis 5 Äquivalente an $(OH + NH_2)$-Äquivalenten an Kettenverlängerungsmitteln C), d.h. niedermolekularen Polyolen oder niedermolekularen Polyaminen in den Polyurethan-Reaktiv-Mischungen eingesetzt.

Uretdionringe enthaltende (dimere) Diisocyanate können im allgemeinen als Diisocyanate betrachtet werden, so daß nur die freien NCO-Gruppen berücksichtigt werden. Unter bestimmten Versuchsbedingungen (Verwendung von Bleikatalysatoren, höhere Verarbeitungstemperatur, z. B. > 140° C) greift der Uretdionring jedoch mit in die Reaktion ein (zusätzliche Verknüpfungsstellen über Allophanat- oder Biuretgruppen), so daß die latenten NCO-Gruppen des Uretdionrings bei der Berechnung mitberücksichtigt werden müssen.

Je nach Viskosität bzw. Schmelzverhalten der Ausgangskomponenten erhält man bei Raumtemperatur gut gießbare, rakelfähige oder auch feste, leicht aufschmelzbare Einkomponenten-PU-Reaktivmischungen. Diese Reaktivmischungen stellen dabei eine heterogene Suspension der festen, stabilisierten Isocyanate in den Polyol- und/oder Polyaminkomponenten dar. Die Hitzevernetzung dieser Mischung erfolgt zumeist erst nach Zugabe geeigneter Katalysatoren D). Ohne diese Katalysatoren werden insbesondere bei Verwendung von Polyolen als höhermolekulare Verbindungen B) oder Kettenverlängerungsmittel C) nicht befriedigende Eigenschaften der Polyurethanformkörper erhalten. Bei alleiniger Verwendung der gegenüber NCO-Gruppen deutlich reaktiveren aromatischen Polyaminverbindungen kann jedoch auf einen Zusatz von Katalysatoren verzichtet werden.

Ein weiteres Merkmal der Einkomponenten-PU-Reaktivsysteme ist, daß die erfindungsgemäß stabilisierten Einkomponentensysteme nach Erreichen einer (von Menge und Art des Stabilisatoramins abhängigen) Temperatur innerhalb weniger Minuten vernetzen. Das bedeutet, daß einerseits unterhalb dieser Temperatur ("Aufdickungstemperatur") ein erwünschter langer Fließweg der noch unvernetzten Reaktivmischung ein völliges Ausfüllen der heißen Werkzeugform erlaubt, andererseits aber die folgende rasche Verfestigung der Gießansätze nach Temperaturerhöhung schnelle Entformungszyklen gestattet. Erfindungsvorteil ist auch die selbst bei höheren Lagertemperaturen (z. B. bis 60° C) sehr lange Lagerzeit der Ausgangsreaktivsysteme. Der Vorteil gegenüber dem Stand der Technik, wo eine Reaktionsverzögerung in Einkomponentensystemen nur durch "Heterogenität" einer oder mehrerer Komponenten erzielt wird, ist hier zusätzlich durch einen Schutz durch eine Polyadduktumhüllung erheblich verbessert, wobei der "Schutz" erst durch den Hitzestoß (oder sehr starke Scherung oder Anlösen mittels hochpolarer Lösungsmittel) aufgehoben werden kann. Unter Verwendung der erfindungsgemäßen Polyisocyanatsuspensionen sind die Verwendungsmöglichkeiten von Einkomponentensystemen deutlich verbreitert und es können auch flüssige, nicht nur erstarrende höhermolekulare Polyamin- und Polyolsysteme, eingesetzt werden und es sind auch nicht nur ausgewählte Kettenverlängerungsmittel (z. B. hochschmelzende Kettenverlängerungsmittel) einsetzbar. Ein wesentliches Merkmal der erfindungsgemäßen Einkomponentensysteme besteht darin, daß man in diesen Systemen auch aromatische Diamine, wie z. B. 4,4'-Diaminodiphenylmethan, 2,4- oder 2,6-Diaminotoluol, 3,5-Diethyl-2,4/2,6-(65/35)-diaminotoluol, 1,5-Diaminonaphthalin oder 3,5-Diethyl-3',5'-diisopropyl-4,4'-diamino-diphenylmethan als Kettenverlängerungsmittel verwenden kann, ohne den Charakter eines Einkomponentensystems einzubüßen. Werden dagegen diese genannten Diamine in einer der bisher üblichen Verfahrenstechniken mit NCO-Prepolymeren umgesetzt, so resultieren extrem kurze Gießzeiten, so daß ein einwandfreier Verlauf dieser Ansätze im Werkzeugteil nicht möglich ist.

Durch die Verwendung von höhermolekularen Polyaminen in dem Einkomponentensystem lassen sich Polyurethan(harnstoff)e mit deutlich günstigeren Eigenschaften (z. B. höheren Festigkeiten, höheren Spannungswerten, höherer Härte und höheren Erweichungsbereichen) erzielen als mit höhermolekularen Polyolen in der PU-Reaktivmischung alleine.

Die erfindungsgemäßen, gegebenenfalls katalysatorhaltigen Einkomponentensysteme werden im wesentlichen durch Hitzestoß verfestigt. Bei Raumtemperatur oder wenig erhöhter Temperatur tritt auch in Gegenwart dieser stark wirksamen Katalysatoren überraschenderweise keine Vernetzungsreaktion ein, so daß man auch katalysatorhaltige Mischungen als langzeitlagerstabile Einkomponentensysteme bezeichnen kann.

Die Verarbeitung der erfindungsgemäßen Einkomponentensysteme richtet sich nach deren Beschaffenheit. Flüssige, bei Raumtemperatur gießbare Systeme lassen sich im Gießprozeß verarbeiten, gegebenenfalls werden sie vor der Verarbeitung kurz erwärmt, z. B. auf 50-70° C. Die Verarbeitung kann auch im Schleuderguß erfolgen; Hohlkörper können durch Einbringung der Reaktivmasse in beheizte Formen unter Verteilung an der Oberfläche durch entsprechende Rotationsbewegungen hergestellt werden.

Auch nach dem slush-molding-Process können beheizte Formen mit der Reaktivmasse gefüllt werden und nach einer gewissen Ausheizzeit/Reaktion an der beheizten Formoberfläche wird überschüssige, noch nicht reagierte Reaktionsmasse aus den Formen wieder ausgegossen.

Bei Mitverwendung von Treibmitteln können zellige Polyurethane hergestellt werden, die gegebenenfalls eine integrale Dichtestruktur aufweisen.

Nicht mehr gießbare, aber noch verlaufende Systeme können z. B. mit einer Rakel auf gewünschte

Unterlagen, z. B. textile Unterlagen, z. B. Vliesen, Gewirken und Geweben, (Spalt)leder, Matritzen (z. B. Veloursleder-Silikon-Matrizen), oder Zwischenträger (z. B. Trennpapiere) unter Bildung von Beschichtungen oder Zurichtungen, aufgebracht und anschließend durch Hitzestoß verfestigt werden.

Plastische Systeme (Pasten) können unter Druck- und Formgebung in der Hitze verpreßt werden, wobei bei 120° C bereits 5 bis 15 Minuten zur Verfestigung ausreichend sind.

Auch durch Tauchverfahren können Oberflächenbeschichtungen, Abdruckformen oder Formkörper hergestellt werden, indem man die beheizten, zu beschichtenden Formen in die Reaktivmasse eintaucht.

Die Reaktivmasse kann auch durch Schlitze oder Düsen in heiße Medien (Heißluft oder heiße Flüssigkeiten) ausgepreßt und dadurch verfestigt werden.

Die Reaktivmasse kann in beheizten Extrudern teilweise oder weitgehend zum Polyurethan reagiert werden und in dieser Form durch Schlitze oder Düsen extrudiert und gegebenenfalls in heißen Medien vollständig ausreagiert werden, oder in heißer Form eingebracht werden, woraus sie nach kurzer Zeit entformt werden kann. Auch nach den Reaktionsspritzgußverfahren (reaction-injection-molding - RIM -) kann die Reaktivmasse verarbeitet werden.

Feste Systeme, insbesondere auf Basis höherschmelzender Ausgangspolyole (45 bis 65° C) werden entweder unter Druck und Formgebung (Spritzguß) oder etwa bei oder oberhalb der Schmelztemperatur des Polyols verarbeitet. Man kann so verfahren, daß man die vorher hergestellten Einkomponentensysteme in Form fester Granulate in eine über den Schmelzpunkt des Polyols aufgewärmte Form (im allgemeinen unterhalb 70° C) einbringt. Nach dem Aufschmelzen der Granulate und der damit gegebenen Werkzeugfüllung wird die Form auf 100 bis 120° C erwärmt und der Inhalt verfestigt.

Die Verfestigungstemperatur der erfindungsgemäßen Einkomponentensysteme ist von Menge und chemischer Konstitution der Amine, die zur Stabilisierung der Polyisocyanate A) verwendet wurden, stark abhängig. Mit zunehmender Verfestigungstemperatur nimmt die aufzuwendende Verfestigungszeit zur Bildung der Polyurethane ab. Die Ausheizdauer kann je nach Temperatur von weniger als 1 Minute bis zu mehreren Stunden betragen. Manchmal ist es vorteilhaft, die Kunststoffe nach Entformen noch einige Zeit bei 100° C zu tempern, um eine vollständige Durchhärtung zu gewährleisten.

Eine Aushärtung der Einkomponenten-Reaktivsysteme kann jedoch auch durch Zugabe von vorzugsweise hochpolaren Lösungsmitteln wie Dimethylformamid, N-Methyl-pyrrolidon, oder mäßig polaren Lösungsmitteln wie Propylencarbonat, Dioxan oder Glykolmonomethyletheracetat erfolgen. Je nach Menge dieser Lösungsmittel kann der durch die Aminstabilisatoren verursachte Stabilisierungseffekt der Polyisocyanate in den Einkomponentensystemen zum Teil oder völlig aufgehoben werden. Die Gießzeit (Topfzeit) solcher Ansätze kann dabei durch die Menge solcher Lösungsmittelzusätze gesteuert werden. Bei geringen Mengen erhält man Systeme mit mehrtägiger Topfzeit bei Raumtemperatur, während bei höheren Dosierungen bereits nach 10 bis 15 Minuten schnelle oder gar schlagartige Verfestigung eintritt. Die Zusatzmenge solcher Lösungsmittel ist auch hier von der Menge und Art des Stabilisatoramins (Güte der Polyadduktnaut an der Isocyanatoberfläche) abhängig und wird nach praktischen Vorversuchen für die jeweiligen Systeme festgelegt. Der technische Vorteil solcher Reaktionsmischungen liegt darin, daß diese Systeme auch ohne Zufuhr von wärme verfestigen. Selbstverständlich kann man auch durch geeignete Dosierung der Lösungsmittel die thermische Verfestigungszeit der Einkomponentensysteme erniedrigen, wobei noch eine ausreichende Lagerstabilität gegeben sein kann.

Weiterhin kann eine Verfestigung der erfindungsgemäßen Einkomponentensysteme auch durch Anwendung hoher Scherkräfte herbeigeführt werden, so z. B. in hochtourigen Rührwerken. Die dabei bei kurzzeitigem Rühren auftretende Wärmetönung erreicht in der Regel nicht die Vernetzungs-Aufdickungstemperatur der Einkomponentensysteme, so daß lediglich durch mechanische Beanspruchung während des Rührprozesses die sich auf der Isocyanatteilchenoberfläche befindliche Polyharnstoffhaut zerstört wird.

Weitere Ausführungsformen der Aminstabilisierung unter verschiedenen Bedingungen bzw. Verfahren zur Herstellung der Einkomponenten-Reaktivsysteme und ihre Aushärtung sind auch aus den Beispielen zu entnehmen.

Bevorzugte Verwendung finden solche PU-Reaktivsysteme, welche höhermolekulare Polyamine (B) oder Kettenverlängerungsmittel (C) - vorzugsweise niedermolekulare aromatische Polyamine - als Komponenten enthalten und somit hochwertige Elastomere, Beschichtungen, zellige Elastomere und Formteile mit gegebenenfalls einer Dichteverteilung mit zelligem Innenkern und dichterer Außenhaut ergeben.

## Beispiele

### Beispiel 1

"Stabilisierung" von dimerem Toluylen-2,4-diisocyanat durch unterschüssige Mengen eines "Amin-Stabilisators" bei Suspendierung in verschiedenen Polyolen bzw. Polyaminen und Lagerstabilität entsprechender Mischungen

Die in der Tabelle 1 angegebenen Mengen "Aminstabilisator" (aliphatische Polyamine, Hydrazin oder

"Hydrazid"-Verbindungen) werden in der Polyol- oder Polyamin-Komponente gelöst. Mit einem hochtourigen Mischgerät werden 348 g (1 Mol) dimerisiertes Toluylen-2,4-diisocyanat (TT) in feingemahlener Form (mittlere Teilchengröße 20 $\pm$ 15 µm) suspendiert. Zuletzt wird der Katalysator zugegeben. In Versuch 7 (mit höhermolekularem Polyetherpolyamin) wird ohne Katalysator gearbeitet.

Man entgast unter Rühren bei Raumtemperatur oder leicht erhöhter Temperatur (bis zu 50° C), füllt in eine kalte oder vorgewärmte Gießform und heizt bei 120° C aus. Nach einer Verfestigungszeit von wenigen Minuten und etwa 1 Stunde Nachheizzeit wird entformt.

Lagerversuche werden in Polyethylenflaschen durchgeführt. Die Gießmischungen werden als stabil betrachtet, wenn sie nach längerer Lagerung flüssig bleiben und auch weiterhin bei 120° C (d.h. unter Umsetzung der bislang umhüllten Polyisocyanatanteile) aushärtbar bleiben.

**Tabelle 1**

| Versuch Nr. | g Polyolkomponente | g Aminstabilisator | Äq-% Stabilisator bzgl. TT | Aushärtungskatalysator | Stabilität der Mischung stabilisiert (erfindungsgem.) bei RT | 50°C | unstabilisiert (Vergleich) bei RT | 50°C |
|---|---|---|---|---|---|---|---|---|
| 1a) | 2000 g linearer Polypropylenglykolether, MG=2000 | 3,0 g 2,5-Diamino-2,5-dimethyl hexan | 2,0 | 0,2 g Pb-Octo-at-Lösung*) | >3 Mon | >3 Mon | Vernetzung in wenigen Tagen | Vernetzung in wenigen Stunden |
| 1b) | 2000 g " | 6 g 3,3'-Dimethyl-4,4'-diamino-dicyclohexyl-methan | 2,5 | 0,2 g " | " | " | " | " |
| 1c) | 2000 g " | 1,9 g 1,4-Diamino-cyclohexan | 1,7 | 0,2 g " | " | " | " | " |
| 2 | 2000 g linearer Propylenglykolether mit 20 % endständigen Ethylenoxidgruppen, MG=2000 | 9 g 2,5-Diamino-2,5-diamino-2,5-dimethyl-hexan | 6,0 | 0,2 g " | " | " | " | " |
| 3a) | 2000 g lineares Polyoxytetramethylendiol, MG=2000 | 8 g " | 5,3 | 0,2 g " | " | " | " | " |
| 3b) | 2000 g " | 8 g " | 5,3 | 0,2 g Dioctylzinn-IV-di-(thioester)**) | " | " | " | " |
| 4 | 4000 g trifunktion. Polypropylen-polyethylenglykolether (80/20), OHZ=28, MG=6000 | 3 g 2,5-Diamino-2,5-diamino-2,5-dimethylhexan | 2,0 | 0,2 g Pb-Octoat-Lösung*) | " | " | " | " |
| 5 | 2600 g Dihydroxy-polybutadien, OHZ=43, MG=2600, (R45HT der Fa. Metallges., D-6000, Frankfurt/Main | 3 g " | 2,0 | 0,2 g " | " | " | Vernetzung in wenigen Wochen | Vernetzung in wenigen Tagen |
| 6 | 2000 g lineares Polyethylenglykol-Adipat, MG=2000 | 8 g 2,5-Dimethyl-piperazin | 7,0 | 0,2 g " | " | " | Vernetzung in 1-2 Mon (Hemmung durch festen Zustand des Polyesters) | Vernetzung in wenigen Stunden |
| 7 | 2600 g aromatisches höhermolekulares Poly- | 1,5 g 2,5-Diamino-2,5-dimethyl-hexan | 1,0 | ohne Katalysator | 3 Mon | 3 Mon | Vernetzung nach | Vernetzung in wenigen |

| | | | | | | | | |
|---|---|---|---|---|---|---|---|---|
| | etherpolyamin der NH-Zahl 43 aus PPG-Ether, (OH-Zahl 56) und Toluylen-2,4-diisocyanat gemäß DE-OS 2 948 419 | | | | | | 1 Stunde | Minuten |
| 8 | 2000 g linearer Polyether von Bsp. 1/1 | 0,15 g Hydrazinhydrat | 3,0 | 0,2 g Pb-Octoat-lösung | ″ | ″ | Vernetzung in etwa 1 | Vernetzung in <1 Stunde |
| 9 | 2000 g ″ | 7,2 g β-Amino-propionsäure-hydrazid | 4,5 | ″ | ″ | ″ | ″ | ″ |

**Tabelle 2**

**Mechanische Eigenschaften von ausgeheizten Polyurethanen**

| Bsp. 1/Vers.-Nr- | | 1a) | 1b) | 1c) | 2 | 3a) | 3b) | 4 | 5 | 6 | 7 | 8 | 9 |
|---|---|---|---|---|---|---|---|---|---|---|---|---|---|
| Zugfestigkeit ***) (DIN 53 504) | [MPa] | 1,3 | 1,3 | 12 | 1,4 | 2,2 | 48 | - | 5,7 | 43 | 13 | 1,0 | 1,2 |
| Reißdehnung (DIN 53 504) | [%] | 70 | 70 | 60 | 90 | 60 | 690 | - | 110 | 520 | 40 | 40 | 50 |
| Weiterreißfestigkeit***) nach (DIN 53 515) | [KN/m] | 2 | 2 | 1,9 | 1,6 | 4 | 21 | - | 11 | 10 | 40 | 1,6 | 1,7 |
| Shore-Härte***) (nach DIN 53 505) | -A | 52 | 52 | 52 | 54 | 70 | 93 | - | 79 | 61 | 91 | 51 | 53 |
| | -D | 11 | - | - | 17 | 24 | 29 | - | 28 | 27 | 36 | - | - |
| Elastizität***) (DIN 53 512) | [%] | 53 | 53 | 54 | 52 | 67 | 66 | - | 59 | 50 | 56 | 50 | 54 |
| Druckverformungsrest***) (DIN 53 517) nach 24 h bei 70°C | [%] | 59 | 59 | 60 | 13 | | | | | | | 62 | 58 |

*) Hier und in den folgenden Beispielen wird unter Bleioctoat-Lösung immer eine 57-%-ige Lösung von Pb-II-2-Ethylhexanoat in Waschbenzin (Octa-Soligen-Pb-24; Fa. Borchers, D-4000, Düsseldorf) verstanden.

**) UL 29 der Fa. Witco/USA.

***) Auch in den folgenden Beispielen erfolgt die Bestimmung der Werte nach den angegebenen DIN-Vorschriften.

**Beispiel 2**

"Aufdickungstemperatur" in Abhängigkeit von Art und Menge des "Amin-Stabilisators"

Als "Aufdickungstemperatur" ist diejenige Temperatur definiert, bei der unter langsamem Aufheizen (~10°C/min) die Mischung durch Ausfallen von Hartsegmenten infolge einer Reaktion von festen Polyisocyanat und DETA pastenartige Konsistenz annimmt.

Die "Aufdickungstemperatur" ist u. a. von der Menge und der Art des Aminstabilisators und der Art des Polyisocyanats abhängig.

Es werden solche "stabilisierte Polyisocyanate" für Einkomponenten-PU-Reaktivsysteme bevorzugt, welche eine "Aufdickungstemperatur" von mindestens 55°C aufweisen. Zur Bestimmung der "Aufdickungstemperatur"

im "DETA-Test" siehe Rezeptur S. 50.

Darunterliegende "Aufdickungstemperaturen" zeigen erfahrungsgemäß eine ungenügende "Stabilisierung" des Polyisocyanats durch Umhüllung mit Reaktionsprodukten mit den Amin-Stabilisatoren bei Verwendung für lagerstabile Einkomponentensysteme an.

Ist die Menge des Aminstabilisators zu groß, so werden Polyisocyanate erhalten, die zwar eine weiter erhöhte "Aufdickungstemperatur" zeigen, jedoch infolge der höheren Stabilisatorkonzentration keine einwandfreie Formteilaushärtung zum fertigen Polyurethan mehr zeigen.

**"DETA-TEST"**

Rezeptur:

1 mol linearer Polypropylenglykolether, MG = 2000
X Äqu.-% "Aminstabilisator"
2 mol dimeres, feinteiliges Toluylendiisocyanat ("TT"), mittlere Teilchengröße 20 ± 15 µm,
1 mol 2,4-/2,8-Diamino-3,5-diethyltoluol-Isomerengemisch (65/35) ("DETA")
0,2 g Bleioctoat-Lösung auf 100 g Polyol

Allgemeine Versuchsbeschreibung:

Der "Aminstabilisator" wird im Polyol gelöst und das TT in dieser Mischung suspendiert. Nach 30 Minuten Reaktionszeit bei Raumtemperatur wird zu dem "stabilisierten" Diisocyanat das DETA und der Katalysator gegeben. Die Aufdickungstemperatur wird wie oben angegeben bestimmt.

Anstelle des TT können auch die anderen ≥30° schmelzenden Polyisocyanate verwendet werden. Gegebenenfalls können auch isolierte, vorab stabilisierte Polyisocyanate anstelle von Aminstabilisator plus Polyisocyanat eingesetzt werden.

**Tabelle 3**

| Vers.-Nr. | Art des "Aminstabilisators" | Menge des Stabilisators [Äq.-%], bezogen auf "TT" 0,5 % | 1,0 % | 1,5 % | 3 % |
|---|---|---|---|---|---|
| | | Aufdickungstemperaturen in °C | | | |
| 1 | Ethylendiamin | RT*) | 60 | 80 | |
| 2 | Diethylentriamin | " | 90 | 150 | |
| 3 | 2,5-Diamino-2,5-dimethylhexan | " | RT*) | 60 | |
| 4 | Neopentandiamin | " | 80 | 130 | |
| 5 | 1,6-Diamino-2,4,4-trimethylhexan | 80 | 105 | 120 | |
| 6 | Bis-aminomethylhexahydro-4,7-methano-indan (TCD-Amin) | RT*) | 85 | 110 | |
| 7 | Isophorondiamin | " | 70 | 85 | |
| 8 | 2,4-Diamino-methyl-cyclohexan | " | 70 | 90 | |
| 9 | 4,4'-Diamino-dicyclohexylmethan | 75 | 85 | 90 | |
| 10 | 3,3'-Dimethyl-4,4'-diamino-dicyclohexylmethan | 70 | 70 | 75 | |
| 11 | 1,4-Diamino-cyclohexan | RT*) | RT*) | 85 | |
| 12 | 2-(2-Amino-ethylamino)-ethanol | " | 75 | 115 | |
| 13 | 1-Amino-3-cyclohexylaminopropan | 90 | 100 | 105 | |
| 14 | N,N'-Dimethyl-ethylendiamin | RT*) | 70 | 80 | |
| 15 | 2,5-Dimethylpiperazin | " | RT*) | 85 | |
| 16 | Triamino-polypropylenether (Jeffamine T-403, MG = 438 von Texaco Chemical Comp./USA) | 85 | 120 | 130 | |
| 17 | Hydrazin(hydrat) | RT*) | RT*) | RT*) | 60 |
| 18 | ohne (Vergleichsversuch) | augenblickliche Aufdickung nach DETA-Zugabe und Vernetzung innerhalb einer Woche bei Raumtemperatur | | | |

*) "Aufdickung" bereits bei Raumtemperatur kennzeichnet eine in diesem Fall nicht ausreichende "Stabilisierungswirkung" (Umhüllungsreaktion) durch den "Stabilisator" (aliphatische Polyamine etc.).

**Beispiel 3**

Geschwindigkeit der Umhüllungsreaktion eines festen Diisocyanats mit unterschiedlichen aliphatischen Diaminen a)-d)

In 200 g eines linearen Polypropylenglykolethers des Molekulargewichts 2000 werden 70 g dimerisiertes Toluylen-2,4-diisocyanat (TT) suspendiert. In jeweils derart hergestellten Isocyanatsuspensionen werden für die "Umhüllungsreaktion" von TT jeweils optimierte Mengen aliphatischer Diamine a)-d) bei Raumtemperatur eingerührt. In bestimmten Zeitabständen werden Proben entnommen. Nach Abfiltrieren vom schwerlöslichen Isocyanat wird nach Versetzen mit Aceton, Wasser und Phenolrot der verbliebene Restamingehalt im Filtrat mit 0,1 n HCl titriert.

**Tabelle 4**

| Aminstabilisator | Äq-% | Restmenge des Aminstabilisators in % der Ausgangsmenge nach Zeit(Stunden) | | | | | |
|---|---|---|---|---|---|---|---|
| | | 0,1 | 0,3 | 1 | 3 | 10 | 30 |
| a) 0,6 g 2,5-Diamino-2,5-dimethylhexan | 2,0 | 80 | 53 | 35 | 28 | 22 | 18 |
| b) 0,2 g Ethylendiamin | 1,67 | 55 | 27 | 18 | 13 | 10 | 6 |
| c) 0,34 g höhermolekulares, trifunktionelles, aliphatisches Polyamin (Jeffamine T-403, vgl. Bsp. 2, Versuch Nr. 16) | 0,2 | 35 | 25 | 21 | 17 | 15 | 8 |
| d) 1,2 g 3,3'-Dimethyl-4,4'-diamino-dicyclohexylmethan | 2,5 | 85 | 65 | 44 | 31 | 27 | 12 |

Es zeigt sich also, daß auch nach längerer Reaktionszeit ein gewisser Rest an Polyamin bleibt, der nur sehr langsamt in dem Reaktionsmedium durch die Polyharnstoffumhüllung weiterreagieren kann.

**Beispiel 4**

Externe Herstellung umhüllter Polyisocyanate in organischen Lösungsmitteln und anschließende Verwendung in Einkomponenten-Polyurethan-Reaktionsmischungen mit aromatischen Diamin-Kettenverlängerungsmitteln

Zu einer Lösung von 0,5 g Ethylendiamin in 100 g Chlorbenzol, Toluol bzw. Ligroin werden jeweils 50 g TT in feinpulverisierter Form zugesetzt und die heterogene Mischung 1 Stunde bei Raumtemperatur gerührt. Das unter diesen Reaktionsbedingungen stabilisierte (umhüllte) Isocyanat wird dann abgesaugt, mit dem obigen Lösungsmittel gewaschen und vorsichtig vakuumgetrocknet.

34,8 g dieses Isocyanates werden nunmehr in 100 g eines linearen Polypropylenglykolethers (Molekulargewicht 2000) suspendiert und danach werden 8,9 g 2,4-/2,6-(65/35)-Diamino-3,5-diethyltoluol-Isomerengemisch und 0,2 g Bleioctoat-Lösung zugesetzt. In allen drei Fällen erfolgt bei Raumtemperatur ein starker Viskositätsaufbau infolge Vorreaktion der noch nicht ausreichend stabilisierten (stabil umhüllten) Isocyanate mit dem aromatischen Diamin (DETA). Der Grund der unzureichenden Stabilisierung könnte in einer zu brüchigen, nicht elastischen Umhüllungsform des gebildeten Polyharnstoffs liegen.

Setzt man in den Lösungsmitteln jedoch außer den aliphatischen Di- oder Polyaminen zusätzlich verschiedene Verbindungen in Kombination ein, so lassen sich mit diesen Hilfsmitteln und aliphatischen Di- und Polyaminen jedoch gute Stabilisierungseffekte erzielen. Dies könnte auf einer elastizifizierenden Wirkung der Hilfsmittel beruhen, die somit eine stabilere Umhüllung ergeben. Die Hilfsmittel können dabei sowohl gegenüber NCO reaktive Gruppen aufweisende Verbindungen sein, z. B. OH-funktionelle Polyether oder aromatische Polyaminopolyether, aber auch höhermolekulare, weichmacherähnliche Verbindungen ergeben eine gute Wirkung.

Reaktionsansatz:

| | |
|---|---|
| 80 | g Ligroin |
| x | g Hilfsmittel |
| 0,5 | g Ethylendiamin |
| 34,8 | g TT |

werden 1 Stunde bei Raumtemperatur gerührt.

Das so stabilisierte "TT" wird abgesaugt, getrocknet und in 100 Teilen eines Polypropylenethers vom Molekulargewicht 2000 (linearer Aufbau) eingerührt. In diese Suspension werden 8,9 g DETA und 0,2 g Bleioctoatlösung eingerührt.

**Tabelle 5**

| Hilfsmittel x | Menge (g) | Aufdickungspunkt |
|---|---|---|
| Polypropylenglykolether, linear, MG 2000 | 15 | 130°C |
| Polypropylenglykolether, linear, MG 1000 | 15 | 128°C |
| aromatische Aminopolyether, MG 2000, hergestellt nach DE-OS-29 48 419 | 15 | 138°C |
| Tetrol aus Ethylendiamin und Propylenoxid, OH-Zahl 630, MG 355 | 10 | 120°C |
| Tetrol aus Ethylendiamin und Propylenoxid, OH-Zahl 470, MG 420 | 10 | 128°C |
| Dioctylphthalat (Weichmacher) | 20 | 100°C |

Wie die Beispiele zeigen, bildet sich in Gegenwart der Weichmacher oder der Co-Reaktanten als Hilfsmittel eine wirksame Umhüllung um das Isocyanat.

Verwendet man anstelle des Ethylendiamins einen höhermolekularen, aliphatischen Aminopolyether (Jeffamine D 2000, Molekulargewicht 2000, gemäß US-30 54 370), so wird ein deutlich verbesserter Effekt beobachtet (Bildung eines elastischeren Umhüllungsmaterials). In den meisten Fällen muß zur Erzielung eines gleichartigen Stabilisierungseffektes wie bei der in situ-Stabilisierung direkt innerhalb des Polyols eine etwas größere Menge an solchen aliphatischen, höhermolekularen Polyaminen eingesetzt werden, um eine ausreichende Wirkung zu erzielen.

Anstelle der höhermolekularen Jeffamine können auch Kombinationen von aliphatischen niedermolekularen Aminen und/oder höhermolekularen aliphatischen Diaminen und höhermolekularen Polyolen und/oder aromatischen Polyaminen verwendet werden, wobei die Beeinflussung durch das Lösungsmittel (infolge von Quellvorgängen oder Diffusionen) beachtet werden muß.

Folgende zwei Beispiele zeigen diesen Lösungsmitteleffekt, der von der Güte der Umhüllung, d.h. der Art und Menge der aliphatischen Diamine (Stabilisierungseffekt) bestimmt wird.

Ansatz:

| | |
|---|---|
| 80 | g Lösungsmittel (Toluol oder Ligroin) |
| 15 | g linearer Polypropylenglykolether, MG 2000 |
| 0,2 | g Ethylendiamin |
| 34,8 | g TT/8,9 g 2,4/2,6(65/35)-Diamino-2,5-diethyl-toluol (DETA) |
| 0,2 | g Bleioctoat-Lösung. |

Wird der Reaktionsansatz in Toluol als Lösungsmittel durchgeführt, so beobachtet man eine alsbaldige Aufdickung des Ansatzes, während sich der in (dem schlechteren Lösungsmittel) Ligroin durchgeführte Ansatz als lagerstabil erweist.

**Beispiel 5**

Externe Herstellung stabilisierter Isocyanate

Allgemeine Bemerkung:

Man kann die externe Stabilisierung eines Isocyanats mit aliphatischen Polyaminen auch in Gegenwart von Wasser durchführen, wenn in Kombination mit einem nieder- oder höhermolekularen Polyol und/oder einem aromatischen nieder- oder höhermolekularen Polyamin gearbeitet wird. Verzichtet man auf diesen Polyolzusatz, so muß man in reinem Wasser als Lösemittel mehr Aminstabilisator verwenden, was jedoch zumeist zu einem zu starken NCO-Verbrauch führt.

Durchführungsbeispiel

Zu einer Lösung von 20 g eines Polyols der in Tabelle 6 aufgeführten Konstitution in 100 ml Wasser werden 1,0 g Ethylendiamin (0,0167 Mol / 7 Äquivalent-%) zugesetzt. Danach erfolgt der Zusatz von 50 g 1,5-Diisocyanatonaphthalin (0,238 Mol; Teilchengröße 20-50 µm). Eine Gasentwicklung tritt bei einer Rührzeit von 1 bis 2 h bei Raumtemperatur nicht ein. Das modifizierte Isocyanat wird abgesaugt und im Vakuum getrocknet.

21 g (0,1 Mol) des so modifizierten Isocyanates werden in 100 g (0,05 Mol) eines linearen Polypropylenglykolethers des Molekulargewichts 2000 suspendiert und dem heterogenen Ansatz eine Lösung von 0,2 g Bleioctoat in 8,9 g (0,05 Mol) 2,4-/2,6-(65/35)-Diamino-3,5-di-ethyltoluol-Isomerengemisch zugesetzt.

Während bei einem Vergleichsversuch mit 1 g Diamin (ohne Zusatz von Polyol) nach kurzer Zeit eine starke Aufdickung beobachtet wird, bleiben die erfindungsgemäßen Ansätze flüssig und zeigen folgende Aufdickungspunkte (s. Tabelle 6).

**Tabelle 6**

| | Polyol (20g/100 g $H_2O$) | Ethylendiamin [g] | Aufdickungspunkt |
|---|---|---|---|
| 1 | ohne Polyol (Vergleich) | 1,0 | Raumtemperatur (ca. 2-3 min) |
| 2 | Tetrol aus Ethylendiamin und Propylenoxid der OH-Zahl 770/MG = 290 | 1,0 | 125°C |
| 3 | Triol aus Trimethylolpropan und Propylenoxid, OH-Zahl 850 | 1,0 | 105°C |
| 4 | ohne Polyol | 3,0 | 56°C |

**Beispiel 6**

Stabilisierungsvergleiche in verschiedenen Solventien (Lösungsmittel/Weichmacher/Hydroxyverbindungen)

Zu einer Lösung von 0,2 g Ethylendiamin (0,0033 Mol; 3,3 Äquivalent-%) in jeweils 80 g der unten angegebenen Solventien werden 34,8 g (0,1 Mol) des aus 2,4-Diisocyanatotoluol durch Dimerisierung hergestellten Uretdiondiisocyanates in einer Teilchengröße von 1 bis 30 µm zugesetzt. Die heterogene Mischung wird 30 bis 60 Minuten bei Raumtemperatur gerührt.

Die stabilisierte Isocyanat-Suspension wird mit 8,9 g (0,05 Mol) 2,4/2,6-(65/35)-Diamino-3,5-diethyl-toluol-Isomerengemisch (DETA) vermischt.

Bei unzureichender Stabilisierung, d.h. unvollständiger oder poröser Umhüllung der Isocyanatoberfläche durch den Polyharnstoff (aus der Reaktion von Isocyanatgruppen und Ethylendiamin) tritt nach kurzer Zeit starker Viskositätsaufbau ein. Man erhält infolge der Vorreaktion des aromatischen Diamins mit dem Diisocyanat eine Reaktionsmischung von pastenartiger Konsistenz (vgl. Beispiele 1-8 in Tabelle 7; nicht erfindungsgemäß).

Bei vollständiger Stabilisierung des Isocyanats gegenüber den aromatischen Aminogruppen bleiben die Reaktionsgemische dagegen flüssig und mindestens 3 Monate bei Raumtemperatur oder wenig erhöhten Temperaturen lagerstabil. Als Maß für die Güte der "Stabilisierung" kann die Aufdickungstemperatur gemessen werden. Bei dem in einem nur engen Temperaturbereich liegenden "Aufdickungspunkt" wird die Stabilisierung der Isocyanate aufgehoben (völlige oder teilweise Zerstörung der Polyharnstoff-Umhüllung).

Zur Messung der Aufdickungstemperatur der jeweiligen Systeme wurden 10 bis 20 g-Proben der jeweiligen Mischungen langsam bis zur schlagartigen Aufdickung erwärmt. Je höher dieser Aufdickungspunkt liegt, desto stabiler verhält sich das nach dem erfindungsgemäßen Verfahren stabilisierte Isocyanat gegenüber Verbindungen mit reaktiven Wasserstoff tragenden Gruppen.

**Tabelle 7**

| Lösungsmittel | Funktionalität (gegenüber NCO-Gruppen) | Molekular-gewicht | Aufdickungspunkt °C | |
|---|---|---|---|---|
| 1. Toluol | 0 | | ≦Raumtemperatur | nicht erfindungsgem. |
| 2. Chlorbenzol | 0 | | " | " |
| 3. Petrolether | 0 | | " | " |
| 4. Ligroin | 0 | | " | " |
| 5. Dioxan | 0 | | " | " |
| 6. Methyl-glykoletheracetat | 0 | | " | " |
| 7. Glykol-dimethylether | 0 | | " | " |
| 8. Phosphorsäuretributylester | 0 | | " | " |
| 9. Dioctylphthalat | 0 | 390 | 80 | erfindungsgemäß |
| 10. Polybutadien (Lithene PM (Fa. ARCO/USA) | 0 | 1000 | 110 | " |
| 11. Ethylenglykol | 2 | | 103 | " |
| 12. N-Methyl-diethanolamin | 2 | | 82 | " |
| 13. Isohexadecanol | 1 | | 62 | " |
| 14. *)Trimethylolpropan + Propylenoxid, OH-Zahl 550 | 3 | 300 | 140 | " |
| 15. *)Ethylendiamin + Propylenoxid, OH-Zahl 470 | 4 | 480 | 130 | " |
| 16. *)Butanol + Propylenoxid | 1 | 1400 | 130 | " |
| 17. *)Butanol + Propylenoxid | 1 | 2000 | 130 | " |
| 18. linearer Propylenglycolether, 15 % Ethylenoxid-Endgruppen, OH-Zahl 28 | 2 | 4000 | 140 | " |
| 19. " OH-Zahl 56 | 2 | 2000 | 143 | " |
| 20. " OH-Zahl 112 | 2 | 1000 | 130 | " |
| 21. " OH-Zahl 35 | 2 | 3200 | 135 | " |
| 22. trifunktion. Polypropylen-ethertriol, OH-Zahl 28 | 3 | 6000 | 125 | " |
| 23. Rizinusöl | 3 | ca. 1000 | 115 | " |
| 24. Polyethylenglykolether | 2 | 400 | 105 | " |

*) Polyether, hergestellt aus

Die Stabilisierung der Isocyanate durch Ethylendiamin ist in den angegebenen Lösungsmitteln 1-8 infolge poröser, brüchiger und quellbarer Polyharnstoffumhüllung unzureichend. Die Aufdickung in Einkomponenten-Polyurethansystemen erfolgt kurz nach Zugabe des aromatischen Diamins.

Erst bei Verwendung von höhermolekularen Verbindungen, vorzugsweise aus der Gruppe der Kohlenwasserstoffe, Ether oder Ester (z. B. Weichmacherverbindungen 9 oder 10) und insbesondere bei Mono- oder Polyhydroxyverbindungen (z. B. Verbindungen 11 bis 23 in Tabelle 7) ist ein deutlicher Stabilisierungseffekt der Isocyanate erkennbar (Bildung einer elastischen, schwer zerstörbaren Polyharnstoffumhüllung). Zu stark polare Weichmacher mit z. B. Phosphorestergruppierungen zeigen eine offensichtlich zu stark lösende Wirkung und verhindern eine wirksame "Umhüllung" der Polyisocyanate. Verwendet man mehr Aminstabilisator, wird auch eine ausreichende Umhüllung erzielt, jedoch wird ein zu hoher NCO-Verlust konstatiert und das Material wird inhomogen/klumpig.

**Beispiel 7**

<u>Verwendung stabilisierter Polyisocyanate in Einkomponenten-Polyurethanrezepturen mit verschiedenen aromatischen Diaminen als Kettenverlängerungsmittel</u>

Rezeptur:

| | |
|---|---|
| 1 | Mol lineares Polypropylenglykoletherdiol, Molekulargewicht 2000 (als höhermolekulares Polyol) |
| 0,02-0,03 | Mol "Amin-Stabilisator" (2,5-Diamino-2,5-dimethylhexan) |
| 2 | Mol dimeres Toluylendiisocyanat (TT) |
| 1 | Mol aromatisches Diamin (s. Tabelle 8) |
| 0,2 | g Bleioctoat-Lösung pro 100 g Polyol |

Allgemeine Versuchsbeschreibung:

35 Teile dimeres Toluylendiisocyanat werden in 40 Teilen des höhermolekularen Polyols suspendiert. Nach Zugabe der in der Tabelle 8 angegebenen Menge "Amin-Stabilisator" läßt man 30 Minuten bei Raumtemperatur unter Bildung des stabilisierten Diisocyanats reagieren.

Nach Zugabe des aromatischen Amins, gelöst und/oder suspendiert in 60 Teilen höhermolekularem Polyol und des Katalysators entgast man unter Rühren bei Raumtemperatur, füllt die Mischung in eine kalte oder leicht vorgewärmte Gießform und heizt bei 120°C aus. Nach wenigen Minuten Verfestigungszeit und einer Stunde Nachheizzeit wird entformt.

Die nicht ausgeheizten Mischungen besitzen bis zu 50°C eine praktisch unbegrenzte Lagerfähigkeit (≧4 Monate). Wird dagegen in der obigen Rezeptur das unstabilisierte dimere Toluylendiisocyanat (ohne Verwendung der aliphatischen Diamin-Stabilisierung) eingesetzt, so tritt eine Aufdickung der Einkomponenten-PU-Mischung bei Raumtemperatur innerhalb weniger Minuten ein; die Durchhärtung schließt sich direkt an.

**Tabelle 8**

PU-Einkomponenten-Rezeptur und Eigenschaften

| Vers.-Nr. | 1 | 2 | 3 | 4 | 5 |
|---|---|---|---|---|---|
| aromat.Diamin (als Kettenverlängerer) | 2,4/2,6(65/35) -Diamino-3,5-diethyltoluol-Isomerengem. | 2,4-Diamino-toluol | 4,4'-Diamino-diphenylmethan | 3,5-Diisopropyl-3',5'-Diethyl-4,4'-diamino-diphenylmethan | 1,5-Diamino-naphthalin |
| Gew.-Teile arom.Diamin | 8,9 | 6,1 | 9,9 | 16,9 | 7,9 |
| arom. Diamin dosiert als | Lösung | Lösung mit suspendiertem Anteil in 60 Teilen Polyol | Lösung | Suspension | Suspension |
| Tle. aliphatischer Amin-Stabilisator auf 100 Tle. Polyol | 0,4 | 0,3 | 0,4 | 0,3 | 0,3 |
| Äq.-% bezgl. TT | 3 | 2,25 | 3 | 3,35 | 2,25 |
| Aufdickungstemp. der Mischung in °C | 68 | 85 | 93 | 85 | 95 |
| Temp., bei der in 1 min Verdickung (Aushärtung) eintritt [°C] | 100 | 108 | 108 | 103 | 102 |
| Mechanische Eigenschaften: | | | | | |
| Zugfestigkeit [MPa] | 8,9 | 7,6 | 9,3 | 11,5 | 9,2 |
| Reißdehnung [%] | 150 | 140 | 110 | 260 | 160 |
| Weiterreißfestigkeit [KN/m] | 11,0 | 12 | 11,4 | 15 | 9 |
| Shore-Härte -A | 91 | 81 | 83 | 86 | 77 |
| Shore-Härte -D | 34 | 29 | 31 | 32 | 26 |
| Elastizität [%] | 46 | 47 | 48 | 45 | 53 |

**Beispiel 8**

Stabilisierte Einkomponenten-PU-Systeme auf Polyesterbasis unter Verwendung verschiedener Katalysatoren

A) Zu 500 g eines bei ca. 55°C aufgeschmolzenen linearen Polyesterdiols aus Adipinsäure und Ethylenglykol (Molekulargewicht 2000) werden 2 g (0,0175 Mol) 2,5-Dimethyl-piperazin zugesetzt (7 Äquivalent-%, bezogen auf NCO). Anschließend werden 87 g (0,25 Mol) dimeres 2,4-Diisocyanatotoluol (TT) in Form eines feingemahlenden Pulvers (10-30 µm) und 1 g einer 50-%-igen Bleioctoat-Lösung in Benzin eingerührt.

Nach kurzem Entgasen der Reaktionsmischung wird die Schmelze in eine Form gegossen, die bei 120°C ausgeheizt wird. Bei einer Verfestigungszeit von wenigen Minuten und ca. 1 bis 2 Stunden Nachheizzeit wird ein transparentes, hochelastisches Material mit den in Tabelle 9 angegebenen mechanischen Eigenschaften erhalten.

B) Werden in Versuch A) anstelle von 2,0 g Bleioctoat-Lösung 2,0 g Fomrez UL 29 (S-haltiger Sn-Katalysator der Fa. Witco/USA) verwendet, so erhält man unter gleichen Arbeitsbedingungen einen transparenten, aber deutlich steiferen Formkörper mit in Tabelle 9 angegebenen Eigenschaften.

C) wird der in Beispiel 8, Versuch A), erwähnte Polyester durch einen linearen Polyester aus Adipinsäure und einem Ethylenglykol/Butandiol-1,4 (1 : 1)-Gemisch (OH-Zahl 56) ersetzt, so wird bei gleicher Arbeitsweise entsprechend A) ein Formkörper mit den in Tabelle 9 angegebenen mechanischen Eigenschaften erhalten.

D) Die Katalyse mit Fomrez UL 29 anstelle von Bleioctoat führt bei der Rezeptur C) ebenfalls zu einem transparenten, aber deutlich steiferen Elastomer.

**Tabelle 9**
Mechanische Eigenschaften

| | A | B | C | D |
|---|---|---|---|---|
| Zugfestigkeit [Mpa] | 35 | 36 | 10,5 | 37 |
| Reißdehnung [%] | 470 | 780 | 334 | 860 |
| Weiterreißfestigkeit [KN/m] | 11,3 | 52 | 7,5 | 45 |
| Shore-Härte -A | 65 | 82 | 69 | 83 |
| Shore-Härte -D | 18 | 29 | 21 | 28 |
| Elastizität [%] | 54 | 58 | 65 | 62 |

Heizt man die Versuche A) bis D) nicht direkt zum Polyurethan aus, sondern läßt den Reaktionsansatz von ca. 55 bis 60°C wieder erkalten, so werden lagerstabile Einkomponenten-Polyurethansysteme in Form von festen Granulaten oder hochviskosen Pasten erhalten.

Nach beliebig langer Zeit können diese Systeme in eine auf ca. 70 bis 100°C geheizte Form gebracht werden. Nach dem Aufschmelzen (60-80°C) verteilt sich die nunmehr dünnflüssige Schmelze innerhalb der Form, wobei die Fließcharakteristik von der Formtemperatur bestimmt wird. Die Endaushärtung erfolgt schließlich bei einer Temperatur von 110-120°C.

Man kann daher diese Einkomponentensysteme im Gießverfahren verarbeiten, wenn die Verarbeitungstemperatur oberhalb der Schmelztemperatur des Polyesters liegt. Zu diesem Zweck empfiehlt es sich, die lagerstabile Reaktionsmischung vor der Verarbeitung aufzuschmelzen (50-70°C), zu entgasen, anschließend in gewünschte vorgewärmte Formwerkzeuge zu gießen und die Formen bei 110-120°C auszuheizen. Nach wenigen Minuten Verfestigungszeit und 30-60 Minuten Temperzeit sind die hochelastischen Elastomeren entformbar.

**Beispiel 9**

Versuch A)

Zu 500 g eines linearen Polyesters aus Adipinsäure und Ethylenglykol mit der OH-Zahl 56 (Molekulargewicht 2000) werden bei etwa 50 bis 70°C 2,0 g (0,0175 Mol) 2,5-Dimethylpiperazin als "Amin-Stabilisator" (4,66 Äq.-%) zugesetzt. Anschließend werden 131 g (0,376 Mol) eines Uretdiondiisocyanats auf Basis 2,4-Diisocyanatotoluol in Form eines feingemahlenen Pulvers (10-40 µm) eingerührt.

Nach wenigen Minuten Rühren werden der stabilisierten Diisocyanatsuspension 22,25 g (0,125 Mol) 2,4-/2,6-(65/35)-Diamino-3,5-diethyltoluol-Isomerengemisch und 1 g Bleioctoat-Lösung zugesetzt. Nach kurzem Entgasen der Reaktionsmischung wird die Schmelze in eine Form gegossen, die bei 120°C ausgeheizt wird. Nach einer Verfestigungszeit von wenigen Minuten und nach ca. 1 bis 2 Stunden Nachheizzeit wird ein hochelastisches Material mit den in Tabelle 10 angegebenen Eigenschaften erhalten.

Versuch B) (härtere Einstellung)

500 g linearem Polyester aus Versuch A) werden 2,5 g (0,022 Mol) 2,5-Dimethylpiperazin als "Amin-Stabilisator" (4,4 Äq.-%) zugesetzt. Anschließend werden 174 g (0,50 Mol) des Uretdiondiisocyanates von A) als feingemahlenes Pulver eingerührt.

Nach wenigen Minuten Rühren werden in die stabilisierte Polyisocyanatsuspension 44,5 g (0,25 Mol) des aromatischen Diamins aus Versuch A) und 1 g des schwefelhaltigen Zinn-IV-Katalysators UL-29 (Fa. Witco) zugegeben. Die Schmelze wird nach kurzem Entgasen der Reaktionsmischung in eine Form gegossen und bei 120° C/1,5 h ausgeheizt, wobei ein hochelastisches Material erhalten wird (vgl. Tabelle 10).

Wiederholt man den Versuch ohne "Amin-Stabilisator", so tritt in Gegenwart des Katalysators bereits Aufdickung und Vernetzung nach ca. 30 Minuten bei 50° C ein.

Erfindungsgemäß mit "Amin-Stabilisator" ist das Reaktionsgemisch bei 60° C nach 18 Stunden praktisch unverändert und zur Auslösung der Polyurethanreaktion ist ein Aufheizen auf 120° C erforderlich.

**Tabelle 10**

Mechanische Eigenschaften

| Vers.-Nr. | 9 A) | 9 B) |
|---|---|---|
| Zugfestigkeit [Mpa] | 40 | 34 |
| Reißdehnung [%] | 475 | 620 |
| Weiterreißfestigkeit [KN/m] | 20,5 | 80 |
| Shore-Härte -A | 81 | 92 |
| Shore-Härte -D | 32 | 42 |
| Elastizität [%] | 40 | 48 |

**Beispiel 10**

"Stabilisiertes" Naphthylendiisocyanat und ein entsprechendes Einkomponenten-Polyurethan-Reaktivsystem

Allgemeine Rezeptur:

100 g (0,05 Mol) eines linearen Polypropylenglykoldiols, Molekulargewicht 2000,
x g Ethylendiamin
21 g (0,01 Mol) Naphthylen-1,5-diisocyanat (mittlere Teilchengröße 20 µm ± 10 µm)
8,9 g (0,05 Mol) aromatisches Diamin DETA und
0,2 g Bleioctoat-Lösung.

Das mit unterschiedlichen Mengen (x) an Ethylendiamin versetzte Polyetherdiol wird durch Zugabe des Diisocyanats in eine stabilisierte Diisocyanatsuspension überführt und anschließend mit aromatischen Diaminen und Katalysator vermischt. Die Aufdickungspunkte in Abhängigkeit von der Stabilisatormenge werden in Tabelle 11 wiedergegeben.

**Tabelle 11**

Aufdickungspunkte in Abhängigkeit von der Stabilisatormenge

| Ethylendiamin g/100 g Polyether | Äq.-% | Aufdickungspunkt (° C) |
|---|---|---|
| 1,0 | 16,6 | 128 |
| 0,75 | 12,5 | 127 |
| 0,60 | 10,0 | 125 |
| 0,5 | 8,3 | 124 |
| 0,2 | 3,33 | Raumtemperatur (~5 min) |
| ohne | - | Raumtemperatur (~1-3 min) |

**Beispiel 11**

Stabilisierung eines Harnstoffdiisocyanates und Verwendung in Einkomponenten-PU-Reaktivmischungen

Rezeptur:

50 g (0,025 Mol) eines linearen Polypropylenoxiddiols mit 20 % endständigen Ethylenoxidgruppen, Molekulargewicht 2000
16 g (0,045 Mol) Harnstoffdiisocyanat
50 g aromatischer Aminopolyether auf Basis eines Polypropylenoxid-etherdiols und Toluylendiisocyanat (s.u.)
0,4 g N,N'-Dimethyl-ethylendiamin und
0,2 g Zinnkatalysator.

Zu einer Suspension von 16 g des aus 2 Mol 2,4-Diisocyanatotoluol und 1 Mol Wasser in Acetonlösung gewonnenen 4,4'-Diisocyanato-3,3'-dimethyl-diphenylharnstoffs (Fp. 180° C, Harnstoffdiisocyanat/THDI) in 50 g eines Polypropylenoxiddiols werden 50 g des Aminopolyethers zugemischt. Danach erfolgt der Zusatz von 0,4 g N,N'-Dimethylethylendiamin und 0,2 g Dibutylzinn-IV-2-ethyl-hexanoat. Diese Mischung ist bei Raumtemperatur praktisch unbegrenzt lagerstabil. Nach dem Ausheizen bei 120-140° C, 30-60 min, erhält man einen elastischen Formkörper einer Shore-A-Härte von 75 bis 80.
Ohne Amin-Stabilisierung dickt der Reaktionsansatz bei Raumtemperatur allmählich auf.

Harnstoffdiisocyanat: (THDI)

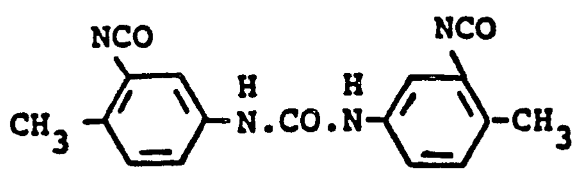

Aminopolyether:
Die Herstellung erfolgte nach Beispiel 2 - Verfahren A - der DE-OS-3 039 600 durch Alkali-Hydrolyse eines linearen NCO-Prepolymers aus einem linearen Polyoxypropylenetherdiol (Molekulargewicht 2000) und 2,4-Toluylendiisocyanat (Molverhältnis 1 : 2) und Carbamatzersetzung mittels eines sauren Ionenaustauschers. Das aromatische Diamin zeigt eine Molmasse von 2400 und einen NH-Wert von 41,5.

**Beispiel 12**

Stabilisierung weiterer Diisocyanate
Zu 100 g eines linearen Polypropylenoxiddiols (Molekulargewicht 2000) werden die unten angegebenen Mengen an Stabilisatordiamin (Ethylendiamin) und anschließend die genannten Diisocyanate in Form feiner Pulver zugesetzt.
Man kann auch niedrig schmelzende Diisocyanate (z. B. 4,4'-Diisocyanatodiphenylmethan - Fp. 40° C - oder andere Diisocyanate, z. B. 1,4-Diisocyanatobenzol (Fp. 95° C)) in feinverteilte Form bringen, wenn die entsprechenden Isocyanate (100 g) aufgeschmolzen und in kalten (0-4° C) Petrolether (300 g) unter intensivem Rühren rasch eingetragen werden. Die festen Isocyanate fallen hierbei in Form eines gleichmäßig feinen Pulvers aus.
Nach Abmischung des Polypropylenoxidethers mit Ethylendiamin und einem der angegebenen Diisocyanate werden der stabilisierten Suspension 0,2 g Bleioctoatlösung und 8,9 g des aromatischen Diamins (DETA) zugesetzt und der Aufdickungspunkt der so hergestellten Reaktivmischungen bestimmt (s. Tabelle). Ohne Stabilisierung erfolgt rasche Aufdickung und man erhält eine nicht mehr verarbeitbare Paste.

**Tabelle 12**

| | Diisocyanat | Ethylendiamin (g/100 g PE) | Aufdickungspunkt (° C) |
|---|---|---|---|
| 1. | 4,4'-Diisocyanato-3,3'-dimethyl-diphenylharnstoff | 0,2 | 142 |
| 2. | 1,4-Diisocyanatobenzol | 0,6 | 125 |
| 3. | 4,4'-Diisocyanato-diphenylmethan | 0,8 | 95 |

**Beispiel 13**

Verwendung von stabilisierten Diisocyanaten in Polyurethanreaktivsystemen mit Polyolvernetzung

Versuchsteil A) Vernetzung mit Polyolen

Zu einer Lösung von 0,5 g Ethylendiamin in 100 g (0,05 Mol) eines linearen Polypropylenoxidetherdiols (Molekulargewicht 2000) werden 31,5 g (0,15 Mol) feingemahlene (mittlere Teilchengröße 20 ± 10 µm) 1,5-Diisocyanatonaphthalin zugesetzt. Anschließend erfolgt eine Zugabe von 0,1 Mol der unten in Tabelle 13 angeführten niedermolekularen Polyole und 0,2 g Bleioctoat-Lösung zu der stabilisierten Diisocyanatsuspension. Bei keiner oder unzureichender Stabilisierung des Isocyanates erfolgt rasche Aufdickung, während die erfindungsgemäßen Reaktionsmischungen bei Raumtemperatur flüssig und lagerstabil bleiben und relativ hohe Aufdickungspunkte (s. Tabelle 13) zeigen.

**Tabelle 13**

| | Polyol (0,1 Mol) | Menge (g) | Aufdickungspunkt (°C) |
|---|---|---|---|
| 1. | Ethylenglykol | 6,2 | 92 |
| 2. | Butandiol-(1,4) | 9,0 | 105 |
| 3. | Diethylenglykol | 10,6 | 125 |
| 4. | Dipropylenglykol | 12,0 | 80-85 |
| 5. | Triol aus Trimethylolpropan und Propylenoxid, OH-Zahl 850 | 12,9 | 85-90 |
| 6. | Tetraol aus Ethylendiamin und Propylenoxid, OH-Zahl 750 | 29,2 | 90-95 |

Versuchteil B) Mischvernetzung mit Polyol/aromatischen Diaminen

Man kann den in Beispiel 13 A) genannten Stabilisierungseffekt des Diisocyanats gegenüber Polyolen noch verstärken, wenn geringe Mengen eines aromatischen Diamins mitverwendet werden.

Bei einer Änderung der Polyoldosierung (statt 0,1 Mol Diol werden 0,09 Mol Diol und 0,01 Mol des aromatischen Diamins DETA eingesetzt) ergeben sich folgende Aufdickungspunkte:

**Tabelle 14**

| | Diol (0,09 Mol) | Menge (g) | aromat. Diamin (0,01 Mol) (g) | Aufdickungspunkt |
|---|---|---|---|---|
| 1. | Ethylenglykol | 5,6 | 1,8 | 135°C |
| 2. | Butandiol-1,4 | 8,1 | 1,8 | 125°C |
| 3. | Diethylenglykol | 9,6 | 1,8 | 153 |
| 4. | Dipropylenglykol | 10,8 | 1,8 | 110 |

**Beispiel 14**

Verwendung von stabilisiertem Naphthylendiisocyanat in Einkomponentensystemen mit Glykolvernetzung

100 g eines Polyethylenglykoladipats, OH-Zahl 56, Molekulargewicht 2000 und 0,6 g Piperazin werden bei 50 bis 60°C vermischt und mit 20 g Naphthylen-1,5-diisocyanat (mittlere Korngröße 20 ± 10 µm) vermischt.

In die stabilisierte Diisocyanatsuspension werden 2,0 g Butandiol-1,4 und 0,1 g Zinn-II-di(2-ethyl-hexanoat) eingemischt. Der Reaktionsansatz ist bei Raumtemperatur lagerstabil. Der Ansatz ergibt beim Ausheizen bei 120°C/0,8 h einen elastischen Polyurethanformkörper der Shore-A-Härte 80.

Ohne Stabilisator bekommt man bei 60°C nach 5 Minuten eine starke Aufdickung, bei Raumtemperatur tritt eine Vernetzung nach ca. 1 bis 2 Tagen ein.

**Beispiel 15**

Stabilisierung von dimerem Diphenylmethan-diisocyanat und Verwendung in Einkomponenten-Reaktivsystemen

100 g eines Polyoxypropylendiols der OH-Zahl 56, Molekulargewicht 2000, werden mit 0,2 g 2,5-Diamino-2,5-dimethylhexan und 25 g dimerem Diphenylmethan-4,4'-diisocyanat vermischt und mit 0,2 g einer 50-%-igen Bleioctoatlösung versetzt.

Versuch B)

In ähnlicher Weise wird eine weitere Reaktionsmischung aus 100 g eines linearen Polyoxypropylendiols mit 20 Mol-% endständigen Oxyethylengruppen (OH-Zahl 56; Molekulargewicht 2000) mit 0,2 g des aliphatischen Diamins, 25 g des dimeren Isocyanats, aber 0,2 g Zinn-II-di(2-ethyl-hexanoat) versetzt.

Das dimere Isocyanat wird wie folgt hergestellt: Zu einer Lösung von 100 g Diphenylmethan-4,4'-diisocyanat in 100 g Toluol und 100 g Petrolether werden 0,25 g Tributylphosphin unter Rühren zugesetzt. Das sich abscheidende Dimere wird noch 2 Stunden weitergerührt, dann abgesaugt und nach Waschen mit Petrolether bei Raumtemperatur im Vakuum unter Feuchtigkeitsausschluß getrocknet. Das Dimere wird in über 95 % Ausbeute und praktisch oligomerenfrei erhalten.

Die aus beiden Reaktionsansätzen (A) bzw. B)) erhaltenen Polyurethane zeigen die in Tabelle 15 angegebenen Werte (infolge Fehlen eines Kettenverlängerungsmittels in diesem Ansatz sind die Elastomereigenschaften auf relativ niedrigem Niveau).

**Tabelle 15**

| | Zugfestigkeit (MPa) | Bruchdehnung (%) | Weiterreißfestigkeit (KN/m) | Shore-A | Elastizität (%) |
|---|---|---|---|---|---|
| Polyurethan A) | 2,5 | 100 | 2,0 | 58 | 64 |
| Polyurethan B) | 2,5 | 110 | 3,5 | 58 | 62 |

**Beispiel 16**

Es werden Polyurethane auf der Basis stabilisierten, dimeren Diphenylmethandiisocyanats und unter Verwendung von aromatischen Diaminen als Kettenverlängerer in unterschiedlichen Härten hergestellt. Der Polyether ist jeweils ein lineares Polyoxypropylendiol mit 20 % endständigen Oxyethyleneinheiten, Molekulargewicht 2000. Als "Amin-Stabilisator" wird das 2,5-Diamino-2,5-dimethylhexan verwendet; als aromatisches Diamin findet DETA (2,4-/2,6-(65/35)-Diamino-3,5-diethyltoluol) Verwendung; als Katalysator findet Zinn-II-di(2-ethyl-hexanoat) Verwendung.

Rezeptur:

**Tabelle 16**

Beispielvarianten

| 1 | 2 | 3 | 4 | |
|---|---|---|---|---|
| 0,3 g | 0,4 g | 0,4 g | 0,6 g | je 100 g Polyether |
| aliphatischer Diaminstabilisator | | | | |
| 38,5 g | 52,0 g | 65,5 g | 79,0 g | " |
| dimeres Diphenylmethandiisocyanat | | | | |
| 5 g | 10 g | 15 g | 20 g | " |
| | | "DETA" | | |
| je 0,2 g Sn-II-octoat | | | | " |

**Tabelle 17**

Mechanische Eigenschaften

| Vers.-Nr. | Zugfestigk. (MPa) | Bruchdehnung (%) | Reißfestigk. (KN/m) | Shore-Härte A | Shore-Härte D | Elastizität (%) |
|---|---|---|---|---|---|---|
| 1 | 5,5 | 170 | 12 | 73 | 22 | 48 |
| 2 | 9,0 | 230 | 22 | 87 | 34 | 47 |
| 3 | 10,3 | 180 | 30 | 92 | 42 | 46 |
| 4 | 13,0 | 170 | 41 | 94 | 48 | 40 |

Während ohne die Stabilisierung mit dem aliphatischen Diamin die entsprechenden Mischungen innerhalb von wenigen Minuten eine Aufdickung zu einer nicht mehr gießfähigen Paste zeigen, sind die stabilisierten Reaktivansätze bei Raumtemperatur praktisch unbegrenzt lagerstabil und auch bei 50°C über Monate lagerbeständig. Mit aromatischen Diamin-Kettenverlängerern werden bessere PU-Elastomereigenschaften erzielt als mit Polyol-Kettenverlängerungsmitteln oder gar ohne Kettenverlängerungsmittel.

**Beispiel 17**

Variation der Menge des aromatischen Kettenverlängererdiamins

Allgemeine Versuchsbeschreibung:

Das dimere Toluylendiisocyanat (TT) wird im Polyetherdiol suspendiert. Nach Zugabe des Stabilisators (3 Äq.-% Stabilisator) läßt man 30 min zur Stabilisierung des Isocyanates bei Raumtemperatur rühren.

Nach Zugabe des aromatischen Diamin-Kettenverlängerers und des Katalysators heizt man bei 120°C aus. Nach wenigen Minuten kann bereits entformt werden. Die nicht ausgeheizten Mischungen besitzen bis 50°C dagegen praktisch unbegrenzte Lagerstabilität.

Rezeptur:

| | |
|---|---|
| 1 Mol | Polyoxypropylenetherdiol, Molekulargewicht 2000, |
| 0,03. | (1+x)Mol 2,5-Diamino-2,5-dimethylhexan |
| | (1+x)Mol dimeres Toluylendiisocyanat |
| x Mol | 2,4-/2,6-(65/35)-Diamino-3,5-diethyltoluol (DETA) |
| 0,2 g | Pb-Octoat-Lösung auf 100 Teile Polyether. |

In der Tabelle 18 werden die weiteren Angaben zu den einzelnen Versuchen 1 bis 4 und die erzielten mechanischen Werte angegeben.

**Tabelle 18**

| Versuch-Nr. | 1 | 2 | 3 | 4 |
|---|---|---|---|---|
| x= | 0,56 | 1,12 | 1,68 | 2,24 |
| ≙ Tl. DETA auf 100 Tl. Polyol | 5 | 10 | 15 | 20 |
| mechanische Werte: | | | | |
| Zugfestigkeit [MPa] | 5,2 | 8,7 | 13,2 | 15 |
| Reißdehnung [%] | 101 | 107 | 104 | 91 |
| Weiterreißfestigkeit [KN/m] | 6,5 | 10 | 22 | 32 |
| Shore-Härte -A | 75 | 83 | 92 | 94 |
| Shore-Härte -D | 22 | 31 | 43 | 52 |
| Elastizität [%] | 50 | 45 | 44 | 40 |
| Druckverformungsrest (nach 24 h bei 70°C) [%] | 26 | 28 | 41 | 56 |

**Beispiel 18**

Variation der Menge des aromatischen Diaminkettenverlängerers; Polyetherdiol mit primären OH-Gruppen

Allgemeine Versuchsbeschreibung:

Das Polyol wird mit dem Stabilisatortriamin vermischt und das Diisocyanat in dieser Mischung suspendiert. Nach 30 Minuten Reaktionszeit bei Raumtemperatur wird das aromatische Diamin "DETA" und der Katalysator zugesetzt (s. Tabelle 19 und die mechanischen Eigenschaften in Tabelle 20).

**Tabelle 19**

Rezepturen:

| Versuch-Nr. | 1 | 2 | 3 |
|---|---|---|---|
| 1 Mol trifunktionelles Polypropylen-polyethylen-glykol-Block-Copolymer, MG 6000 (endständige Oxyethylenreste mit prim. OH-Gruppen) | 100 Tl. | 100 Tl. | 100 Tl. |
| 0,03 bis 0,1 Mol Stabilisator: Triamino-polypropylenether ("T-403" von Texaco, MG 438) | 0,11 Tl. | 0,2 Tl. | 0,4 Tl. |
| (1,5+x) Mol dimeres Toluylen-diisccyanat | mit x=3 ≙26,25 Tl. | x=6,72 ≙48 | x=13,5 ≙89,9 |
| x Mol 2,4-/2,6-(65/35)-Diamino-3,5-diethyltoluol-Isomerengem. (DETA) | 8,9 Tl. | 20 Tl. | 40 Tl. |
| Pb-II-Octoat-Lösung (50 %) | 0,2 Tl. | 0,2 Tl. | 0,4 Tl. |

**Tabelle 20**

Mechanische Eigenschaften der gebildeten Polyurethane

| | | | |
|---|---|---|---|
| Zugfestigkeit [MPa] | 6,0 | 11,5 | 19,4 |
| Reißdehnung [%] | 225 | 200 | 20 |
| Weiterreißfestigkeit [KN/m] | 7,7 | 26,3 | nicht meßbar |
| Shore-Härte -A | 81 | 94 | 99 |
| Shore-Härte -D | 27 | 44 | 62 |
| Elastizität [%] | 55 | 44 | 42 |

**Beispiel 19**

Abhängigkeit der Aushärtedauer von der Aushärtetemperatur

Versuchsbeschreibung:

0,4 g Ethylendiamin (1,66 Äq.-%) als Stabilisator werden in 400 g Polyoxypropylenetherdiol, Molekulargewicht 2000, gelöst. Nach Einrühren von 140 g dimerem Toluylendiisocyanat läßt man 30 Minuten zur Stabilisierung des Diisocyanats reagieren. Zuletzt werden 35,6 g des aromatischen Diamins "DETA" und 0,8 g Blei-octoat-Lösung zugesetzt. Die Mischung zeigt eine Aufdickungstemperatur von 90°C und ist innerhalb von etwa 1 Minute bei 110°C (Vernetzungstemperatur) aushärtbar.

Durchführung der Messungen:

Auf eine Kofler-Heizbank werden dünne Schichten der Mischung aufgetragen und die Zeit (in Sekunden) bis zur Vernetzung (Aushärtedauer) für verschiedene Temperaturen gemessen. Die Aushärtedauer bei Temperaturen ≤80°C ergibt sich aus Lagerversuchen.

**Tabelle 21**

| | | | | | | | | | | | |
|---|---|---|---|---|---|---|---|---|---|---|---|
| Aushärtetemperatur (°C) | 150 | 140 | 130 | 120 | 110 | 100 | 90 | 80 | 70 | 60 | 50 |
| Aushärtedauer (sec) | 10 | 15 | 25 | 40 | 70 | 150 | 360 | $10^3$ | $5x10^3$ | $10^5$ | |

(extrapolierte Werte aus Lagerversuchen)

**Beispiel 20**

Abhängigkeit der Aushärtetemperatur von der Menge an aliphatischem Aminstabilisator

Versuchsbeschreibung:
Rezeptur wie im vorhergehenden Beispiel 19 mit abgewandelten Mengen an Ethylendiamin.

Durchführung der Messungen:
Die für eine Aushärtedauer von 1 Minute notwendige Aushärtetemperatur sowie die Aufdickungstemperatur werden für verschiedene zugesetzte Mengen Stabilisatoramin auf der Kofler-Heizbank bestimmt. Einige Aufdickungstemperaturen werden mit einem Reagenzglas im Ölbad genauer bestimmt und befriedigende Übereinstimmung mit den auf der Kofler-Bank ermittelten Temperaturen festgestellt.

**Tabelle 22**

| | | | | | |
|---|---|---|---|---|---|
| Mol-% Ethylendiamin, bezogen auf TT | 0,25 | 0,5 | 1,0 | 1,66 | 3,33 |
| Aufdickungstemp. (°C) | RT | RT | 65 | 90 | 110 |
| Aushärtetemp. (°C) | 88 | 92 | 100 | 110 | 120 |

**Beispiel 21**

Kombinierte Wirkung zweier verschiedener Aminstabilisatoren

Rezeptur:

| | |
|---|---|
| 100 | g (0,05 Mol) Polyoxypropylenetherdiol, MG=2000 |
| x | g aliphatischer Diaminstabilisator |
| 35 | g dimerisiertes Toluylen-2,4-diisocyanat (TT) |
| 8,9 | g 2,4-/2,6-Diamino-3,5-diethyltoluol-Isomerengemisch (DETA) |
| 0,2 | g Pb-Octoat-Lösung (50 % in Waschbenzin) |

Versuchsbeschreibung:
a) Im Polyol werden 0,1 g Ethylendiamin gelöst. Nach Zugabe von TT wird 30 Minuten bei Raumtemperatur gerührt und anschließend der Kettenverlängerer und der Katalysator zugegeben.
b) Im Polyol werden 0,2 g 2,5-Diamino-2,5-dimethylhexan gelöst. Man verfährt weiter wie unter a).
c) Man löst zunächst 0,05 g Ethylendiamin im Polyol. Man läßt die gesamte Menge TT 15 Minuten mit der Polyol/Ethylendiamin-Mischung reagieren; gibt 0,1 g 2,5-Diamino-2,5-dimethylhexan zu, rührt weitere 15 Minuten bei Raumtemperatur und verfährt weiter wie unter a).
d) Man verfährt ähnlich wie unter c), indem zuerst mit 0,1 g 2,5-Diamino-2,5-dimethylhexan und anschließend mit 0,05 g Ethylendiamin stabilisiert wird.

Man bestimmt wie in Beispiel 2 die Aufdickungstemperatur.
Man beurteilt die Oberflächengüte eines in einer offenen Gießform durch Ausheizen bei 120°C erzeugten Formteils.

**Tabelle 23**

| Versuch | a) | b) | c) | d) |
|---|---|---|---|---|
| Oberflächengüte | etwas wellig | glatt | glatt | glatt |
| Aufdickungstemp. | 90°C | 60°C | 80°C | 70°C |

Das Beispiel zeigt, daß es möglich ist, durch Übereinanderlegen zweier verschiedener Schutzschichten auf demselben Diisocyanat-Korn die Verarbeitungseigenschaften von PUR-EK-Systemen weiter zu optimieren: In Versuch c) bewirkt die mit Ethylendiamin aufgebaute innere Schicht eine gegenüber Versuch b) erhöhte Aufdickungstemperatur, die mit 2,5-Diamino-2,5-dimethylhexan aufgebaute äußere Schutzschicht gewährleistet gute Ausheiztemperatur und gute Qualität der ausgeheizten Polyurethane.

**Beispiel 22**

Verwendung stabilisierter Polyisocyanate zur Herstellung von geschäumten Polyurethanen

Zur Herstellung eines geschäumten Formteils werden zunächst folgende Polyolkomponenten bei Raumtemperatur mit einem Schnellrührer 30 Sekunden intensiv miteinander vermischt:

| | | |
|---|---|---|
| | 70 Teile | Polyol A (Polyether, Funktionalität 2, MG=4000, Additionsprodukt von Propylenoxid (80 Gew.-Teile) und Ethylenoxid (20 Gew.-Teile) an Propylenglykol) |
| | 20 Teile | Polyol B (Polyether, Funktionalität 3, MG=4800, Additionsprodukt von Propylenoxid und Ethylenoxid (50/50) an 1,1,1-Trimethylolpropan) |
| | 1 Teil | Ethylenglykol |
| + | 14 Teile | Butandiol-1,4 |
| | 105 Teile | Polyolgemisch |

Dann werden 39 Teile dimerisiertes 2,4-Toluylendiisocyanat unter den gleichen Bedingungen gleichmäßig im Polyolgemisch dispergiert und durch direkt anschließende Zugabe von 0,72 Teilen 2,5-Diamino-2,5-dimethylhexan wird das feste Polyisocyanat umhüllt. Nach ca. 15 Minuten werden 0,72 Teile eines Silikonpolyether-Copolymeren (Schaumstabilisator OS-50, Bayer AG), 0,28 Teile einer ca. 50-%-igen Lösung von Blei-II-octoat (als Katalysator) und 8,7 Teile Trichlorfluormethan (als Treibmittel) untergemischt. Die Mischung wird innerhalb von 2 Sekunden in ein senkrecht stehendes, auf 80°C thermostatisiertes Formwerkzeug eingetragen. Nach 10 Minuten wird eine 10 mm dicke, hochelastische Probeplatte der Rohdichte 786 kg/m$^3$ entnommen. Die Probe besitzt eine Oberflächenhärte von 50 Shore-A. Das zur Herstellung der geschäumten Probeplatte eingesetzte Gemisch ohne Treibmittel ist bei Raumtemperatur über mehrere Wochen lagerstabil.

Ohne Zugabe des aliphatischen Diamins verfestigt sich aber das Gemisch innerhalb von ca. 24 Stunden und ist nicht mehr verarbeitbar.

**Beispiel 23**

Reaktion von Einkomponenten-PU-Reaktivmischungen unter Trimerisierung (durch Katalysatorzusatz) zum Polyurethan

Ein Gemisch aus 100 Teilen eines Polyoxypropylenamines, Molekulargewicht 2000 (Jeffamine D 2000 der Fa. Texaco Chem. Comp./USA) und 0,75 Teilen Ethylendiamin wird mit 62,5 Teilen feinpulvrigem 4,4'-Diisocyanatodiphenylmethan intensiv bei Raumtemperatur vermischt (NCO : $NH_2$-Äquivalentverhältnis=5 : 1). Nach der Zugabe von 2 g N,N',N''-Tris(3-dimethylaminopropyl)-s-triazin erhält man ein lagerstabiles Gemisch, welches bei 90°C innerhalb von 30 Sekunden aushärtet. Härtet man nicht thermisch aus, sondern versetzt die Mischung mit 10 g Dimethylformamid bei Raumtemperatur, so härtet das Reaktivgemisch unter Wärmeentwicklung sehr schnell aus.

Die erhaltenen hochelastischen und harten Probekörper sind unlöslich in siedendem Dimethylformamid. Sie zeigen im IR-Spektrum die für den Isocyanuratring typischen Absorptionen.

**Beispiel 24**

Beeinflussung des Aufdickungspunktes und der Lagerstabilität durch Zugabe von Lösungsmitteln

Herstellung des Ausgangsmaterials

2000 g eines linearen Polyethers der OH-Zahl 56, Molekulargewicht 2000, 80 Gew.-% Propylenoxid- und 20 Gew.-% Ethylenoxid-Einheiten, werden mit 1 g Ethylendiamin intensiv vermischt. In dieses Gemisch werden 700 g dimerisiertes 2,4-Toluylendiisocyanat eingetragen und intensiv verrührt. Ca. 20 Minuten nach Beendigung des Rührens werden 180 g Diethyltoluylendiamin - DETA - (35 % 2,6-Diamino- und 65 % 2,4-Diamino-3,5-diethyltoluol) und 4 g einer ca. 50-%-igen Lösung von Blei-di(2-ethylhexoat) in einem aliphatischen Kohlenwasserstoff untergemischt.

50 g dieser Mischung werden mit einer abgemessenen Volumenmenge eines Lösungsmittels versetzt, der Aufdickungspunkt bestimmt (Durchführung s. Beispiel 2) und das Lagerverhalten der Probe beobachtet. Die Ergebnisse sind in folgender Tabelle 24 zusammengefaßt.

**Tabelle 24**

| Lösungsmittel | Menge auf 50 g Gemisch | Aufdickungs-punkt | Lagerverhalten bei Raumtemperatur | | |
|---|---|---|---|---|---|
| ohne | - | 80°C | | 4 Wochen | lagerstabil |
| Dimethylformamid | 1,0 ml | 75°C | nach ca. | 10 Tagen | Verfestigung |
| | 2,5 ml | 75°C | " | 6 " | " |
| | 5,0 ml | 67°C | " | 30 min | " |
| | 12,5 ml | | " | 15 min | " |
| Tetramethylensulfon | 1,0 ml | 80°C | " | 12 Tagen | " |
| | 2,5 ml | 75°C | " | " | " |
| | 5,0 ml | 70°C | " | " | " |
| | 12,5 ml | 65°C | " | " | " |
| Propylencarbonat | 1,0 ml | 68°C | " | " | " |
| | 2,5 ml | 65°C | " | 9 " | " |
| | 5,0 ml | 65°C | " | 9 " | " |
| | 12,5 ml | 65°C | " | 6 " | " |
| | 20,0 ml | 60°C | " | 6 " | " |
| Dimethylacetamid | 1,0 ml | 75°C | " | 12 " | " |
| | 2,5 ml | 65°C | " | 6 " | " |
| | 5,0 ml | - | " | 30 min | " |
| | 12,5 ml | - | " | 15 min | " |
| N-Methylpyrrolidon | 1,0 ml | 76°C | " | 12 Tagen | " |
| | 2,5 ml | 70°C | " | 10 " | " |
| | 5,0 ml | - | " | 30 min | " |
| | 12,5 ml | - | " | 15 min | " |
| Nitrobenzol | 1,0 ml | 80°C | " | 12 Tagen | " |
| | 2,5 ml | 76°C | " | 12 " | " |
| | 5,0 ml | 67°C | " | 10 " | " |
| | 12,5 ml | - | " | 30 min | " |
| Benzonitril | 1,0 ml | 78°C | " | 12 Tagen | " |
| | 2,5 ml | 75°C | " | 12 " | " |
| | 5,0 ml | 70°C | " | 10 " | " |
| | 12,5 ml | - | " | 30 min | " |
| N,N'-Tetramethyl-harnstoff | 1,0 ml | 75°C | " | 12 Tagen | " |
| | 2,5 ml | 70°C | " | 12 " | " |
| | 5,0 ml | 67°C | " | 10 " | " |
| | 12,5 ml | - | " | 15 min | " |
| o-Dichlorbenzol | 1,0 ml | 80°C | " | 12 Tagen | " |
| | 2,5 ml | 70°C | " | 10 " | " |
| | 5,0 ml | 70°C | " | 10 " | " |
| | 12,5 ml | 62°C | " | 6 " | " |
| Chlorbenzol | 1,0 ml | 80°C | " | 12 " | " |
| | 2,5 ml | 80°C | " | 12 " | " |
| | 5,0 ml | 70°C | " | 20 " | " |
| | 12,5 ml | 65°C | " | 6 " | " |
| Toluol | 1,0 ml | 75°C | " | 12 " | " |
| | 2,5 ml | 67°C | " | 12 " | " |
| | 5,0 ml | 65°C | " | 12 " | " |
| | 12,5 ml | 60°C | " | 6 " | " |
| Waschbenzin | 1,0 ml | 80°C | " | 12 " | " |
| | 2,5 ml | 78°C | " | 12 " | " |
| | 5,0 ml | 72°C | " | 12 " | " |
| | 12,5 ml | 70°C | " | 8 " | " |

**Beispiel 25**

Vernetzung durch Scherkräfte unter Aufbrechung der Umhüllungsschicht

Eine bei Raumtemperatur flüssige und lagerstabile Einkomponentenmischung (Lagerstabilität $\geqslant 3$ Monate

bei Raumtemperatur) aus 0,05 Mol eines linearen Polyoxypropylenetherdiols mit 20 % Ethylenoxideinheiten am Kettenende, Molekulargewicht 2000, 0,05 g 2,5-Diamino-2,5-dimethylhexan, 0,1 Mol dimerem Toluylendiisocyanat und 0,05 Mol des aromatischen Diamins "DETA" und 0,2 g 50-%-ige Bleioctoatlösung wird in einem hochtourigen Rührwerk (Starmix der Fa. Braun) bei ca. 5000 U/min des Messerkreuzes einer starken mechanischen Belastung ausgesetzt. Die Temperatur des Reaktionsansatzes steigt bei dem 2 bis 3 Minuten dauerenden Rührprozeß auf 35 bis 40°C. Danach wird der noch flüssige Ansatz in eine kalte Form gegossen. Nach kurzer Zeit beginnt sich die Mischung zu verfestigen und man erhält nach 10 bis 20 Stunden bei Raumtemperatur einen elastischen Formkörper der Härte 75 bis 80 (Shore A). Durch Zusatz geringer Mengen an polaren Lösungsmitteln (z. B. 3 bis 5 % N-Methyl-pyrrolidon, bezogen auf 100 g Mischung) kann die Vernetzungszeit deutlich verringert werden (5 bis 10 Stunden bei Raumtemperatur).

**Beispiel 26**

Einkomponentenmischung auf Basis aromatischer Aminopolyether

Rezeptur:

| | |
|---|---|
| 100 | g eines aromatischen Aminopolyethers auf Basis eines Polyoxypropylenetherdiols (Molekulargewicht 2000)/Toluylendiisocyanats (Molverhältnis 1 : 2), hergestellt nach dem Verfahren gemäß DE-OS-2 948 419, mit einer Aminzahl=46,7 und einem Molekulargewicht von 2350, |
| 0,35 | g Piperazin, |
| 12 | g gemahlenes 1,5-Diisocyanato-naphthalin (Korngröße 1-20 μm). |

Versuchsbeschreibung:

Das Piperazin wird im Aminopolyether gelöst und dazu das feingemahlene Diisocyanat rasch eingetragen. Man erhält eine bei Raumtemperatur gut lagerstabile Suspension des stabilisierten Diisocyanats.

Nach dem Ausheizen der Mischung bei 120°C (30-60 min) erhält man einen elastischen Formkörper mit folgenden mechanischen Eigenschaften:

**Tabelle 25**

| | |
|---|---|
| Shore-Härte -A | 90 |
| Shore-Härte -D | 30 |
| Zugfestigkeit [MPa] | 9,5 |
| Reißdehnung [%] | 200 |
| Weiterreißfestigkeit [KN/m] | 15,5 |
| Elastizität [%] | 55 |

**Beispiel 27**

Zu einer Suspension von 34,8 g (0,1 Mol) TT in 100 g (0,05 Mol) eines linearen PPG-Ethers (Molgewicht: 2000, OH-Zahl=56) wird eine Lösung von 8,9 g (0,05 Mol) 2,4-Diamino-3,5-diethyltoluol und 0,2 g Bleioctoat (Octasoligen Blei) zugegeben. Der Reaktionsansatz besitzt eine Gießzeit von ca. 5 Minuten bei Raumtemperatur und verfestigt nach 10 - 15 Minuten zu einer steifen pastenartigen Masse. Werden obiger Suspension vor der Zugabe des aromatischen Diamins dagegen die in nachstehender Tabelle angegebenen Mengen an 2,5-Diamino-2,5-dimethylhexan zugesetzt, so sind die Verfestigungszeiten der Ansätze deutlich verzögert.

**Tabelle 26**

| | aliphat. Diamin (g) (Stabilisator) | Verfestigungszeit bei RT |
|---|---|---|
| 1. | - | 10 - 15 Minuten |
| 2. | 0,005 g (0,035 Mol-%) | 30 Minuten |
| 3. | 0,01 (0,07 Mol-%) | 1,0 Stunde |
| 4. | 0,015 (0,104 Mol-%) | 1,5 Stunden |
| 5. | 0,02 (0,15 Mol-%) | 2,0 Stunden |

**Beispiel 28**

Zu einer Suspension von 52,2 g (0,15 Mol) dimerisiertem Toluylen-2,4-diisocyanat in 100 g (0,05 Mol) eines linearen Polypropylenglykol-Ethers (Molekulargewicht: 2000; OH-Zahl: 56) wird eine Lösung von 6,2 g (0,1 Mol) Ethylenglykol und 0,3 g Blei-octoat-Lösung (Octasoligen Pb 24, Fa. Borchers, Düsseldorf) zugegeben. Die entstandene Suspension besitzt eine Viskosität von ca. 2000 cP (25°C). Der Reaktionsansatz besitzt bei Raumtemperatur eine Gießzeit von ca. 1 Stunde und verfestigt nach ca. 3 Stunden zu einer steifen, pastenartigen Masse. Werden dagegen zu der obigen Suspension vor der Zugabe des Ethylenglykols und der Bleiverbindung die in untenstehender Tabelle angegebenen Mengen an 2,5-Diamino-2,5-dimethylhexan zugesetzt, so wird die Verfestigungszeit der Ansätze deutlich verzögert:

**Tabelle 27**

| | g aliphat. Diamin (Mol-% bez. auf Diisocyanat) | Verfestigungszeit |
|---|---|---|
| 1 | - | ca. 3 Stunden |
| 2 | 0,011 g 0,05 % | ca. 6 Stunden |
| 3 | 0,054 g 0,25 % | ca. 12 Stunden |
| 4 | 0,108 g 0,5 % | ca. 24 Stunden |
| 5 | 0,216 g 1,0 % | nach 5 Tagen: visk. 23 000 cP (noch gießfähig) |
| 6 | 0,432 g 2,0 % | nach 5 Tagen: visk. 17 000 cP (noch gießfähig) |

**Beispiel 29**

a) Mischung eines stabilisierten Diisocyanats in einem höhermolekularen Polyamin

In 100 Teile eines aromatischen, höhermolekularen Polyetherpolyamins der NH-Zahl 80,4 (Herstellung s. unter c)) werden 0,4 Teile 4,4'-Diamino-3,3'-dimethyldicyclohexylmethan als Stabilisator-Diamin gelöst. In dieser Mischung werden bei 60°C 54,2 Teile feingemahlenes dimeres Toluylen-2,4-diisocyanat suspendiert. Nach halbstündigem Rühren bei 60°C werden 20 Teile aufgeschmolzenes 3,5,3',5'-Tetraethyl-4,4'-diamino-diphenylmethan langsam eingerührt. Man rührt noch 4 Stunden bei 70°C nach und erhält eine bis 70°C vollkommen lagerstabile Gießmasse mit einer Aufdickungstemperatur von 100°C und einer Viskosität von 18 Pa.s bei 50°C oder 1,5 Pa.s bei 76°C.

b) Verwendung von a) zur Polyurethanherstellung

Zur Herstellung einer Hart-Elastomerplatte wird die Gießmasse in einer mit Silikontrennmittel eingestrichene offene Gießform eingefüllt und 6 Stunden bei 120°C verfestigt und getempert. Man erhält ein Elastomer mit den in Tabelle 6 wiedergegebenen mechanischen Eigenschaften:

**Tabelle 28**

| | | Beispiel-Nummern | | | | |
|---|---|---|---|---|---|---|
| | | 29 | 30 | 31 | 32 | 33 |
| Zugfestigkeit (DIN 53 504) | [MPa] | 19 | 3,5 | 4,0 | 9,5 | 15 |
| Reißdehnung (DIN 53 504) | [%] | 80 | 150 | 150 | 200 | 420 |
| Weiterreißfestigkeit nach DIN 53 515 | [KN/m] | 94 | 5,0 | 4,5 | 12,1 | 42 |
| Shore-Härte nach DIN 53 505 | -A | 100 | 72 | 70 | 87 | 90 |
| | -D | 70 | 21 | 20 | 35 | - |
| Elastizität nach DIN 53 512 | [%] | 47 | 61 | 62 | 40 | 57 |

c) Herstellung des höhermolekularen Polyamins

1 Mol eines linearen Polypropylenglykols der OH-Zahl 112 (Molekulargewicht 1000) wird mit 2 Mol Toluylen-2,4-diisocyanat durch 4-stündiges Erwärmen bei 80°C in ein NCO-Prepolymer überführt. Eine 25-%-ige Acetonlösung des NCO-Prepolymers wird nun so zu einer 10-%-igen wäßrigen Natriumhydroxidlösung (2,2 Mol NaOH) gegeben, daß die Reaktionstemperatur nicht über 25°C steigt. 30 Minuten wird bei dieser Temperatur weitergerührt und dann 2 Stunden zum Rückfluß erhitzt. Nach halbstündigem Stehen wird die untere, wäßrige Salzlösung aus dem zweiphasigen Reaktionsgemisch abgetrennt und verworfen. Die obere Phase wird bei 20 mbar/80°C und dann bei 1 Mbar/100°C von Wasser- und Acetonresten befreit. Durch Absaugen des 60°C warmen Produktes über eine Drucknutsche (3 bar Überdruck) werden geringe Reste Salz abgetrennt und das Polyetheramin mit einer NH-Zahl von 80,4 (Molekulargewicht 1390) isoliert.

**Beispiel 30**

a) Herstellung der Polyisocyanat-Suspension

In 100 Teile eines difunktionellen, höhermolekularen aliphatischen Polyetherdiamins der NH-Zahl 28 (Herstellung s. unter c)) werden 9,7 Teile dimeres Toluylen-2,4-diisocyanat suspendiert. Man erhält eine Gießmasse, die bis 50°C vollkommen lagerbeständig ist, bei Raumtemperatur eine Viskosität von 0,8 Pa.s besitzt und eine Aufdickungstemperatur von 80°C aufweist.

b) Verwendung zur Herstellung einer Elastomerplatte

Die Gießmasse wird in eine mit Silikontrennmittel eingestrichene offene Gießform eingefüllt und 6 Stunden bei 120°C verfestigt und getempert. Man erhält ein Elastomer mit den in Tabelle 28 angegeben Eigenschaften.

c) Herstellung des höhermolekularen Aminopolyols

Aus einem linearen Polypropylenetherdiol der OH-Zahl 28 wird durch reduktive Aminierung mit Ammoniak/Wasserstoff nach dem Verfahren der BE-PS-634 731 ein höhermolekulares, difunktionelles, aliphatisches Polyetherdiamin der NH-Zahl 28 erhalten.

**Beispiel 31**

a) Herstellung des Polyisocyanat-Suspension

In 100 Teile eines aliphatischen, höhermolekularen Polyethertriamins der NH-Zahl 37,8 (Herstellung s. Beispiel 42 c)) werden 0,1 Teile 4,4'-Diamino-3,3'-dimethyl-dicyclohexylmethan gelöst und 12,12 Teile dimeres, feinpulverisiertes Toluylen-2,4-diisocyanat suspendiert. Man erhält eine Gießmasse, die bis 50°C vollkommen lagerbeständig ist, bei Raumtemperatur eine Viskosität von 1 Pa.s besitzt und eine Aufdickungstemperatur von 70°C aufweist.

b) Verwendung zur Polyurethanherstellung

Zur Herstellung einer Elastomerplatte wird die Gießmasse in eine mit Silikontrennmittel eingestrichene offene Gießform eingefüllt und 6 Stunden bei 120°C verfestig und getempert. Man erhält eine Elastomerplatte mit den in Tabelle 28 wiedergegebenen Eigenschaften.

**Beispiel 32**

a) Herstellung der Polyisocyanat-Suspension

In 100 Teile eines aliphatischen höhermolekularen Polyethertriamins der NH-Zahl 35 (Herstellung s. unter c)) werden 0,2 Teile 4,4'-Diamino-3,3'-dimethyl-dicyclohexylmethan gelöst und 29,5 Teile dimeres Toluyen-2,4-

diiscoyanat suspendiert. Nach 30-minütigen Rühren bei Raumtemperatur werden 8,9 Teile eines Isomerengemisch aus 2,4-Diamino- und 2,6-Diamino-3,5-diethyltoloul (Isomerenverhältnis 65 : 35) als Kettenverlängerungsmittel zugegeben. Man erhält eine Gießmasse, die bis 50°C vollkommen lagerbeständig ist, bei Raumtemperatur eine Viskosität von 1,3 Pa.s besitzt und eine Aufdickungstemperatur von 80°C aufweist.

b) Verwendung der Suspension zur Polyurethanherstellung

Zur Herstellung einer Elastomerplatte wird die Gießmasse in eine mit Silikontrennmittel eingestrichene offene Gießform eingefüllt und 6 Stunden bei 120°C verfestig und getempert. Man erhält ein Elastomer mit den in der Tabelle 28 angegebenen Eigenschaften.

**Beispiel 33**

a) Herstellung der Polyisocyanat-Suspension

200 Teile des höhermolekularen aromatischen Polyetherdiamins der NH-Zahl 47,4 (Herstellung s. unter c)) werden mit 0,5 Teilen Ethylendiamin und 0,2 Teilen 3,3'-Diemethyl-4,4'-diaminodicyclohexylmethan vermischt. Danach erfolgt zugabe von 20 Teilen pulverisiertem 1,5-Diisocyanatonaphthalin. Diese Mischung verhält sich bei Raumtemperatur mindestens 2 Monate lagerstabil. Bei 120°C erfolgt jedoch eine rasche Verfestigung.

b) Verwendung der Suspension zur Polyurethanherstellung

In mit Silikontrennmittel ausgesprühten Gießformen gegossene Reaktionsansätze ergeben nach Verfestigung bei 120 bis 125°C hochelastische Polyurethanelastomere mit den in Tabelle 28 angegebenen Eigenschaften.

c) Herstellung des höhermolekularen Polyamins

1 Mol eines linearen Polypropylenetherglykols der OH-Zahl 56 und 2 Mol Toluylen-2,4-diisocyanat werden durch 4-stündiges Erhitzen auf 80°C in ein NCO-Prepolymer (3,58 % NCO) überführt. Dazu werden 810 g des 45°C warmen NCO-Prepolymers unter intensivem Rühren so zu einer gekühlten Lösung von 52,2 g Kaliumhydroxid und 500 ml Wasser und 300 ml Aceton gegeben (NCO : $OH^{\ominus}$ -Verhältnis = 1 : 1,35), daß eine Innentemperatur von 25°C nicht überschritten wird. 30 Minuten wird bei dieser Temperatur weitergerührt und dann 2 Stunden zum Rückfluß erhitzt. Nach 30-minütigem Stehen werden die untere, wäßrige Salzlösung aus dem zweiphasigen Reaktionsgemisch abgetrennt und verworfen. Die obere Phase wird bei 20 mbar/80°C und dann bei 1 mbar/100°C von Wasser- und Acetonresten befreit. Durch Absaugen des 60°C warmen Produkts über eine Drucknutsche (3 bar Überdruck) werden geringe Reste Salz abgetrennt und das Polyetheramin mit einer NH-Zahl von 47,6 isoliert.

**Beispiel 34**

a) Herstellung der Polyisocyanat-Suspension

30 Teile Bis-(3,3'-diisocyanato-4,4'-dimethylphenyl)-harnstoff, 30 Teile eines linearen Polypropylenetherdiols des Molekulargewichts 2000 und 100 Teile des nach Beispiel 33 c) hergestellten aromatischen Polyetherdiamins der NH-Zahl 47,4 werden in einem Starmix bei Raumtemperatur suspendiert. Anschließend wird eine Lösung von 0,6 Teilen 3,3'-Dimethyl-4,4'-diaminodicyclohexylmethan in 100 Teilen des in Beispiel 33 c) genannten aromatischen Polyetherdiamins der NH-Zahl 47,4 verrührt. Bei Raumtemperatur ist die Suspension der stabilisierten Diisocyanate lagerstabil.

b) Verwendung der Suspension zur Elastomerherstellung

Die Suspension härtet bei 120°C in kurzer Zeit aus. Nach 2- bis 4-stündiger Lagerung bei 120°C erhält man hochelastische Formkörper mit einer Härte von 85 bis 87 Shore A.

**Beispiel 35**

a) Herstellung der Polyisocyanat-Suspension

In 100 Teilen des aromatischen Polyetherpolyamins der NH-Zahl 47,4 (s. Beispiel 33 c)) werden 0,15 Teile 3,3'-Dimethyl-4,4'-diamino-dicyclohexylmethan und 16,8 Teile dimeres Toluylen-2,4-diisocyanat dispergiert.

b) Verwendung der stabilisierten Polyisocyanat-Suspension

Das nach a) hergestellte, lagerstabile Einkomponentensystem wird nach untenstehenden, im Prinzip bekannten Verfahrenstechniken, verarbeitet, wobei je nach Geometrie des Werkzeuges verschieden gestaltete Formkörper erhalten werden konnten.

1. Tauchverfahren

In das auf 50°C erwärmte Einkomponentensystem wurde ein mit 120°C heißem Silikonöl gefüllter länglicher, dünnwandiger, zylindrischer Glaskolben mit einem Durchmesser von 3 cm getaucht. Die Außenoberfläche des Glaszylinders war mit wenig Silikontrennmittel beschichtet. Es erfolgt spontane Verfestigung des

Einkomponentensystems an der beheizten Zylinderwand, wobei für eine Schichtdicke von 1 mm ca. 1 Minute erforderlich ist. Die Verfestigung des flüssigen Systems erfolgt nur an der heißen Glaswand. Man erhält je nach Tauchzeit dünn- bis dickwandige, hochelastische Hohlkörper mit guten mechanischen Eigenschaften.

2. Rotationsverfahren

Obiges Einkomponentensystem wurde auf 50° C erwärmt und die nun gut gießbare Reaktionsmischung wird (in geringeren Mengen als dem Rohrvolumen entspricht) in ein sorgfältig gereinigtes Eisenrohr (Durchmesser 4 cm) gegossen. Beide Rohrenden werden verschlossen. Während der nun folgenden Rotation horizontal zur Rohrlängsrichtung wird der Rohrmantel von außen gleichmäßig mit einem Heißluftgebläse auf 110 bis 130° C erwärmt. Nach kurzer Rotationszeit kann auf diese Weise eine Rohrinnenbeschichtung durchgeführt werden, wobei die Dicke der Beschichtung durch die Menge des eingefüllten Einkomponentensystems bestimmt wird.

3. Slush Molding

Eine auf 50 bis 80° C erwärmte Form wird mit dem Einkomponenten-PU-Reaktivsystem gefüllt. Danach wird auf 120° C erwärmt. Hierbei erfolgt die Verfestigung des Systems zunächst an der Forminnenwand und schreitet allmählich nach innen fort. Infolge der schlechteren Wärmeleitfähigkeit der verfestigten Außenschichten bleibt der Formkern relativ lange flüssig. Nach einer bestimmten Zeit (in der die Verfestigung des Formkerns noch nicht eingetreten ist) wird die Form geleert, der Formkörper entfernt und 4 bis 5 Stunden bei 120° C nachgetempert. Die Wanddicke des Elastomers ist wie beim Tauchverfahren von der Ausheizzeit abhängig. Da das noch flüssige Material voll weiterverwertbar ist, kann nach Zugabe der fehlenden Menge dieser Arbeitszyklus beliebig oft wiederholt werden.

**Beispiel 36**

a) Herstellung der stabilisierten Polyisocyanat-Suspension

Ein Gemisch aus 100 Teilen eines langkettigen Polyetherpolyamins der NH-Zahl 47 (Herstellung s. Beispiel 33 c)), 1,15 Teilen Diazabicyclooctan (als Katalysator) und 0,6 Teilen eines Ethylenoxid/Propylenoxid-Dimethylsiloxan-Blockpolymers als oberflächenaktives Silikon wird mit 0,07 Teilen 2,5-Diamino-2,5-dimethylhexan (als Aminstabilisator) versetzt. Anschließend werden 16,54 Teile dimerisiertes 2,4-Toluylendiisocyanat mit einem Schnellrührer innerhalb von 1 Minute untergerührt. Es wird eine lagerstabile Suspension erhalten.

b) Verwendung der Suspension zur Polyurethanherstellung

800 Teile dieser Suspension werden mit 200 Teilen Kupferpulver intensiv vermischt, entgast (Wasserstrahlvakuum, Raumtemperatur, 1 Stunde) und von dieser Mischung 1000 g in ein auf 95° C aufgeheiztes, senkrecht stehendes Plattenwerkzeug (Formteilgröße 20 x 39,4 x 1 cm) von unten innerhalb von ca. 3 Sekunden eingetragen. Nach 5 Minuten wird ein elastisches Formteil mit einer Oberflächenhärte von 94 Shore A (43 Shore D/25° C) und einer Dichte von 1,27 g/$cm^3$ entnommen. Die Oberfläche des Formteils ist fehlerfrei.

**Beispiel 37**

a) Herstellung der Suspension des stabilisierten Diisocyanats

Ein Gemisch aus 100 Teilen eines langkettigen, aromatischen Polyetherdiamins der NH-Zahl 47,4 (Herstellung s. Beispiel 33 c)), 1,15 Teilen Diazabicyclooctan (als Katalysator) und 0,6 Teilen eines Polyethersiloxanblockcopolymers wird mit 0,07 Teilen 2,5-Diamino-2,5-dimethylhexan versetzt. Anschließend werden 16,54 Teile dimerisiertes 2,4-Toluylendiisocyanat mit einem Schnellrührer untergemischt. Es wird eine lagerstabile Suspension erhalten. Durch diese Suspension wird ca. 2,5 Stunden lang ein kräftiger Luftstrom geleitet, um eine cremige Suspension zu erhalten.

b) Verwendung der Suspension

590 g des cremigen Gemisches werden ohne weiteres Rühren in ein auf 95° C aufgeheiztes, senkrecht stehendes Plattenwerkzeug (Formteilgröße 20 x 39,4 x 1cm) von unten innerhalb von ca. 2 Sekunden eingetragen. Nach 5 Minuten wird ein Formteil mit einer Oberflächenhärte von 73 Shore A (bei 25° C), einer Dichte von 0,75 g/$cm^3$ und einer integralen Dichteverteilung (massive Randzone, zelliger Kern) entnommen. Die Oberfläche des Formteils ist fehlerfrei.

**Beispiel 38** (Vergleichsversuch)

In ein Gemisch aus 40 Teilen eines Polyethers der OH-Zahl 42, mittlere Funktionalität 2,78 (Molekulargewicht 3700), der durch Addition von Ethylenoxid und Propylenoxid an ein Gemisch aus Trimethylolpropan und Propylenglykol erhalten wurde, 60 Teilen eines trifunktionellen Polyols der OH-Zahl 865, das durch Addition von 0,9 Mol Propylenoxid an 1 Mol Trimethylolpropan erhalten wurde, 0,2 Teile einer ca. 50-%-igen Lösung von Blei-

ll-octoat in Benzin und x Teilen 3,3'-Dimethyl-4,4'-diamino-dicyclohexylmethan (als Amin-Stabilisator) werden innerhalb von 2 Minuten 88,5 Teile dimerisiertes 2,4-Toluylendiisocyanat eingerührt. Von den hergestellten Proben wird die Viskosität über mehrere Tage verfolgt.

**Tabelle 29**

Aufgetragen sind die Viskositäten in mPa.s bei 25°C in Abhängigkeit von x und der Lagerzeit in Tagen.

| [Tage] Zeit | x<br>0<br>(Vergleich) | <br>0,5 |
|---|---|---|
| 0 | 26 000 | 40 000 |
| 1 | 200 000 | 65 000 |
| 2 | >400 000 | 65 000 |
| 5 | fest | 65 000 |
| 8 | fest | 70 000 |
| 14 | fest | 70 000 |

Wie der Vergleichsversuch zeigt, reagiert das dimere Toluylen-2,4-diisocyanat durch Lagerung in OH-Polyethern plus niedermolekularen Polyolen an der Oberfläche ständig bis zur Verfestigung des Systems weiter, bildet jedoch keine "stabilisierende Umhüllung" aus, die zu einer retardierten Reaktivität des Polyisocyanates führt.

Erst eine Umhüllungsreaktion mit einer geringen Menge eines aliphatischen Diamins ergibt ein Polyharnstoffumhülltes Polyisocyanat verminderter Reaktivität, das zu stabilen Einkomponenten-PU-Reaktivmischungen führt.

**Beispiel 39**

In jeweils 1000 g eines linearen Polypropylenetherdiols (Molekulargewicht 2000) werden gelöst:

Versuch 1 1,66 g Hydrazinhydrat

Versuch 2 3,0 g Methylhydrazin

Versuch 3 2,0 g Hydrazinoethanol

Man rührt jeweils 35 g dimeres Toluylendiisocyanat als feingemahlenes Pulver ein. Nach 30 Minuten Rühren bei Raumtemperatur werden der Suspension des stabilisierten Polyisocyanats jeweils 89 g 2,4-/2,6-Diamino-3,5-di-ethyltoluol-Isomerengemisch (65/35) und jeweils 2,0 g Pb-Octoat-Lösung zugesetzt.

Die Mischungen sind lagerstabil und besitzen die in Tabelle 30 angegebenen Aufdickungstemperaturen.

Zur Herstellung von Elastomerprüfplatten wird die Gießmasse jeweils in eine mit Silikontrennmittel eingestrichene offene Gießform eingefüllt und 6 Stunden bei 120°C verfestigt und getempert. Man erhält Elastomere mit den in der Tabelle 31 angegebenen Eigenschaften:

**Tabelle 30**

| Vers.-Nr (Bsp. 2) | Art des "Amin-stabilisators" | Menge des Stabilisators in g | inÄquiv.-% | Aufdickungstemperatur in °C |
|---|---|---|---|---|
| 1) | Hydrazinhydrat | 3,32 | 3,33 | 60 |
| 2) | Methylhydrazin | 6,0 | 12,0 | 90 |
| 3) | Hydrazinoethanol | 4,0 | 6,0 | 85 |
| 4) | ohne (Vergleichsversuch) | augenblickliche Aufdickung nach DETA-Zugabe und vollständige Vernetzung innerhalb einer Woche bei Raumtemperatur *) | | |

*) "Aufdickung" bereits bei Raumtemeperatur kennzeichnet eine in diesem Fall nicht ausreichende "Stabilisierungswirkung" (Umhüllungsreaktion) durch den "Stabilisator".

**Tabelle 31**

Mechanische Eigenschaften von ausgeheizten Polyurethanen

| Bsp. 3/Vers.-Nr. | | 1 | 2 | 3 |
|---|---|---|---|---|
| Zugfestigkeit ***) (DIN 53 504) | [MPa] | 8,9 | 8,7 | 8,3 |
| Reißdehung (DIN 53 504) | [%] | 150 | 160 | 165 |
| Weiterreißfestigkeit ***) (nach DIN 53 515) | [KN/m] | 11,0 | 10,6 | 11,1 |
| Shore-Härte ***) (nach DIN 53 505) | -A | 91 | 88 | 87 |
| | -D | 34 | - | - |
| Elastizität ***) (DIN 53 512) | [%] | 46 | 47 | 44 |

***) Auch in den folgenden Beispielen erfolgt die Bestimmung der Werte nach den angegebenen DIN-Vorschriften

**Beispiel 40**

In 1000 g eines höhermolekularen, aromatischen Polyetherdiamins der NH-Zahl 80,4 (Herstellung s. Beispiel 29 c)) werden 40,0 g einer Lösung von Oxalsäurehydrazid-Methylhydrazid

FIG4/20

(10-%-ig in Ethylenglykol) verteilt.

In diese Hydrazid-Lösung im Aminopolyether werden 475 g feinpulverisiertes dimeres Toluylen-2,4-diisocyanat suspendiert. Man erhält eine bei Raumtemperatur vollkommen stabile Gießmasse (Lagerzeit 3 Monate bei Raumtemperatur geprüft). Die Gießmasse besitzt bei 50° C eine Viskosität von 18 Pa.s und eine Lagerzeit bei 50° C von mehreren Tagen.

Oberhalb von 60° C dickt die Gießmasse unter Polymerbildung auf, d.h. bei langsamem Erwärmen von 10 bis 20 g der Gießmasse tritt oberhalb 60° C eine schnelle Eindickung auf.

Zur Herstellung einer Elastomerplatte wird die Gießmasse in eine mit Silikon-Trennmittel eingestrichene, offene Gießform eingefüllt und 6 Stunden bei 120° C verfestigt und getempert. Man erhält ein Elastomer mit den in Tabelle 32 angegebenen Eigenschaften.

**Tabelle 32**

Mechanische Zigenschaften von ausgeheizten Polyurethanen

| Beispiel-Nr. | | 40 | 41 | 42 |
|---|---|---|---|---|
| Zugfestigkeit ***) (DIN 53 504) | [MPa] | 22 | 25 | 16 |
| Reißdehung (DIN 53 504) | [%] | 100 | 400 | 800 |
| Weiterreißfestigkeit ***) nach (DIN 53 515 | [KN/m] | 50 | 55 | 46 |
| Shore-Härte ***) (nach Din 53 505) | -A | 99 | 94 | 89 |
| " | -D | 64 | 39 | 33 |
| Elastizität ***) (DIN 53 512) | [%] | 38 | 52 | 60 |

**Beispiel 41**

In 1000 g eines höhermolekularen aromatischen Polyetherdiamins der NH-Zahl 47,4 (Herstellung s. Beispiel 33 c)) werden 3,0 g einer 20-%-igen Lösung von β-Semicarbazidopropionsäurehydrazid $H_2N.H_2N.CO.NH.CH_2.CH_2.CO.NH.NH_2$ in heißem Diethylenglykol verrührt und in diese Mischung 166 g dimeres

Toluylen-2,4-diisocyanat suspendiert. Man erhält eine bei 50° C vollkommen lagerstabile Gießmasse mit einer Viskosität von 3 Pa.s bei 50° C. Die Gießmasse zeigt eine sehr schnelle Aufdickung bei Temperaturen ab 78° C.

Die Gießmasse wird wie in Beispiel 40 beschrieben zu einer Elastomerprüfplatte thermisch verfestigt und diese zeigt die in Tabelle 4 aufgeführten Eigenschaften.

**Beispiel 42**

a) Suspension eines Hydrazid-stabilisierten Polyisocyanats in einem höhermolekularen Polyamin

In 1000 g eines höhermolekularen Polyetherpolyamins mit aromatischen Endgruppen und einer NH-Zahl von 37,8 (Herstellung s.u.) werden 8,0 g einer 50-%-igen Lösung von Ethylenbissemicarbazid

$H_2N.NH.CO.NH.CH_2.CH_2.NH.CO.NH.NH_2$

in heißem Wasser eingerührt und die Mischung bei 100° C bei 0,3 mbar entwässert.

In diese Lösung des Bissemicarbazids im Aminopolyether werden 130 g dimeres Toluylen-2,4-diisocyanat suspendiert. Man erhält eine bis 50° C vollkommen lagerstabile Gießmasse mit einer Viskosität von 1,3 Pa.s bei 50° C. Die Gießmasse zeigt eine Aufdickungstemperatur von 90° C.

b) Verwendung

Zur Herstellung einer Elastomer-Prüfplatte wird die Gießmasse wie in Beispiel 41 in Formen bei 120° C/6 h verfestigt und getempert. Die Elastomereigenschaften sind in Tabelle 32 wiedergegeben.

c) Das höhermolekulare Polyetherpolyamin wird wie folgt hergestellt:

1 Mol eines linearen Polypropylenglykols der OH-Zahl 42,6 und 2 Mol Toluylen-2,4-diisocyanat werden durch 4-stündiges Erhitzen auf 80° C in ein NCO-Prepolymer (2,82 % NCO) überführt. Dann werden 1000 g des 45° C warmen NCO-Prepolymers unter intensivem Rühren so zu einer gekühlten Lösung von 52,2 g Kaliumhydroxid und 500 ml Wasser und 400 ml Aceton gegeben (NCO : $OH^{\ominus}$-Verhältnis = 1 : 1,35), daß eine Innentemperatur von 25° C nicht überschritten wird. 30 Minuten wird bei dieser Temperatur weitergerührt und dann 2 Stunden zum Rückfluß erhitzt. Die Aufarbeitung erfolgt wie in Beispiel 29 a) angegeben. Der Aminopolyether hat eine NH-Zahl von 37,8.

**Beispiel 43**

a) Herstellung einer lagerstabilen Beschichtungspaste

In 1000 g eines linearen Aminopolyethers der NH-Zahl 47,4 (nach Beispiel 33 c)) werden eine 50° C warme Lösung von 0,8 g β-Semicarbazido-propionsäurehydrazid in 2,4 g Diethylenglykol zusammen mit 10 g 2,4-/2,6-Diamino-3,5-diethyltoluol-Isomeren-gemisch (65/35) - DETA - untergerührt und mit 186 g feingepulvertem 4,4'-Diisocyanato-3,3'-dimethyl-diphenyluretdion (dimeres Toluylendiisocyanat) versetzt. Die erhaltene PU-Reaktivmischung mit dem suspendierten, Polyaddukt-umhüllten Diisocyanat ist trotz der Anwesenheit des gelösten aromatischen Diaminkettenverlängerers DETA bei Raumtemperatur lagerstabil. Zur Pigmentierung werden der Reaktivmischung 10 Gew.-% einer Pigmentanreibung von 50 Teilen Titandioxid in 50 Teilen Dioctylphthalat zugefügt. Die Viskosität der Streichpaste beträgt 20 000 mPa.s/25° C.

b) Verwendung zur Textilbeschichtung

Ein Polyestergewebe (220 g/m²) wird auf einer Beschichtungsanlage im Direktstreichverfahren beidseitig beschichtet. Über eine Rakelvorrichtung wird die Beschichtungsmasse auf der ersten Seite des Gewebes in einer Menge von 250 g/m² aufgebracht und in einem 12 m langen Trockenkanal bei einer Fahrgeschwindigkeit von 6 m/min bei Kanaltemperaturen von 120/130/130° C zur Reaktion gebracht.

In der gleichen Verfahrensweise wird das Gewebe anschließend auf der anderen Seite mit 180 g/m² beschichtet und ausgeheizt. Es wird eine beidseitig blasen- und lunkerfreie Beschichtung erhalten, wobei eine hervorragende Haftung der Beschichtungslage zum nicht-grundierten Polyestergewebe erzielt wird.

Stellt man getrennt durch Aufrakeln auf ein Trennpapier in gleichartiger Weise einen Film aus der Streichpaste her, so liegt der 100 %-Modul des Filmes bei 10 bis 11 mPa.

**Beispiel 44**

a) Herstellung einer lagerstabilen Beschichtungspaste

750 g eines linearen, aromatischen Aminopolyethers der NH-Zahl 47,4 (Herstellung s. Beispiel 33 c)) und 250 g eines aliphatischen Amino-polyoxypropylenethers der NH-Zahl 35 (hergestellt durch reduktive Aminierung eines auf Trimethylolpropan verzweigten Polyoxypropylenglykols) werden mit 1,3 g 3,3'-Dimethyl-4,4'-diaminodicyclohexylmethan vermischt. Unter Rühren werden 150 g feinteiliges, dimeres Toluylen-2,4-diisocyanat eingetragen, wobei sich eine mit Polyharnstoff umhüllte Diisocyanatsuspension in Polyaminoethern (Viskosität 10 000 mPa.s/25° C, Lagerzeit mindestens 3 Monate) bildet.

b) Verwendung zur lösungsmittelfreien Textilbeschichtung

Die Suspension nach a) wird wie in Beispiel 43 b) auf ein nicht vorbehandeltes Polyamidgewebe von 180 $g/m^2$ Gewicht im Direktstreichverfahren mit je 100 $g/m^2$ beidseitig aufgetragen und jeweils bei 130°C im Trockenkanal zum Polyurethan(harnstoff) ausreagiert. Die elastomere Beschichtungsschicht weist eine hervorragende Haftung zum Polyamidgewebe auf. Die Knickechtheit (bestimmt mit dem Bally-Flexometer) ist sowohl bei Raumtemperatur als auch bei -10°C gut (10 000 bzw. 20 000 Knickungen).

**Patentansprüche** für die Vertragsstaaten CH und LI.

1. Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb 30°C in feinteiliger Form und einer Teilchengröße von 0,5 bis 200 µm, mit zwei- oder mehrfunktionellen, nieder- und/oder höhermolekularen Verbindungen mit aliphatisch gebundenen primären und/oder sekundären Aminogruppen und/oder -CO.NH.$NH_2$-Endgruppen und/oder Hydrazin(en), Alkyl-substituierten Hydrazinen und/oder N,N'-Dialkylhydrazinen wie N,N'-Dimethylhydrazin, und einem Molekulargewicht von 32 bis 6000, oder ihren Mischungen, als "Amin-Stabilisatoren" in einer Menge von 0,1 bis 25 Äquivalentprozent Amin pro NCO, in einem flüssigen Medium aus nieder- und/oder höhermolekularen Mono- und/oder Polyolen und/oder nieder- und/oder höhermolekularen aromatischen Polyaminen und/oder höhermolekularen aliphetischen Polyaminen und/oder Weichmachern und gegebenenfalls Wasser, gegebenenfalls in Gegenwart von unpolaren oder wenig polaren Lösungsmitteln bei Temperaturen unterhalb der Schmelztemperatur der Polyisocyanate zu einer Suspension von Polyaddukt-umhüllten stabilisierten Polyisocyanaten in dem flüssigen Medium umsetzt und gegebenenfalls die stabilisierten Polyisocyanate aus den Monoalkoholen, Weichmachern, Wasser und/oder Lösungsmitteln isoliert und in den Polyolen und/oder Polyaminen suspendiert.

2. Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb 30°C in feinteiliger Form und mit einer Teilchengröße von 0,5 bis 200 µm, mit Hydrazin, Alkylhydrazinen, N,N'-Dialkylhydrazinen mit $C_1$-$C_6$-Alkylgruppen wie N,N'-Dimethylhydrazin und/oder zwei- oder mehrfunktionellen, nieder- und/oder höhermolekularen Verbindungen mit -CO.NH.$NH_2$-Endgruppen und einem Molekulargewicht von 32 bis 6000 oder ihren Mischungen als "Amin-Stabilisatoren" in einer Menge von 0,1 bis 25 Äquivalentprozent einer Hydrazin- oder Hydrazid-Endgruppe pro NCO-Gruppe in einem flüssigen Medium aus nieder- und/oder höhermolekularen Mono- und/oder Polyolen und/oder nieder- und/oder höhermolekularen aromatischen Polyaminen und/oder höhermolekularen aliphatischen Polyaminen mit Molekulargewichten von 60 bis 6000 und/oder Weichmachern und gegebenenfalls Wasser, gegebenenfalls in Gegenwart von unpolaren oder wenig polaren Lösungsmitteln, bei Temperaturen unterhalb des Schmelzpunktes der Polyisocyanate zu einer Suspension von Polyaddukt-umhüllten, stabilisierten Polyisocyanaten in dem flüssigen Medium umsetzt und gegebenenfalls die stabilisierten Polyisocyanate aus den Monoalkoholen, Weichmachern, Wasser oder wenig polaren Lösungsmitteln isoliert und anschließend in den Polyolen und/oder Polyaminen suspendiert.

3. Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb 30°C in feinteiliger Form und einer Teilchengröße von 0,5 bis 200 µm mit zwei- oder mehrfunktionellen, nieder- und/oder höhermolekularen Verbindungen mit aliphatisch gebundenen primären und/oder sekundären Aminogruppen und/oder -CO.NH.$NH_2$-Endgruppen und/oder Hydrazin(en), Alkyl-substituierten Hydrazinen und/oder N,N'-Dialkylhydrazinen wie N,N'-Dimethylhydrazin und einem Molekulargewicht von 32 bis 6000, oder ihren Mischungen, als "Amin-Stabilisatoren" in einer Menge von 0,1 bis 25 Äquivalentprozent Amin pro NCO, in einem flüssigen Medium aus höhermolekularen aromatischen und/oder aliphatischen Polyaminen mit Molekulargewichten von 400 bis 6000 und gegebenenfalls niedermolekularen aromatischen Polyaminen sowie gegebenenfalls nieder- und/oder höhermolekularen Polyolen und/oder gegebenenfalls Weichmachern oder Wasser, gegebenenfalls in Gegenwart von unpolaren oder wenig polaren Lösungsmitteln bei Temperaturen unterhalb der Schmelztemperatur der Polyisocyanate, zu einer Suspension von Polyaddukt-umhüllten, stabilisierten Polyisocyanaten in den höhermolekularen Polyaminen umsetzt und gegebenenfalls die stabilisierten Polyisocyanate aus den Weichmachern, Wasser und/oder wenig polaren Lösungsmitteln isoliert und anschließend in den höhermolekularen Polyaminen suspendiert.

4. Verfahren nach Ansprüchen 1 bis 3, dadurch gekennzeichnet, daß man als feste Polyisocyanate solche mit Schmelzpunkten oberhalb 80°C aus der Reihe 1,5-Naphthalindiisocyanat, 1,4-Phenylendiiocyanat, 2,3'-Diisocyanato-4,4'-dimethyl-N,N'-diphenylharnstoff, dimeres 1-Methyl-2,4-diisocyanatobenzol, dimeres 4,4'-Diisocyanato-diphenylmethan oder 3-3'-Dimethyl-4,4'-diisocyanatodiphenyl einsetzt.

5. Verfahren nach Ansprüchen 1 bis 4, dadurch gekennzeichnet, daß man 0,1 bis 8 Äquivalentprozent der "Amin-Stabilisatoren" pro NCO-Gruppe einsetzt.

6. Verfahren nach Ansprüchen 1 bis 5, dadurch gekennzeichnet, daß man die Umsetzung der festen Polyisocyanate mit den "Amin-Stabilisatoren" direkt in höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen als Kettenverlängerungsmittel mit Molekulargewichten von 60 bis 399, unter Bildung der Suspension durchführt.

7. Verfahren nach Ansprüchen 1 bis 6, dadurch gekennzeichnet, daß man die Komponenten in solchen Mengen umsetzt, wie dies der Rezeptur der Einkomponenten-PU-Reaktivsysteme entspricht.

8. Verfahren nach Ansprüchen 1 und 4 bis 7, dadurch gekennzeichnet, daß man die festen Polyisocyanate mit zwei- oder mehrfunktionellen, nieder- und/oder höhermolekularen Verbindungen mit aliphatisch gebundenen Aminogruppen und Molekulargewichten von 32 bis 6000 als "Amin-Stabilisatoren" in einem flüssigen Medium aus höhermolekularen Polyonen und niedermolekularen aromatischen Polyaminen als Kettenverlängerungsmittel, gegebenefalls zusätlich 0 bis 90 Mol-% niedermolekularen Polyolen als Kettenverlängerungsmitteln, zu einer Suspension von Polyaddukt-umhüllten, stabilisierten Polyisocyanaten in dem flüssigen Medium umsetzt.

9. Verfahren nach Anspruch 1, dadurch gekennzeichnet, daß man die festen Polyisocyanate mit "Amin-Stabilisatoren" auf Basis niedermolekularer aliphatischer Polyamine in Wasser als flüssigem Medium in Gegenwart von einer nieder- und/oder höhermolekularen Polyolkomponente und/oder einer nieder- oder höhermolekularen Polyaminkomponente, vorzugsweise 2 bis 25 Gew.-% einer höhermolekularen Polyol- oder Polyaminkomponente, umsetzt, das stabilisierte Polyisocyanat abfiltriert, gegebenenfalls vorsichtig trocknet und in der isolierten, feinpulvrigen Form den höhermolekularen Polyolen und/oder Polyaminen und gegebenenfalls weiteren Ausgangskomponenten für die Einkomponenten-PU-Reaktivmischung zumischt.

10. Verfahren nach Ansprüchen 3 bis 5 und 7, dadurch gekennzeichnet, daß man als höhermolekulare Polyaminoverbindung solche aromatischen Polyaminoverbindungen verwendet, wie sie durch Hydrolyse von entsprechenden NCO-Prepolymeren auf der Basis höhermolekularer Polyhydroxylverbindungen und überschüssigen aromatischen Diisocyanaten durch, vorzugsweise basische, Hydrolyse erhalten werden.

11. Verfahren nach Ansprüchen 3 bis 5, und 7, dadurch gekennzeichnet, daß man die festen Polyisocyanate bei niedrigen Temperaturen, vorzugsweise Raumtemperatur, in höhermolekularen, aliphatischen Polyaminen als Aminstabilisatoren und flüssigem Medium in Suspension umsetzt.

12. Polyaddukt-umhüllte, stabilisierte, feste, feinteilige Polyisocyanate retardierter Reaktivität nach Verfahrensansprüchen 1 bis 11 suspendiert in nieder- und/oder höhermolekularen Polyolen und/oder nieder- und/oder höhermolekularen aromatischen Polyaminen und/oder höhermolekularen aliphatischen Polyaminen, mit einem NCO-Restgehalt von mindestens 75 % und weniger als 99,9 % der ursprünglich vorhandenen NCO-Gruppen der unstabilisierten Polyisocyanate, und einer Aufdickungstemperatur dieser Suspension von oberhalb 55°C.

13. Polyaddukt-umhüllte Polyisocyanate nach Anspruch 12, suspendiert in einem Medium aus höhermolekularen Polyolen und/oder höhermolekularen aromatischen und/oder aliphatischen Polyaminen mit Molekulargewichten von 400 bis 6000, und niedermolekularen Polyolen und/oder aromatischen Polyaminen mit Molekulargewichten von 60 bis 399.

14. Polyaddukt-umhüllte Polyisocyanate nach Anspruch 12, suspendiert in höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen des Molekulargewichts 62 bis 399 und gegebenenfalls untergeordneten Mengen, bezogen auf höhermolekulare Polyamine, an höhermolekularen Polyolen.

15. Polyaddukt-umhüllte, stabilisierte, feste, feinteilige Polyisocyanate nach Ansprüchen 12 und 13 dadurch gekennzeichnet, daß nach Verfahren 1 bis 8 die festen Polyisocyanate, mit zwei- oder mehrfunktionellen, nieder- und/oder höhermolekularen Verbindungen mit aliphatisch gebundenen Aminogruppen und Molekulargewichten von 32 bis 6000 als "Amin-Stabilisatoren" in einem flüssigen Medium aus höhermolekularen Polyolen und niedermolekularen, aromatischen Polyaminen als Kettenverlängerungsmitteln sowie gegebenenfalls zusätzlich 0 bis 90 Mol-% an niedermolekularen Polyolen als Kettenverlängerungsmitteln hergestellt werden oder daß man die Polyaddukt-umhüllten Polyisocyanate durch Umsetzung der festen Polyisocyanate mit den "Amin-Stabilisatoren" in einem flüssigen Medium aus höhermolekularen Polyolen herstellt und die niedermolekularen, aromatischen Polyamine und gegebenenfalls die zusätzlichen niedermolekularen Polyole als Kettenverlängerungsmittel anschließend zumischt.

16. Polyaddukt-umhüllte, stabilisierte, feste, feinteilige Polyisocyanate retardierter Reaktivität, hergegestellt nach Anspruch 2, suspendiert in nieder- und/oder höhermolekularen Polyolen und/oder nieder- und/oder höhermolekularen aromatischen Polyaminen und/oder höhermolekularen aliphatischen Polyaminen, mit einem NCO-Restgehalt von mindestens 75 % und weniger als 99,9 % der ursprünglich vorhandenen NCO-Gruppen der unstabilisierten Polyisocyanate, und einer Aufdickungstemperatur der Suspension von oberhalb 55°C.

17. Polyaddukt-umhüllte Polyisocyanate nach Ansprüchen 12 bis 14 und 16, dadurch gekennzeichnet, daß sie einen Feststoffgehalt an Polyaddukt-umhüllten Polyisocyanaten von 3 bis 70 Gew.-% in der Suspension aufweisen.

18. Polyaddukt-umhüllte Polyisocyanate nach Ansprüchen 12, 13, 16 und 17, dadurch gekennzeichnet, daß sie in hohermolekularen Polyolen mit Molekulargewichten von 400 bis 6000 unter Zusatz von niedermolekularen aromatischen Diaminen und gegebenenfalls niedermolekularen Polyolen mit Molekulargewichten bis 399 suspendiert sind.

19. Verwendung von Polyaddukt-umhüllten Polyisocyanaten zur Herstellung von Polyurethanen aus
A) Polyisocyanaten
B) höhermolekularen Polyhydroxyl- und/oder Polyaminoverbindungen, gegebenenfalls
C) niedermolekularen Kettenverlängerungsmitteln, gegebenenfalls
D) Polyurethankatalysatoren und gegebenenfalls

E) üblichen Hilfs- und Zusatzstoffen, dadurch gekennzeichnet, daß man die nach Ansprüchen 1 bis 7 erhaltenen, Polyaddukt-umhüllten, suspendierten Polyisocyanate als Polyisocyanat-Komponente A), höhermolekulare Komponente B) und gegebenenfalls niedermolekulare Polyol- und/oder Polyamin-Kettenverlängerungsmittelkomponente C), gegebenenfalls unter Zusatz von weiteren, nicht-stabilisierten Polyisocyanaten A), weiteren höhermolekularen Verbindungen B) und weiteren Kettenverlängerungsmitteln C), gegebenenfalls unter Zusatz von tert.-Amin- und/oder Metallkatalysatoren D) und gegebenenfalls Hilfsstoffen E) zur Herstellung von fließfähigen oder leicht auf schmelzenden Polyurethan-Einkomponenten-Reaktivsystemen mit einer Aufdickungstemperatur von $\geqslant$ 55° C einsetzt, und diese durch Hitze, Scherkräfte und/oder polare Lösungsmittel zu massiven oder zelligen Polyurethanen aushärtet.

20. Verwendung nach Anspruch 19, dadurch gekennzeichnet, daß man die durch Umsetzung von festen Polyisocyanaten in feinteiliger Form mit Hydrazin, Alkylhydrazinen, N,N'-Dialkylhydrazinen (mit $C_1$-$C_6$-Alkylgruppen) wie N,N'-Diemethylhydrazin und/oder mit zwei- oder mehrfunktionellen, nieder- und/oder höherfunktionellen Verbindungen mit -CO.NH.$NH_2$-Endgruppen, und einem Molekulargewicht von 32 bis 6000, oder ihren Mischungen, als "Amin-Stabilisatoren", in einer Menge von 0,1 bis 25 Äquivalentprozent an Aminogruppen, bezogen auf die NCO-Gruppen, in Suspension in höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen vorliegenden, durch Polyadduktumhüllung stabilisierten Polyisocyanate als Komponente A), B) und gegebenenfalls C), gegebenenfalls unter Zusatz von weiteren, nicht-stabilisierten Polyisocyanaten A), höhermolekularen Verbindungen B) und Kettenverlängerungsmitteln C), gegebenenfalls unter Zusatz von tert.-Amin- und/oder Metall-Katalysatoren D) und Hilfsstoffen E) zur Herstellung von PU-Einkomponenten-Reaktivsystemen mit einer Aufdickungstemperatur von $\geqslant$ 55°C einsetzt und diese durch Hitze, Scherkräfte und/oder polare Lösungsmittel zu massiven oder zelligen Polyurethanen aushärtet.

21. Verwendung nach Anspruch 19, dadurch gekennzeichnet, daß man die durch Reaktion von freien Polyisocyanaten in feinteiliger Form mit 0,1 bis 25 Äquivalentprozent Aminogruppen, bezogen auf NCO-Gruppen, mit zwei- oder mehrfunktionellen, nieder- und/ oder höhermolekularen Verbindungen mit aliphatisch gebundenen, primären und/oder sekundären Aminogruppen und/oder -CO.NH.$NH_2$-Endgruppen und/oder Hydrazin(en), Alkyl-substituierten Hydrazinen und/oder N,N'-Dialkylhydrazinen wie N,N'-Dimethylhydrazin, mit einem Molekulargewicht von 32 bis 6000, oder ihren Mischungen, als "Amin-Stabilisatoren" in Suspension in höhermolekularen aromatischen und/oder aliphatischen Polyaminen mit Molekulargewichten von 400 bis 6000 und gegebenenfalls niedermolekularen Polyolen und/oder aromatischen Polyaminen des Molekulargewichts 62 bis 399 und gegebenenfalls untergeordneten Mengen, bezogen auf höhermolekulare Polyamine, an höhermolekularen Polyolen vorliegenden, durch Polyadduktumhüllung stabilisierten Polyisocyanate als Komponente A), B) und gegebenenfalls C), gegebenenfalls unter Zusatz von weiteren, nicht-stabilisierten Polyisocyanaten A), höhermolekularen Verbindungen B) und Kettenverlängerungsmitteln C), gegebenenfalls unter Zusatz von tert.-Amin- und/oder Metall-Katalysatoren D) und Hilfsstofen E) zur Herstellung von PU-Einkomponenten-Reaktivsystemen mit einer Aufdickungstemperatur von $\geqslant$ 55°C einsetzt und diese durch Hitze, Scherkräfte und/oder polare Lösungsmittel zu massiven oder zelligen Polyurethanen aushärtet.

22. Verwendung nach Anspruch 19, dadurch gekennzeichnet, daß man die durch Umsetzung von festen Polyisocyanaten in feinteiliger Form mit zwei- oder mehrfunktionellen, nieder- und/oder höhermolekularen Verbindungen mit aliphatisch gebundenen, primären und/oder sekundären Aminogruppen und einem Molekulargewicht von 60 bis 3000 (oder ihren Mischungen), als "Amin-Stabilisatoren" in einer Menge von 0,1 bis 25 Gew.-% an Aminogruppen, bezogen auf NCO, in Suspension in höhermolekularen Polyolen mit Molekulargewichten von 400 bis 6000, gegebenenfalls unter Zusatz von niedermolekularen aromatischen Polyaminen und/oder niedermolekularen aromatischen Polyaminen und/oder niedermolekularen Polyolen mit Molekulargewichten bis 399, als Polyisocyanatkomponente A) und Komponente B) und als Kettenverlängererkomponente C) niedermolekulare aromatische Polyamine aus der Stabilisierungsreaktion oder als zugesetztes Kettenverlängerungsmittel C) gegebenenfalls unter Zusatz von weiteren, nichtstabilisierten Polyisocyanaten A), höhermolekularen Verbindungen B) und Kettenverlängerungsmitteln C), gegebenenfalls unter Zusatz von tert.-Amin- und/oder Metallkatalysatoren D) und Hilfsstoffen E) zur Herstellung von PU-Einkomponenten-Reaktivsystemen mit einer Aufdickungstemperatur von $\geqslant$ 55°C einsetzt und diese durch Hitze, Scherkräfte und/oder polare Lösungsmittel zu massiven oder zelligen Polyurethanen aushärtet.

23. Verwendung nach Ansprüchen 19 bis 22, dadurch gekennzeichnet, daß das NCO/($NH_2$+OH)-Äquivalent-Verhältnis von A/(B+C) bei der Polyurethanbildung 0,5 : 1 bis 1,5 : 1 beträgt.

24. Verwendung nach Ansprüchen 19 bis 23, dadurch gekennzeichnet, daß pro ($NH_2$+OH)-Äquivalent an höhermolekularen Polyolen und/oder Polyaminen (B) 0,3 bis 10 (OH+$NH_2$)-Äquivalente an niedermolekularen Polyolen und/oder Polyaminen (C) zur Polyurethanbildung verwendet werden.

25. Verwendung nach Ansprüchen 19 bis 24, dadurch gekennzeichnet, daß man als Katalysatoren D) 0,001 bis 5 Gew.-% bezogen auf A+B, an organischen Blei- und/oder Zinnverbindungen in den Einkomponentensystemen einsetzt.

26. Verwendung nich Ansprüchen 19 bis 25 dadurch gekennzeichnet, daß man organische Bleiverbindungen aus der Reihe

a) organischer Salze des zweiwertigen Bleis mit Carbonsäuren,

b) Dithiocarbamate des zweiwertigen Bleis der Formel

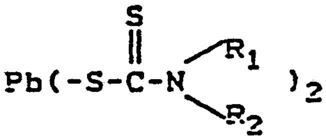

worin

$R_1$ und $R_2$ verschieden sein können und einen $C_1$-$C_{20}$-Alkylrest bedeuten,

c) Tetraorgano-Blei-IV-Verbindungen, wobei der organische Rest einen niederen Alkylrest, wie z. B. Methyl oder Ethyl, bedeutet und

d) Verbindungen aus 1,3-Dicarbonylverbindungen, wie z. B. Acetylaceton, mit zweiwertigem Blei einsetzt, wenn in den Einkomponentensystemen Polyetherpolyole (B) mit sekundären Hydroxylgruppen verwendet werden und daß man organische Zinnverbindungen aus der Gruppe von Zinn(II)-Salzen von Carbonsäuren oder Zinn(IV)-Verbindungen wie Dibutylzinnoxid, Dibutylzinndichlorid, Dibutylzinndiacetat, Dibutylzinndilaurat, Dibutylzinnmaleat oder schwefelhaltige Zinnverbindungen der Formel

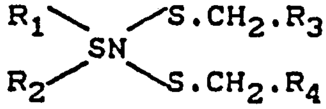

worin

$R_1$ und $R_2$ Alkylreste mit 1 bis 10 C-Atomen,

$R_3$ und $R_4$ Wasserstoff und/oder $C_1$-$C_{18}$-Alkyl und/oder den Rest $COOR_1$ bedeuten,

zusetzt, wenn in den Einkomponentensystemen Polyole B) mit im wesentlichen primären Hydroxylgruppen verwendet werden.

27. Verwendung nach Ansprüchen 19, 23 und 24, dadurch gekennzeichnet, daß man Polyaddukt-umhüllte Polyisocyanate nach Anspruch 15 einsetzt.

**Patentansprüche** für die Vertragstaaten AT, BE, DE, FR, GB, IT, NL, SE

1. Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb 30°C in feinteiliger Form und einer Teilchengröße von 0,5 bis 200 µm, mit zwei- oder mehrfunktionellen, nieder- und/oder höhermolekularen Verbindungen mit aliphatisch gebundenen primären und/oder sekundären Aminogruppen und/oder -CO.NH.$NH_2$-Erdgruppen und/oder Hydrazin(en), Alkyl-substituierten Hydrazinen und/oder N,N'-Dialkylhydrazinen wie N,N'-Dimethylhydrazin, und einem Molekulargewicht von 32 bis 6000, oder ihren Mischungen, als "Amin-Stabilisatoren" in einer Menge von 0,1 bis 25 Äquivalentprozent Amin pro NCO, in einem flüssigen Medium aus nieder- und/oder höhermolekularen Mono- und/oder Polyolen und nieder- und/oder höhermolekularen aromatischen Polyaminen und/oder höhermolekularen aliphatischen Polyaminen und/oder Weichmachern und gegebenenfalls Wasser, gegebenenfalls in Gegenwart von unpolaren oder wenig polaren Lösungsmitteln bei Temperaturen unterhalb der Schmelztemperatur der Polyisocyanate zu einer Suspension von Polyaddukt-umhüllten stabilisierten Polyisocyanaten in dem flüssigen Medium umsetzt und gegebenenfalls die stabilisierten Polyisocyanate aus den Monoalkoholen, Weichmachern, Wasser und/oder Lösungsmitteln isoliert und in den Polyolen und Polyaminen suspendiert.

2. Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb 30°C in feinteiliger Form und mit einer Teilchengröße von 0,5 bis 200 µm, mit Hydrazin(hydrat), Alkylhydrazinen, N,N'-Dialkylhydrazinen mit $C_1$-$C_6$-Alkylgruppen wie N,N'-Dimethylhydrazin und/oder zwei- oder mehrfunktionellen, nieder- und/oder höhermolekularen Verbindungen mit -CO.NH.$NH_2$-Endgruppen und einem Molekulargewicht von 32 bis 6000 oder ihren Mischungen als "Amin-Stabilisatoren" in einer Menge von 0,1 bis 25 Äquivalentprozent einer Hydrazin- oder Hydrazid-Endgruppe pro NCO-Gruppe in einem flüssigen Medium aus nieder- und/oder höhermolekularen Mono- und/oder Polyolen und/oder nieder- und/oder höhermolekularen aromatischen Polyaminen und/oder höhermolekularen aliphatischen Polyaminen mit Molekulargewichten von 60 bis 6000 und/oder Weichmachern und gegebenenfalls Wasser, gegebenenfalls in Gegenwart von unpolaren oder wenig polaren Lösungsmitteln, bei Temperaturen unterhalb des Schmelzpunktes der Polyisocyanate zu einer Suspension von Polyaddukt-umhüllten, stabilisierten Polyisocyanaten in dem flüssigen Medium umsetzt und gegebenenfalls die stabilisierten Polyisocyanate aus den Monoalkoholen, Weichmachern, Wasser oder wenig polaren Lösungsmitteln isoliert und anschließend in den Polyolen und/oder Polyaminen suspendiert.

3. Verfahren zur Herstellung von durch Polymerumhüllung stabilisierten, festen Polyisocyanaten mit retardierter Reaktivität, dadurch gekennzeichnet, daß man feste Polyisocyanate mit Schmelzpunkten oberhalb

30°C in feinteiliger Form und einer Teilchengröße von 0,5 bis 200 µm mit zwei- oder mehrfunktionellen, nieder- und/oder höhermolekularen Verbindungen mit aliphatisch gebundenen primären und/oder sekundären Aminogruppen und/oder -CO.NH.$NH_2$-Endgruppen und/oder Hydrazin(hydrat), Alkyl-substituierten Hydrazinen und/oder N,N'-Dialkylhydrazinen wie N,N'-Dimethylhydrazin und einem Molekulargewicht von 32 bis 6000, oder ihren Mischungen, als "Amin-Stabilisatoren" in einer Menge von 0,1 bis 25 Äquivalentprozent Amin pro NCO, in einem flüssigen Medium aus höhermolekularen aromatischen und/oder aliphatischen Polyaminen mit Molekulargewichten von 400 bis 6000 und gegebenenfalls niedermolekularen aromatischen Polyaminen sowie gegebenenfalls nieder- und/oder höhermolekularen Polyolen und/oder gegebenenfalls Weichmachern oder Wasser, gegebenenfalls in Gegenwart von unpolaren oder wenig Polaren Lösungsmitteln bei Temperaturen unterhalb der Schmelztemperatur der Polyisocyanate, zu einer Suspension von Polyaddukt-umhüllten, stabilisierten Polyisocyanaten in den höhermolekularen Polyaminen umsetzt und gegebenenfalls die stabilisierten Polyisocyanate aus den Weichmachern, Wasser und/oder wenig polaren Lösungsmitteln isoliert und anschließend in den höhermolekularen Polyaminen suspendiert.

4. Verfahren nach Ansprüchen 1 bis 3, dadurch gekennzeichnet, daß man als feste Polyisocyanate solche mit Schmelzpunkten oberhalb 80°C aus der Reihe 1,5-Naphthalin-diisocyanat, 1,4-Phenylendiisocyanat, 2,3'-Diisocyanato-4,4'-dimethyl-N,N'-diphenylharnstoff, dimeres 1-Methyl-2,4-diisocyanatobenzol, dimeres 4,4'-Diisocyanato-diphenylmethan oder 3,3'-Dimethyl-4,4'-diisocyanatodiphenyl einsetzt.

5. Verfahren nach Ansprüchen 1 bis 4, dadurch gekennzeichnet, daß man 0,1 bis 8 Äquivalentprozent der "Amin-Stabilisatoren" pro NCO-Gruppe einsetzt.

6. Verfahren nach Ansprüchen 1 bis 5, dadurch gekennzeichnet, daß man die Umsetzung der festen Polyisocyanate mit den "Amin-Stabilisatoren" direkt in höhermolekularen Polyolen und höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen als Kettenverlängerungsmittel mit Molekulargewichten von 60 bis 399, unter Bildung der Suspension durchführt.

7. Verfahren nach Ansprüchen 1 bis 6, dadurch gekennzeichnet, daß man die Komponenten in solchen Mengen umsetzt, wie dies der Rezeptur der Einkomponenten-PU-Reaktivsysteme entspricht.

8. Verfahren nach Ansprüchen 1 und 4 bis 7, dadurch gekennzeichnet, daß man die festen Polyisocyanate mit zwei- oder mehrfunktionellen, nieder- und/oder höhermolekularen Verbindungen mit aliphatisch gebundenen Aminogruppen und Molekulargewichten von 32 bis 6000 als "Amin-Stabilisatoren" in einem flüssigen Medium aus höhermolekularen Polyolen und niedermolekularen, aromatischen Polyaminen als Kettenverlängerungsmittel, gegebenenfalls zusätzlich 0 bis 90 Mol-% niedermolekularen Polyolen als Kettenverlängerungsmitteln, zu einer Suspension von Polyaddukt-umhüllten, stabilisierten Polyisocyanaten in dem flüssigen Medium umsetzt.

9. Verfahren nach Anspruch 1, dadurch gekennzeichnet, daß man die festen Polyisocyanate mit "Amin-Stabilisatoren" auf Basis niedermolekularer aliphatischer Polyamine in Wasser als flüssigem Medium in Gegenwart von eine nieder- und/oder höhermolekularen Polyolkomponente und/oder einer nieder- oder höhermolekularen Polyaminkomponente, vorzugsweise 2 bis 25 Gew.-% einer höhermolekularen Polyol- oder Polyaminkomponente, umsetzt, das stabilisierte Polyisocyanat abfiltriert, gegebenenfalls vorsichtig trocknet und in der isolierten, feinpulvrigen Form den höhermolekularen Polyolen und/oder Polyaminen und gegebenenfalls weiteren Ausgangskomponenten für die Einkomponenten-PU-Reaktivmischung zumischt.

10. Verfahren nach Ansprüchen 1 bis 5 und 7, dadurch gekennzeichnet, daß man als höhermolekulare Polyaminoverbindung solche aromatischen Polyaminoverbindungen verwendet, wie sie durch Hydrolyse von entsprechenden NCO-Prepolymeren auf der Basis höhermolekularer Polyhydroxylverbindungen und überschüssigen aromatischen Diisocyanaten durch, vorzugsweise basische, Hydrolyse erhalten werden.

11. Verfahren nach Anspruch 3, 4, 5 und 7, dadurch gekennzeichnet, daß man die festen Polyisocyanate bei niedrigen Temperaturen, vorzugsweise Raumtemperatur, in höhermolekularen, aliphatischen Polyaminen als Aminstabilisatoren und flüssigem Medium in Suspension umsetzt.

12. Polyaddukt-umüllte stabilisierte feste, feinteilige Polyisocyanate retardierter Reaktivität nach Verfahrensansprüchen 1 bis 11 suspendiert in nieder- und/oder höhermolekularen Polyolen und nieder- und höhermolekularen aromatischen Polyaminen und/oder höhermolekularen aliphatischen Polyaminen, mit einem NCO-Restgehalt von mindestens 75 % und weniger als 99,9 % der ursprünglich vorhandenen NCO-Gruppen der unstabilisierten Polyisocyanate, und einer Aufdickungstemperatur dieser Suspension von oberhalb 55°C.

13. Polyaddukt-umhüllte Polyisocyanate nach Anspruch 12 suspendiert in einem Medium aus höhermolekularen Polyolen und höhermolekularen aromatischen und/oder aliphatischen Polyaminen mit Molekulargewichten von 400 bis 6000, und niedermolekularen Polyolen und/oder aromatischen Polyaminen mit Molekulargewichten von 60 bis 399.

14. Polyaddukt-umhüllte Polyisocyanate nach Anspruch 12 suspendiert in höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen des Molekulargewichts 62 bis 399 und gegebenenfalls untergeordneten Mengen, bezogen auf höhermolekulare Polyamine, an höhermolekularen Polyolen.

15. Polyaddukt-umhüllte, stabilisierte, feste, feinteilige Polyisocyanate retardierter Reaktivität, hergestellt nach Anspruch 2, suspendiert in nieder- und/oder höhermolekularen Polyolen und/oder nieder- und/oder höhermolekularen aromatischen Polyaminen und/oder höhermolekularen aliphatischen Polyaminen, mit einem NCO-Restgehalt von mindestens 75 % und weniger als 99,9 % der ursprünglich vorhandenen NCO-Gruppen

der unstabilisierten Polyisocyanate, und einer Aufdickungstemperatur der Suspension von oberhalb 55°C.

16. Polyaddukt-umhüllte Polyisocyanate nach Ansprüchen 12 bis 15, dadurch gekennzeichnet, daß sie einen Feststoffgehalt an Polyaddukt-umhüllten Polyisocyanaten von 3 bis 70 Gew.-% in der Suspension aufweisen.

17. Polyaddukt-umhüllte Polyisocyanate nach Ansprüche 12, 13, 15 und 16, dadurch gekennzeichnet, daß in höhermolekularen Polyolen mit Molekulargewichten von 400 bis 6000 unter Zusatz von niedermolekularen aromatischen Diaminen und gegebenenfalls niedermolekularen Polyolen mit Molekulargewichten bis 399 suspendiert sind.

18. Polyaddukt-umhüllte, stabilisierte, feste, feinteilige Polyisocyanate nach Ansprüchen 12 und 13, dadurch gekennzeichnet, daß nach Verfahren 1 bis 7 und 8 die festen Polyisocyanate mit zwei- oder mehrfunktionellen, nieder- und/oder höhermolekularen Verbindungen mit aliphatisch gebundenen Aminogruppen und Molekulargewichten von 32 bis 6000 als "Amin-Stabilisatoren" in einem flüssigen medium aus höhermolekularen Polyolen und niedermolekularen, aromatischen Polyaminen als Kettenverlängerungsmitteln sowie gegebenenfalls zusätzlich 0 bis 90 Mol-% an niedermolekularen Polyolen als Kettenverlängerungsmitteln hergestellt werden oder daß man die Polyaddukt-umhüllten Polyisocyanate durch Umsetzung der festen Polyisocyanate mit den "Amin-Stabilisatoren" in einem flüssigen Medium aus höhermolekularen Polyolen herstellt und die niedermolekularen, aromatischen Polyamine und gegebenenfalls die zusätzlichen niedermolekularen Polyole als Kettenverlängerungsmittel anschließend zumischt.

19. Verwendung von Polyaddukt-umhüllten Polyisocyanaten zur Herstellung von Polyurethanen aus
A) Polyisocyanaten
B) höhermolekularen Polyhydroxyl- und/oder Polyaminoverbindungen, gegebenenfalls
C) niedermolekularen Kettenverlängerungsmitteln, gegebenenfalls
D) Polyurethankatalysatoren und gegebenenfalls
E) üblichen Hilfs- und Zusatzstoffen,
dadurch gekennzeichnet, daß man die nach Ansprüchen 1 bis 11 erhaltenen, Polyaddukt-umhüllten suspendierten Polyisocyanate als Polyisocyanat-Komponente A), höhermolekulare Komponente B) und gegebenenfalls niedermolekulare Polyol- und/oder Polyamin-Kettenverlängerungsmittelkomponente C), gegebenenfalls unter Zusatz von weiteren, nicht-stabilisierten Polyisocyanaten A), weiteren höhermolekularen Verbindungen B) und weiteren Kettenverlängerungsmitteln C), unter Zusatz von tert.-Amin- und/oder Metallkatalysatoren D) und gegebenenfalls Hilfsstoffen E) zur Herstellung von fließfähigen oder leicht aufschmelzenden Polyurethan-Einkomponenten-Reaktivsystemen mit einer Aufdickungstemperatur von $\geqslant$ 55°C einsetzt, und diese durch Hitze, Scherkräfte und/oder polare Lösungsmittel zu massiven oder zelligen Polyurethanen aushärtet.

20. Verwendung nach Anspruch 19 dadurch gekennzeichnet, daß man die durch Umsetzung von festen Polyisocyanaten in feinteiliger Form mit Hydrazin, Alkylhydrazinen, N,N'-Dialkylhydrazinen (mit $C_1$-$C_6$-Alkylgruppen) wie N,N'-Dimethylhydrazin und/oder mit zwei- oder mehrfunktionellen, nieder- und/oder höherfunktionellen Verbindungen mit -CO.NH.$NH_2$-Endgruppen, und einem Molekulargewicht von 32 bis 6000, oder ihren Mischungen, als "Amin-Stabilisatoren", in einer Menge von 0,1 bis 25 Äquivalentprozent an Aminogruppen, bezogen auf die NCO-Gruppen, in Suspension in höhermolekularen Polyolen und/oder höhermolekularen aromatischen und/oder aliphatischen Polyaminen mit Molekulargewichten von 400 bis 6000, gegebenenfalls unter Zusatz von niedermolekularen Polyolen und/oder aromatischen Polyaminen vorliegenden, durch Polyadduktumhüllung stabilisierten Polyisocyanate als Komponente A), B) und gegebenenfalls C), gegebenenfalls unter Zusatz von weiteren, nicht-stabilisierten Polyisocyanaten A), höhermolekularen Verbindungen B) und Kettenverlängerungsmitteln C), gegebenenfalls unter Zusatz von tert.-Amin- und/oder Metall-Katalysatoren D) und Hilfsstoffen E) zur Herstellung von PU-Einkomponenten-Reaktivsystemen mit einer Aufdickungstemperatur von $\geqslant$ 55°C einsetzt und diese durch Hitze, Scherkräfte und/oder polare Lösungsmittel zu massiven oder zelligen Polyurethanen aushärtet.

21. Verwendung nach Anspruch 19 dadurch gekennzeichnet, daß man die durch Reaktion von freien Polyisocyanaten in feinteiliger Form mit 0,1 bis 25 Äquivalentprozent Aminogruppen, bezogen auf NCO-Gruppen, mit zwei- oder mehrfunktionellen, nieder- und/oder höhermolekularen Verbindungen mit aliphatisch gebundenen, primären und/oder sekundären Aminogruppen und/oder -CO.NH.$NH_2$-Endgruppen und/oder Hydrazin(en), Alkyl-substituierten Hydrazinen und/oder N,N'-Dialkylhydrazinen wie N,N'-Dimethylhydrazin, mit einem Molekulargewicht von 32 bis 6000, oder ihren Mischungen, als "Amin-Stabilisatoren" in Suspension in höhermolekularen aromatischen und/oder aliphatischen Polyaminen mit Molekulargewichten von 400 bis 6000 und gegebenenfalls niedermolekularen Polyolen und/oder aromatischen Polyaminen des Molekulargewichts 62 bis 399 und gegebenenfalls untergeordneten Mengen, bezogen auf höhermolekulare Polyamine, an höhermolekularen Polyolen vorliegenden, durch Polyadduktumhüllung stabilisierten Polyisocyanate als Komponente A), B) und gegebenenfalls C), gegebenenfalls unter Zusatz von weiteren, nichtstabilisierten Polyisocyanaten A), höhermolekularen Verbindungen B) und Kettenverlängerungsmitteln C), gegebenenfalls unter Zusatz von tert.-Amin- und/oder Metall-Katalysatoren D) und Hilfsstoffen E) zur Herstellung von PU-Einkomponenten-Reaktivsystemen mit einer Aufdickungstemperatur von $\geqslant$ 55°C einsetzt und diese durch Hitze, Scherkräfte und/oder polare Lösungsmittel zu massiven oder zelligen Polyurethanen aushärtet.

22. Verwendung nach Anspruch 19, dadurch gekennzeichnet, daß man die durch Umsetzung von festen Polyisocyanaten in feinteiliger Form mit zwei- oder mehrfunktionellen, nieder- und/oder höhermolekularen Verbindungen mit aliphatisch gebundenen, primären und/oder sekundären Aminogruppen und einem Molekulargewicht von 60 bis 3000 (oder ihren Mischungen), als "Amin-Stabilisatoren" in einer Menge von 0,1

bis 25 Gew.-% an Aminogruppen, bezogen auf NCO, in Suspension in höhermolekularen Polyolen mit Molekulargewichten von 400 bis 6000, gegebenenfalls unter Zusatz von niedermolekularen aromatischen Polyaminen und/oder niedermolekularen Polyolen mit Molekulargewichten bis 399, als Polyisocyanatkomponente A) und Komponente B) und als Kettenverlängererkomponente C) niedermolekulare aromatische Polyamine aus der Stabilisierungsreaktion oder als zugesetztes Kettenverlängerungsmittel C) gegebenenfalls unter Zusatz von weiteren, nicht-stabilisierten Polyisocyanaten A), höhermolekularen Verbindungen B) und Kettenverlängerungsmitteln C), gegebenenfalls unter Zusatz von tert.-Amin- und/oder Metallkatalysatoren D) und Hilfsstoffen E) zur Herstellung von PU-Einkomponenten-Reaktivsystemen mit einer Aufdickungstemperatur von $\geqslant$ 55°C einsetzt und diese durch Hitze, Scherkräfte und/oder polare Lösungsmittel zu massiven oder zelligen Polyurethanen aushärtet.

23. Verwendung nach Ansprüchen 19 bis 22, dadurch gekennzeichnet, daß das $NCO/(NH_2+OH)$-Äquivalent-Verhältnis von $A/(B+C)$ bei der Polyurethanbildung 0,5 : 1 bis 1,5 : 1 beträgt.

24. Verwendung nach Ansprüchen 19 bis 23, dadurch gekennzeichnet, daß pro $(NH_2+OH)$-Äquivalent an höhermolekularen Polyolen und/oder Polyaminen (B) 0,3 bis 10 $(OH+NH_2)$-Äquivalente an niedermolekularen Polyolen und/oder Polyaminen (C) zur Polyurethanbildung verwendet werden.

25. Verwendung nach Ansprüchen 19 bis 24, dadurch gekennzeichnet, daß man als Katalysatoren D) 0,001 bis 5 Gew.-% bezogen auf A+B, an organischen Blei- und/oder Zinnverbindungen in den Einkomponentensystemen einsetzt.

26. Verwendung nach Ansprüchen 19 bis 25, dadurch gekennzeichnet, daß man organische Bleiverbindungen aus der Reihe

a) organischer Salze des zweiwertigen Bleis mit Carbonsäuren,

b) Dithiocarbamate des zweiwertigen Bleis der Formel

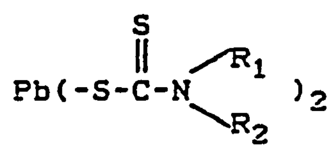

worin

$R_1$ und $R_2$ verschieden sein können und einen $C_1$-$C_{20}$-Alkylrest bedeuten,

c) Tetraorgano-Blei-IV-Verbindungen, wobei der organische Rest einen niederen Alkylrest, wie z. B. Methyl oder Ethyl, bedeutet und

d) Verbindungen aus 1,3-Dicarbonylverbindungen, wie z. B. Acetylaceton, mit zweiwertigem Blei einsetzt, wenn in den Einkomponentensystemen Polyetherpolyole (B) mit sekundären Hydroxylgruppen verwendet werden und

daß man organische Zinnverbindungen aus der Gruppe von Zinn(II)-Salzen von Carbonsäuren oder Zinn(IV)-Verbindungen wie Dibutylzinnoxid, Dibutylzinndichlorid, Dibutylzinndiacetat, Dibutylzinndilaurat, Dibutylzinnmaleat oder schwefelhaltige Zinnverbindungen der Formel

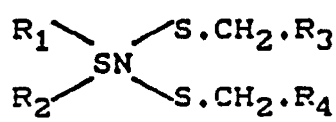

worin

$R_1$ und $R_2$ Alkylreste mit 1 bis 10 C-Atomen,

$R_3$ und $R_4$ Wasserstoff und/oder $C_1$-$C_{18}$-Alkyl und/oder den Rest $COOR_1$ bedeuten,

zusetzt, wenn in den Einkomponentensystemen Polyole B) mit im wesentlichen primären Hydroxylgruppen verwendet werden.

27. Verwendung nach Ansprüchen 19, 23 und 24, dadurch gekennzeichnet, daß man Polyaddukt-umhüllte Polyisocyanate nach Anspruch 18 einsetzt.

**Claims** for the Contacting states AT, BE, DE, FR, GB, IT, NL, SE

1. Process for the production of solid polyisocyanates which are stabilised by a polymer coating and display retarded reactivity characterised in that solid polyisocyanates with melting points above 30°C are reacted in a finely divided form and with a particle size of 0.5 to 200 µm, with di- or higher functional, low and/or relatively high molecular weight compounds containing aliphatically bound, primary and/or secondary amino groups and/or -CO.NH.NH end groups and/or hydrazine(s), alkylsubstituted hydrazines and/or N,N'-dialkylhydrazines such as N,N'-dimethylhydrazine, and having a molecular weight of 32 to 6000, or mixtures thereof, as "amine stabilisers" in a quantity of 0.1 to 25 equivalent per cent of amine per NCO, in a liquid medium of low and/or

relatively high molecular weight mono- and/or polyols and low and/or relatively high molecular weight aromatic polyamines and/or relatively high molecular weight aliphatic polyamines and/or plasticisers and optionally water, optionally in the presence of non-polar or slightly polar solvents at temperatures below the melting temperature of the polyisocyanates to form a suspension of polyadduct-coated stabilised polyisocyanates in the liquid medium and the stabilised polyisocyanates are optionally isolated from the monoalcohols, plasticisers, water and/or solvents and suspended in the polyols and polyamines.

2. Process for the production of solid polyisocyanates which are stabilised by a polymer coating and display retarded reactivity, characterised in that solid polyisocyanates with melting points above 30°C are reacted in a finely divided form and with a particle size of 0.5 to 200 μm, with hydrazine (hydrate), alkyl hydrazines, N,N'-dialkylhydrazines containing $C_1$-$C_6$-alkyl groups such as N,N'-dimethylhydrazine and/or di- or higher functional, low and/or relatively high molecular weight compounds containing -CO.NH.$NH_2$ end groups and having a molecular weight of 32 to 6000 or mixtures thereof as "amine stabilisers" in a quantity of 0.1 to 25 equivalent per cent of a hydrazine or hydrazide end group per NCO group in a liquid medium of low and/or relatively high molecular weight mono- and/or polyols and/or low and/or relatively high molecular weight aromatic polyamines and/or relatively high molecular weight aliphatic polyamines with molecular weights of 60 to 6000 and/or plasticisers and optionally water, optionally in the presence of non-polar or slightly polar solvents, at temperatures below the melting point of the polyisocyanates to form a suspension of polyadduct-coated, stabilised polyisocyanates in the liquid medium and the stabilised polyisocyanates are optionally isolated from the monoalcohols, plasticisers, water or slightly polar solvents and then suspended in the polyols and/or polyamines.

3. Process for the production of solid polyisocyanates which are stabilised by a polymer coating and display retarded reactivity, characterised in that solid polyisocyanates with melting points above 30°C are reacted in a finely divided form and with a particle size of 0.5 to 200 μm with di- or higher functional, low and/or relatively high molecular weight compounds containing aliphatically bound primary and/or secondary amino groups and/or -CO.NH.$NH_2$ end groups and/or hydrazine (hydrate), alkyl-substituted hydrazines and/or N,N'-dialkylhydrazines such as N,N'-dimethylhydrazine and having a molecular weight of 32 to 6000, or mixtures thereof, as "amine stabilisers" in a quantity of 0.1 to 25 equivalent per cent of amine per NCO, in a liquid medium of relatively high molecular weight aromatic and/or aliphatic polyamines with molecular weights of 400 to 6000 and optionally low molecular-weight aromatic polyamines and optionally low and/or relatively high molecular weight polyols and/or optionally plasticisers or water, optionally in the presence of non-polar or slightly polar solvents at temperatures below the melting temperature of the polyisocyanates, to form a suspension of polyadduct-coated, stabilised polyisocyanates in the relatively high molecular weight polyamines and the stabilised polyisocyanates are optionally isolated from the plasticisers, water and/or slightly polar solvents and then suspended in the relatively high molecular weight polyamines.

4. Process according to Claims 1 to 3, characterised in that the solid polyisocyanates used are those with melting points above 80°C from the series comprising 1,5-naphthalene-diisocyanate, 1,4-phenylene diisocyanate, 2,3'-diisocyanato-4,4'-dimethyl-N,N'-diphenylurea, dimeric 1-methyl-2,4-diisocyanatobenzene, dimeric 4,4'-diisocyanato-diphenylmethane or 3,3'-dimethyl-4,4'-diisocyanatodiphenyl.

5. Process according to Claims 1 to 4, characterised in that 0.1 to 8 equivalent per cent of the "amine stabilisers" are used per NCO group.

6. Process according to Claims 1 to 5, characterised in that the reaction of the solid polyisocyanates with the "amine stabilisers" is carried out directly in relatively high molecular weight polyols and relatively high molecular weight aromatic and/or aliphatic polyamines with molecular weights of 400 to 6000, optionally with the addition of low molecular weight polyols and/or aromatic polyamines as chain-extending agents with molecular weights of 60 to 399, to form the suspension.

7. Process according to Claims 1 to 6, characterised in that the components are reacted in quantities corresponding to the formulation for one-component PU reactive systems.

8. Process according to Claims 1 and 4 to 7, characterised in that the solid polyisocyanates are reacted with di- or higher functional, low and/or relatively high molecular weight compounds containing aliphatically bound-amino groups and having molecular weights of 32 to 6000 as "amine stabilisers" in a liquid medium of relatively high molecular weight polyols and low molecular weight, aromatic polyamines as chain-extending agents, optionally, in addition, 0 to 90 mol % of low molecular weight polyols as chain-extending agents, to form a suspension of polyadduct-coated, stabilised polyisocyanates in the liquid medium.

9. Process according to Claim 1, characterised in that the solid polyisocyanates are reacted with "amine stabilisers" based on low molecular weight aliphatic polyamines, in water as the liquid medium, in the presence of a low and/or relatively high molecular weight polyol component and/or a low or relatively high molecular weight polyamine component, preferably 2 to 25 % by weight of a relatively high molecular weight polyol or polyamine component, the stabilised polyisocyanate is filtered off, optionally dried carefully, and added, in the isolated, finely powdered form, to the relatively high molecular weight polyols and/or polyamines and, optionally, other starting components for the one-component PU reactive mixture.

10. Process according to Claims 1 to 5 and 7, characterised in that those aromatic polyamino compounds of the kind obtained by hydrolysis of corresponding NCO prepolymers based on relatively high molecular weight polyhydroxyl compounds and excess aromatic diisocyanates, by, preferably basic, hydrolysis, are used as the relatively high molecular weight polyamino compound.

11. Process according to Claim 3, 4, 5 and 7, characterised in that the solid polyisocyanates are reacted at low

temperatures, preferably room temperature, in suspension in relatively high molecular weight, aliphatic polyamines as the amine stabilisers and liquid medium.

12. Polyadduct-coated, stabilised, solid, finely divided polyisocyanates with retarded reactivity according to process Claims 1 to 11, suspended in low and/or relatively high molecular weight polyols and low and/or relatively high molecular weight aromatic polyamines and/or relatively high molecular weight aliphatic polyamines, with an NCO residual content of at least 75 % and less than 99.9 % of the NCO groups originally present in the unstabilised polyisocyanates, and with a thickening temperature of this suspension of higher than 55° C.

13. Polyadduct-coated polyisocyanates according to Claim 12, suspended in a medium of relatively high molecular weight polyols and relatively high molecular weight aromatic and/or aliphatic polyamines with molecular weights of 400 to 6000, and low molecular weight polyols and/or aromatic polyamines with molecular weights of 60 to 399.

14. Polyadduct-coated polyisocyanates according to Claim 12, suspended in relatively high molecular weight aromatic and/or aliphatic polyamines with molecular weights of 400 to 6000, optionally with the addition of low molecular weight polyols and/or aromatic polyamines of a molecular weight of 62 to 399 and optionally minor quantities, based on the relatively high molecular weight polyamines, of relatively high molecular weight polyols.

15. Polyadduct-coated, stabilised, solid, finely divided polyisocyanates with retarded reactivity, prepared according to Claim 2, suspended in low and/or relatively high molecular weight polyols and/or low and/or relatively high molecular weight aromatic polyamines and/or relatively high molecular weight aliphatic polyamines, with an NCO residual content of at least 75 % and less than 99.9 % of the NCO groups originally present in the unstabilised polyisocyanates, and with a thickening temperature of the suspension of higher than 55° C.

16. Polyadduct-coated polyisocyanates according to Claims 12 to 15 characterised in that they have a solids content of polyadduct-coated polyisocyanates of 3 to 70 % by weight in the suspension.

17. Polyadduct-coated polyisocyanates according to Claims 12, 13, 15 and 16, characterised in that they are suspended in relatively high molecular weight polyols with molecular weights of 400 to 6000 with the addition of low molecular weight aromatic diamines and optionally low molecular weight polyols with molecular weights of up to 399.

18. Polyadduct-coated, stabilised, solid, finely divided polyisocyanates according to Claims 12 and 13, characterised in that according to processes 1 to 7 and 8 the solid polyisocyanates are prepared with di- or higher functional, low and/or relatively high molecular weight compounds containing aliphatically bound amino groups and having molecular weights of 32 to 6000 as "amine stabilisers" in a liquid medium of relatively high molecular weight polyols and low molecular weight, aromatic polyamines as chain-extending agents and optionally,in addition 0 to 90 mol % of low molecular weight polyols as chain-extending agents,

or in that the polyadduct-coated polyisocyanates are prepared by reacting the solid polyisocyanates with the "amine stabilisers" in a liquid medium of relatively high molecular weight polyols and the low molecular weight, aromatic polyamines and optionally the additional low molecular weight polyols as chain-extending agents are then added.

19. Use of polyadduct-coated polyisocyanates for the production of polyurethanes from

A) polyisocyanates,

B) relatively high molecular weight polyhydroxyl and/or polyamino compounds, optionally

C) low molecular weight chain-extending agents, optionally

D) polyurethane catalysts and optionally

E) customary auxiliaries and additives,

characterised in that the polyadduct-coated, suspended polyisocyanates obtained according to Claims 1 to 11, as polyisocyanate component A), the relatively high molecular weight component B) and optionally the low molecular weight polyol and/or polyamine chain-extending agent component C), optionally with the addition of other, non-stabilised polyisocyanates A), other relatively high molecular weight compounds B) and other chain-extending agents C), with the addition of tert.-amine and/or metal catalysts D) and optionally auxiliaries E) are used for the preparation of flowable or readily fusible polyurethane one-component reactive systems with a thickening temperature of $\geqslant$ 55° C which are cured by heat, shearing forces and/or polar solvents to form solid or cellular polyurethanes.

20. Use according to Claim 19, characterised in that the polyisocyanates stabilised by coating with a polyadduct and obtained by the reaction of solid polyisocyanates in a finely divided form with hydrazine, alkyl hydrazines, N,N'dialkylhydrazines (containing $C_1$-$C_6$-alkyl groups) such as N,N'-dimethylhydrazine and/or with di- or higher functional, low and/or higher functional compounds containing $-CO.NH.NH_2$ end groups and having a molecular weight of 32 to 6000, or mixtures thereof, as "amine stabilisers", in a quantity of 0.1 to 25 equivalent per cent of amino groups, based on the NCO groups, and present in suspension in relatively high molecular weight polyols and/or relatively high molecular weight aromatic and/or aliphatic polyamines with molecular weights of 400 to 6000, optionally with the addition of low molecular weight polyols and/or aromatic polyamines, as component A), B) and optionally C), are used, optionally with the addition of other, non-stabilised polyisocyanates A), relatively high molecular weight compounds B) and chain-extending agents C), optionally with the addition of tert.-amine and/or metal catalysts D) and auxiliaries E) for the production of PU one-component reactive systems with a thickening temperature of $\geqslant$ 55° C which are cured by heat, shearing

forces and/or polar solvents to form solid or cellular polyurethanes.

21. Use according to Claim 19, characterised in that the polyisocyanates stabilised by coating with a polyadduct and obtained by the reaction of free polyisocyanates in a finely divided form with 0.1 to 25 equivalent per cent of amino groups, based on NCO groups, with di- or higher functional, low and/or relatively high molecular weight compounds containing aliphatically bound, primary and/or secondary amino groups and/or -CO.NH.$NH_2$ end groups and/or hydrazine(s), alkyl-substituted hydrazines and/or N,N'-dialkylhydrazines such as N,N'-dimethylhydrazine, with a molecular weight of 32 to 6000, or mixtures thereof, as "amine stabilisers" present in suspension in relatively high molecular weight aromatic and/or aliphatic polyamines with molecular weights of 400 to 6000 and optionally low molecular weight polyols and/or aromatic polyamines of a molecular weight of 62 to 399 and optionally minor quantities, based on relatively high molecular weight polyamines, of relatively high molecular weight polyols, as component A), B) and optionally C), are used, optionally with the addition of other, non-stabilised polyisocyanates A), relatively high molecular weight compounds B) and chain-extending agents C), optionally with the additioin of tert.-amine and/or metal catalysts D) and auxiliaries E) for the production of PU one-component reactive systems with, a thickening temperature of ⩾ 55°C which are cured by heat, shearing forces and/or polar solvents to form solid or cellular polyurethanes.

22. Use according to Claim 19, characterised in that the................ by the reaction of solid polyisocyanates in a finely divided form with di- or higher functional, low and/or relatively high molecular weight compounds containing aliphatically bound, primary and/or secondary amino groups and having a molecular weight of 60 to 3000 (or mixtures thereof), as "amine stabilisers" in a quantity of 0.1 to 25 % by weight of amino groups, based on NCO, in suspension in relatively high molecular weight polyols with molecular weights of 400 to 6000, optionally with the addition of low molecular weight aromatic polyamines and/or low molecular weight polyols with molecular weights of up to 399, as polyisocyanate component A) and component B) and, as chain-extending component C), low molecular weight aromatic polyamines from the stabilisation reaction or, as the added chain-extendine agent C) optionally with the addition of other, non-stabilised polyisocyanates, A), relatively high molecular weight compounds B) and chain-extending agents C), optionally with the addition of tert.-amine and/or metal catalysts D) and auxiliaries E) are used for the production of PU one-component reactive systems with a thickening temperature of ⩾ 55°C which are cured by heat, shearing forces and/or polar solvents to form solid or cellular polyurethanes.

23. Use according to Claims 19 to 22, characterised in that the NCO/($NH_2$+OH) equivalent ratio of A/(B+C) in the polyurethane-forming reaction is 0.5 : 1 to 1.5 : 1.

24. Use according to Claims 19 to 23, characterised in that 0.3 to 10 (OH+$NH_2$) equivalents of low molecular weight polyols and/or polyamines (C) are used per ($NH_2$+OH) equivalent of relatively high molecular weight polyols and/or polyamines (B) for the polyurethane-forming reaction.

25. Use according to Claims 19 to 24, characterised in that, as catalysts D), 0.001 to 5 % by weight, based on A+B, of organic lead and/or tin compounds are used in the one-component systems.

26. Use according to Claims 19 to 25, characterised in that organic lead compounds from the series comprising

a) organic salts of divalent lead with carboxylic acids,

b) dithiocarbamates of divalent lead of the formula

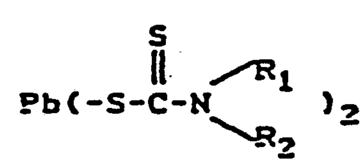

wherein

$R_1$ and $R_2$ can be different and denote a $C_1$-$C_{20}$-alkyl radical,

c) tetraorgano-lead-IV compounds, the organic radical denoting a lower alkyl radical such as, for example methyl or ethyl, and

d) compounds of 1,3-dicarbonyl compounds such as, for example, acetyl acetone, with divalent lead,

are used when polyether polyols (B) containing secondary hydroxyl groups are used in the one-component systems and in that organic tin compounds from the group comprising tin(II) salts of carboxylic acids or tin(IV) compounds such as dibutyl tin oxide, dibutyl tin dichloride, dibutyl tin diacetate, dibutyl tin dilaurate, dibutyl tin maleate or sulphurcontaining tin compounds of the formula

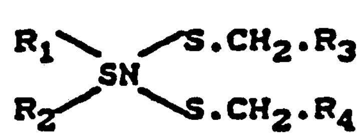

wherein

$R_1$ and $R_2$ denote alkyl radicals with 1 to 10 C atoms,

$R_3$ and $R_4$ denote hydrogen and/or $C_1$-$C_{18}$-alkyl and/or the radical $COOR_1$, are added when polyols B) containing essentially primary hydroxyl groups are used in the one-component systems.

27. Use according to Claims 19, 23 and 24, characterised in that polyadduct-coated polyisocyanates according to Claim 18 are used.

**Claims** for the Contracting states CH, LI

1. Process for the production of solid polyisocyanates which are stabilised by a polymer coating and display retarded reactivity characterised in that solid polyisocyanates with melting points above 30°C are reacted in a finely divided form and with a particle size of 0.5 to 200 μm, with di- or higher functional, low and/or relatively high molecular weight compounds containing aliphatically bound, primary and/or secondary amino groups and/or -CO.NH.$NH_2$ end groups and/or hydrazine(s), alkylsubstituted hydrazines and/or N,N'-dialkylhydrazines such as N,N'-dimethylhydrazine, and having a molecular weight of 32 to 6000, or mixtures thereof, as "amine stabilisers" in a quantity of 0.1 to 25 equivalent per cent of amine per NCO, in a liquid medium of low and/or relatively high molecular weight mono- and/or polyols and/or low and/or relatively high molecular weight aromatic polyamines and/or relatively high molecular weight aliphatic polyamines and/or plasticisers and optionally water, optionally in the presence of non-polar or slightly polar solvents at temperatures below the melting temperature of the polyisocyanates to form a suspension of polyadduct-coated stabilised polyisocyanates in the liquid medium and the stabilised polyisocyanates are optionally isolated from the monoalcohols, plasticisers, water and/or solvents and suspended in the polyols and/or polyamines.

2. Process for the production of solid polyisocyanates which are stabilised by a polymer coating and display retarded reactivity, characterised in that solid polyisocyanates with melting points above 30°C are reacted in a finely divided form and with a particle size of 0.5 to 200 μm, with hydrazine, alkyl hydrazines, N,N'-dialkylhydrazines containing $C_1$-$C_6$-alkyl groups such as N,N'-dimethylhydrazine and/or di- or higher functional, low and/or relatively high molecular weight compounds containing -CO.NH.$NH_2$ end groups and having a molecular weight of 32 to 6000 or mixtures thereof as "amine stabilisers" in a quantity of 0.1 to 25 equivalent per cent of a hydrazine or hydrazide end group per NCO group in a liquid medium of low and/or relatively high molecular weight mono- and/or polyols and/or low and/or relatively high molecular weight aromatic polyamines and/or relatively high molecular weight aliphatic polyamines with molecular weights of 60 to 6000 and/or plasticisers and optionally water, optionally in the presence of non-polar or slightly polar solvents, at temperatures below the melting point of the polyisocyanates to form a suspension of polyadduct-coated, stabilised polyisocyanates in the liquid medium and the stabilised polyisocyanates are optionally isolated from the monoalcohols, plasticisers, water or slightly polar solvents and then suspended in the polyols and/or polyamines.

3. Process for the production of solid polyisocyanates which are stabilised by a polymer coating and display retarded reactivity, characterised in that solid polyisocyanates with melting points above 30°C are reacted in a finely divided form and with a particle size of 0.5 to 200 μm with di- or higher functional, low and/or relatively high molecular weight compounds containing aliphatically bound primary and/or secondary amino groups and/or -CO.NH.$NH_2$ end groups and/or hydrazine(s), alkyl-substituted hydrazines and/or N,N'-dialkylhydrazines such as N,N'-dimethylhydrazine and having a molecular weight of 32 to 6000, or mixtures thereof as "amine stabilisers" in a quantity of 0.1 to 25 equivalent per cent of amine per NCO, in a liquid medium of relatively high molecular weight aromatic and/or aliphatic polyamines with molecular weights of 400 to 6000 and optionally low molecular weight aromatic polyamines and optionally low and/or relatively high molecular weight polyols and/or optionally plasticisers or water, optionally in the presence of non-polar or slightly polar solvents at temperatures below the melting temperature of the polyisocyanates, to form a suspension of polyadduct-coated, stabilised polyisocyanates in the relatively high molecular weight polyamines and the stabilised polyisocyanates are optionally isolated from the plasticisers, water and/or slightly polar solvents and then suspended in the relatively high molecular weight polyamines.

4. Process according to Claims 1 to 3, characterised in that the solid polyisocyanates used are those with melting points above 80°C from the series comprising 1,5-naphthalene-diisocyanate, 1,4-phenylene diisocyanate, 2,3'-diisocyanato-4,4'-dimethyl-N,N'-diphenylurea, dimeric 1-methyl-2,4-diisocyanatobenzene, dimeric 4,4'-diisocyanato-diphenylmethane or 3,3'-dimethyl-4,4'-diisocyanatodiphenyl.

5. Process according to Claims 1 to 4, caracterised in that 0.1 to 8 equivalent per cent of the "amine stabilisers" are used per NCO group.

6. Process according to Claims 1 to 5, characterised in that the reaction of the solid polyisocyanates with the "amine stabilisers" is carried out directly in relatively high molecular weight polyols and/or relatively high molecular weight aromatic and/or aliphatic polyamines with molecular weights of 400 to 6000, optionally with the addition of low molecular weight polyols and/or aromatic polyamines as chain-extending agents with molecular weights of 60 to 399, to form the suspension.

7. Process according to Claims 1 to 6, characterised in that the components are reacted in quantities corresponding to the formulation for one-component PU reactive systems.

8. Process according to Claims 1 and 4 to 7, characterised in that the solid polyisocyanates are reacted with

di- or higher functional, low and/or relatively high molecular weight compounds containing aliphatically bound amino groups and having molecular weights of 32 to 6000 as "amine stabilisers" in a liquid medium of relatively high molecular weight polyols and low molecular weight, aromatic polyamines as chain-extending agents, optionally, in addition, 0 to 90 mol % of low molecular weight polyols as chain-extending agents, to form a suspension of polyadduct-coated, stabilised polyisocyanates in the liquid medium.

9. Process according to Claim 1, characterised in that the solid polyisocyanates are reacted with "amine stabilisers" based on low molecular weight aliphatic polyamines, in water as the liquid medium, in the presence of a low and/or relatively high molecular weight polyol component and/or a low or relatively high molecular weight polyamine component, preferably 2 to 25 % by weight of a relatively high molecular weight polyol or polyamine component, the stabilised polyisocyanate is filtered off, optionally dried carefully, and added, in the isolated, finely powdered form, to the relatively high molecular weight polyols and/or polyamines and, optionally, other starting components for the one-component PU reactive mixture.

10. Process according to Claims 3 to 5 and 7, characterised in that those aromatic polyamino compounds of the kind obtained by hydrolysis of corresponding NCO prepolymers based on relatively high molecular weight polyhydroxyl compounds and excess aromatic diisocyanates, by, preferably basic, hydrolysis, are used as the relatively high molecular weight polyamino compound.

11. Process according to Claims 3 to 5, and 7, characterised in that the solid polyisocyanates are reacted at low temperatures, preferably room temperature, in suspension in relatively high molecular weight, aliphatic polyamines as the amine stabilisers and liquid medium.

12. Polyadduct-coated, stabilised, solid, finely divided polyisocyanates with retarded reactivity according to process Claims 1 to 11, suspended in low and/or relatively high molecular weight polyols and/or low and/or relatively high molecular weight aromatic polyamines and/or relatively high molecular weight aliphatic polyamines, with an NCO residual content of at least 75 % and less than 99.9 % of the NCO groups originally present in the unstabilised polyisocyanates, and with a thickening temperature of this suspension of higher than 55° C.

13. Polyadduct-coated polyisocyanates according to Claim 12, suspended in a medium of relatively high molecular weight polyols and/or relatively high molecular weight aromatic and/or aliphatic polyamines with molecular weights of 400 to 6000, and low molecular weight polyols and/or aromatic polyamines with molecular weights of 60 to 399.

14. Polyadduct-coated polyisocyanates according to Claim 12, suspended in relatively high molecular weight aromatic and/or aliphatic polyamines with molecular weights of 400 to 6000, optionally with the addition of low molecular weight polyols and/or aromatic polyamines of a molecular weight of 62 to 399 and optionally minor quantities, based on the relatively high molecular weight polyamines, of relatively high molecular weight polyols.

15. Polyadduct-coated, stabilised, solid, finely divided polyisocyanates according to Claims 12 and 13, characterised in that, according to processes 1 to 8, the solid polyisocayanates are prepared with di- or higher functional low and/or relatively high molecular weight compounds containing aliphatically bound amino groups and having molecular weights of 32 to 6000 as the "amine stabilisers", in a liquid medium of relatively high molecular weight polyols and low molecular weight, aromatic polyamines as chain-extending agents, and optionally, in addition, 0 to 90 mol % of low molecular weight polyols as chain-extending agents, or in that the polyadduct-coated polyisocyanates are prepared by reacting the solid polyisocyanates with the "amine stabilisers" in a liquid medium of relatively high molecular weight polyols and the low molecular weight, aromatic polyamines and, optionally, the additional low molecular weight polyols as chain-extending agents, are then added.

16. Polyadduct-coated, stabilised, solid, finely divided polyisocyanates with retarded reactivity, prepared according to Claim 2, suspended in low and/or relatively high molecular weight polyols and/or low and/or relatively high molecular weight aromatic polyamines and/or relatively high molecular weight aliphatic polyamines, with an NCO residual content of at least 75 % and less than 99.9 % of the NCO groups originally present in the unstabilised polyisocyanates, and with a thickening temperature of the suspension of higher than 55° C.

17. Polyadduct-coated polyisocyanates according to Claims 12 to 14 and 16, characterised in that they have a solids content of polyadduct-coated polyisocyanates of 3 to 70 % by weight in the suspension.

18. Polyadduct-coated polyisocyanates according to Claims 12, 13, 16 and 17, characterised in that they are suspended in relatively high molecular weight polyols with molecular weights of 400 to 6000 with the addition of low molecular weight aromatic diamines and optionally low molecular weight polyols with molecular weights of up to 399.

19. Use of polyadduct-coated polyisocyanates for the production of polyurethanes from

A) polyisocyanates,

B) relatively high molecular weight polyhydroxyl and/or polyamino compounds, optionally

C) low molecular weight chain-extending agents, optionally

D) polyurethane catalysts and, optionally,

E) customary auxiliaries and additives, characterised in that the polyadduct-coated, suspended polyisocyanates obtained according to Claims 1 to 7, as polyisocyanate component A), the relatively high molecular weight component B) and optionally the low molecular weight polyol and/or polyamine chain-extending agent component C), optionally with the addition of other, non-stabilised polyisocyanates A), other

relatively high molecular weight compounds B) and other chain-extending agents C), optionally with the addition of tert.-amine and/or metal catalysts D) and optionally auxiliaries E) are used for the preparation of flowable or readily fusible polyurethane one-component reactive systems with a thickening temperature of ⩾ 55°C which are cured by heat, shearing forces and/or polar solvents to form solid or cellular polyurethanes.

20. Use according to Claim 19, characterised in that the polyisocyanates stabilised by coating with a polyadduct and obtained by the reaction of solid polyisocyanates in a finely divided form with hydrazine, alkyl hydrazines, N,N'dialkylhydrazines (containing $C_1$-$C_6$-alkyl groups) such as N,N'-dimethylhydrazine and/or with di- or higher functional, low and/or higher functional compounds containing -CO.NH.$NH_2$ end groups and having a molecular weight of 32 to 6000, or mixtures thereof, as "amine stabilisers", in a quantity of 0.1 to 25 equivalent per cent of amino groups, based on the NCO groups, and present in suspension in relatively high molecular weight polyols and/or relatively high molecular weight aromatic and/or aliphatic polyamines with molecular weights of 400 to 6000, optionally with the addition of low molecular weight polyols and/or aromatic polyamines, as component A), B) and optionally C), are used, optionally with the addition of other, non-stabilised polyisocyanates A), relatively high molecular weight compounds B) and chain-extending agents C), optionally with the addition of tert.-amine and/or metal catalysts D) and auxiliaries E), for the production of PU one-component reactive systems with a thickening temperature of 55°C which are cured by heat, shearing forces and/or polar solvents to form solid or cellular polyurethanes.

21. Use according to Claim 19, characterised in that the polyisocyanates stabilised by coating with a polyadduct and obtained by the reaction of free polyisocyanates in a finely divided form with 0.1 to 25 equivalent per cent of amino groups, based on NCO groups, with di- or higher functional, low and/or relatively high molecular weight compounds containing aliphatically bound, primary and/or secondary amino groups and/or -CO.NH.$NH_2$ end groups and/or hydrazine(s) alkyl-substituted hydrazines and/or N,N'-dialkylhydrazines such as N,N'-dimethylhydrazine, with a molecular weight of 32 to 6000, or mixtures thereof, as "amine stabilisers" present in suspension in relatively high molecular weight aromatic and/or aliphatic polyamines with molecular weights of 400 to 6000 and optionally low molecular weight polyols and/or aromatic polyamines of a molecular weight of 62 to 399 and optionally minor quantities, based on relatively high molecular weight polyamines, of relatively high molecular weight polyols, as component A), B) and optionally C), are used, optionally with the addition of other, non-stabilised polyisocyanates A), relatively high molecular weight compounds B) and chain-extending agents C), optionally with the addition of tert.-amine and/or metal catalysts D) and auxiliaries E) for the production of PU one-component reactive systems with a thickening temperature of ⩾ 55°C which are cured by a heat, shearing forces and/or polar solvents to form solid or cellular polyurethanes.

22. Use according to Claim 19, characterised in that the................ by the reaction of solid polyisocyanates in a finely divided form with di- or higher functional, low an or relatively high molecular weight compounds containing aliphatically bound, primary and/or secondary amino groups and having a molecular weight of 60 to 3000 (or mixtures thereof), as "amine stabilisers" in a quantity of 0.1 to 25 % by weight of amino groups, based on NCO, in suspension in relatively high molecular weight polyols with molecular weights of 400 to 6000, optionally with the addition of low molecular weight aromatic polyamines and/or low molecular weight aromatic polyamines and/or low molecular weight polyols with molecular weights of up to 399, as polyisocyanate component A) and component B) and, as chain-extending component C), low molecular weight aromatic polyamines from the stabilisation reaction or, as the added chain-extending agent C) optionally with the addition of other, non-stabilised polyisocyanates A) relatively high molecular weight compounds B) and chain-extending agents C), optionally with the addition of tert.-amine and/or metal catalysts D) and auxiliaries E) are used or the production of one-component reactive systems with a thickening temperature of ⩾ 55°C which are solid or cellular polyurethanes.

23. Use according to Claims 19 to 22, characterised in that the NCO/($NH_2$+OH) equivalent ratio of A/(B+C) in the polyurethane-forming reaction is 0.5 : 1 to 1.5 : 1.

24. Use according to Claims 19 to 23, characterised in that 0.3 to 10 (OH+$NH_2$) equivalents of low molecular weight polyols and/or polyamines (C) are used per ($NH_2$+OH) equivalent of relatively high molecular weight polyols and/or polyamines (B) for the polyurethane-forming reaction.

25. Use according to Claims 19 to 24, characterised in that, as catalysts D), 0.001 to 5 % by weight, based on A+B, of organic lead and/or tin compounds are used in the one-component systems.

26. Use according to Claims 19 to 25, characterised in that organic lead compounds from the series comprising

a) organic salts of divalent lead with carboxylic acids,

b) dithiocarbamates od divalent lead of the formula

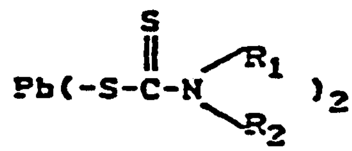

wherein
$R_1$ and $R_2$ can be different and denote a $C_1$-$C_{20}$-alkyl radical,
c) tetraorgano-lead-IV compounds, the organic radical denoting a lower alkyl radical such as, for example methyl or ethyl, and
d) compounds of 1,3-dicarbonyl compounds such as, for example, acetyl acetone, with divalent leads,
are used when polyether polyols (B) containing secondary hydroxyl groups are used in the one-component systems and in that organic tin compounds form the group comprising tin(II) salts of carboxylic acids or tin(IV) compounds such as dibutyl tin oxide, dibutyl tin dichloride, dibutyl tin diacetate, dibutyl tin dilaurate, dibutyl tin maleate or sulphur-containing tin compounds of the formula

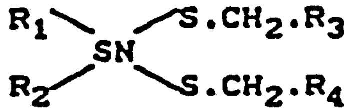

wherein
$R_1$ and $R_2$ denote alkyl radicals with 1 to 10 C atoms,
$R_3$ and $R_4$ denote hydrogen and/or $C_1$-$C_{18}$-alkyl and/or the radical $COOR_1$,
are added when polyols B) containing essentially primary hydroxyl groups are used in the one-component systems.

27. Use according to Claims 19, 23 and 24, characterised in that polyadduct-coated polyisocyanates according to Claim 15 are used.

**Revendications** pour les Etats contractants AT, BE, DE, FR, GB, IT, NL, SE

1. Procédé de production de polyisocyanates solides, stabilisés par formation d'une enveloppe polymérique, à réactivité retardée, caractérisé en ce qu'on fait réagir des polyisocyanates solides ayant des points de fusion au-dessus de 30°C, sous la forme finement divisée et avec une grosseur de particules de 0,5 à 200 µm, avec des composés difonctionnels ou polyfonctionnels, de bas poids moléculaire et/ou de poids moleculaire élevé, portant des groupes amino primaires et/ou secondaires à liaison aliphatique et/ou des groupes $-CO.NH.NH_2$ terminaux et/ou une ou plusieurs hydrazines, des hydrazines à substituants alkyle et/ou des N,N'-dialkylhydrazines telles que la N,N'-diméthylhydrazine, et d'un poids moléculaire de 32 à 6000, ou leurs mélanges, comme "agents stabilisants aminés" en une quantité de 0,1 à 25 % d'équivalents d'amine par groupe NCO, dans un milieu liquide formé de mono-ols et/ou de polyols de bas poids moléculaire et/ou de poids moléculaire élevé et/ou de polyamines aromatiques de bas poids moléculaire et/ou de poids moléculaire élevé et/ou de polyamines aliphatiques de poids moléculaire élevé et/ou de plastifiants et, le cas échéant, d'eau, éventuellement en présence de solvants non polaires ou peu polaires à des températures inférieures à la température de fusion des polyisocyanates, pour former une suspension de polyisocyanates stabilisés à enveloppe de produit de polyaddition dans le milieu liquide, et on isole éventuellement les polyisocyanates stabilisés des mono-alcools, des plastifiants, de l'eau et/ou des solvants et on les met en suspension dans les polyols et/ou dans les polyamines.

2. Procédé de production de polyisocyanates solides, stabilisés par formation d'une enveloppe polymérique, à réactivité retardée, caractérisé en ce qu'on fait réagir des polyisocyanates solides ayant des points de fusion au-dessus de 30°C sous la forme de fines particules d'un diamètre de 0,5 à 200 µm, avec l'hydrazine (ou son hydrate), des alkylhydrazins, des N,N'-dialkylhydrazines portant des groupes alkyle en $C_1$ à $C_6$ telles que la N,N'-diméthylhydrazine et/ou des composés difonctionnels ou polyfonctionnels de bas poids moléculaire et/ou de poids moléculaire élevé portant des groupes terminaux $-CO.NH.NH_2$ et ayant un poids moléculaire de 32 à 6000 ou leurs melanges comme "agents stabilisants aminés" en une quantité de 0,1 à 25 % d' équivalents d'un groupe terminal hydrazino ou hydrazido par groupe NCO, dans un milieu liquide formé de mono-ols et/ou de polyols de bas poids moléculaire et/ou de poids moléculaire élevé et/ou de polyamines aromatiques de bas poids moléculaire et/ou de poids moléculaire élevé et/ou de polyamines aliphatiques de poids moléculaire élevé, avec des poids moléculaires de 60 à 6000, et/ ou de plastifiants et, le cas échéant, d'eau, éventuellement en présence de solvants non polaires ou peu polaires, à des températures en dessous du point de fusion des polyisocyanates pour former une suspension de polyisocyanates stabilisés, à enveloppe de produit de polyaddition, en milieu liquide et, le cas échéant, on isole les polyisocyanates stabilisés des mono-alcools, des

plastifiants, de l'eau ou des solvants peu polaires et on les met ensuite en suspension dans les polyols et/ou dans des polyamines.

3. Procédé de production de polyisocyanates solides stabilisés par formation d'une enveloppe de polymère, à réactivité retardée, caractérisé en ce qu'on fait réagir des polyisocyanates solides ayant des points de fusion au-dessus de 30°C sous la forme de fines particules d'un diamètre de 0,5 à 200 µm avec des composés difonctionnels ou polyfonctionnels, de bas poids moléculaire et/ou de poids moléculaire élevé, portant des groupes amino primaires et/ou secondaires à liaison aliphatique et/ou des groupes $-CO.NH.NH_2$ terminaux et/ou hydrazine (ou son hydrate), des hydrazines à substituants alkyle et/ou des N,N'-dialkylhydrazines telles que la N,N'-diméthylhydrazine et avec un poids moléculaire de 32 à 6000, ou leurs mélanges, comme "agents stabilisants aminés" en une quantite de 0,1 à 25 % d'equivalents d'amine par NCO, dans un milieu liquide formé de polyamines aromatiques et/ou aliphatiques de poids moléculaire élevé ayant des poids moléculaires de 400 à 6000, et, le cas échéant, de polyamines aromatiques de bas poids moléculaire ainsi que, le cas échéant, de polyols de bas poids moléculaire et/ou de poids moléculaire élevé et/ou éventuellement de plastifiants ou d'eau, éventuellement en présence de solvants non polaires ou peu polaires à des températures inférieures à la température de fusion des polyisocyanates, pour former une suspension de polyisocyanates stabilisés, enveloppés de produit de polyaddition, dans les polyamines de poids moléculaire élevé et, le cas échéant, on isole les polyisocyanates stasbilises des plastifiants, de l'eau et/ou de solvants peu polaires et on les met ensuite en suspension dans les polyamines de poids moléculaire élevé.

4. Procédé suivant les revendications 1 à 3, caractérisé en ce qu' on utilise comme polyisocyanates solides des polyisocyanates ayant des points de fusion au-dessus de 80°C, de la série du 1,5-diisocyanatonaphtalène, du 1,4-phénylènediisocyanate, de la 2,3'-diisocyanato-4,4'-dimethyl-N,N'-diphénylurée, du 1-méthyl-2,4-diisocyanatobenzène dimère, du 4,4'-diisocyantodiphénylméthane dimère ou du 3,3'-diméthyl-4,4'-diisocyanatodiphényle.

5. Procédé suivant les revendications 1 à 4, caractérisé en ce qu'on utilise 0,1 à 8 % d'équivalents des "agents stabilisants aminés" par groupe NCO.

6. Procédé suivant les revendications 1 à 5, caractérisé en ce qu'on conduit la réaction des polyisocyanates solides avec les "agents stabilisants amines" directement dans des polyols de poids moléculaire élevé et/ou des polyamines aromatiques et/ou aliphatiques de poids moléculaire élevé, ayant des poids moléculaires de 400 à 6000, le cas échéant avec addition de polyols de bas poids moléculaire et/ou de polyamines aromatiques comme agents d'allongement de chaîne ayant des poids moléculaires de 80 à 399 avec formation de la suspension.

7. Procédé suivant les revendications 1 à 6 caractérisé en ce qu'on fait réagir les composants en des quantités qui correspondent à la formulation des systèmes réactifs de formation de polyuréthannes à un seul composant.

8. Procédé suivant les revendications 1 et 4 à 7 caractérisé en ce qu' on fait réagir les polyisocyanates solides avec des composés difonctionnels ou polyfonctionnels de bas poids moléculaire et/ou de poids moléculaire élevé portant des groupes amino à liaison aliphatique et ayant des poids moléculaires de 32 à 6000 comme "agents stabilisants aminés" dans un milieu liquide formé de polyols de poids moléculaire élevé et de polyamines aromatiques de bas poids moléculaire comme agents d'allongement de chaîne, additionnés, le cas échéant, de 0 à 90 moles % de polyols de bas poids moléculaire comme agents d'allongement de chaîne, pour former une suspension de polyisocyanates stabilisés à enveloppe formée de produit de polyaddition, dans le milieu liquide.

9. Procédé suivant la revendication 1, caractérisé en ce qu'on fait réagir les polyisocyanates solides avec des "agents stabilisants aminés" à base de polyamines aliphatiques de bas poids moléculaire dans de l'eau comme milieu liquide en présence d'un composant polyol de bas poids moléculaire et/ou de poids moleculaire élevé et/ou d'un composant polyamine de bas poids moléculaire ou de poids moléculaire élevé, de préference 2 à 25 % en poids d'un composant polyol ou polyamine de poids moléculaire élevé, on sépare par filtration le polyisocyanate stabilisé, on le sèche avec précaution, le cas échéant, et on l'ajoute, sous la forme de poudre fine isolée, aux polyols et/ou aux polyamines de poids moléculaire élevé et, le cas échéant, à d'autres composants de départ du mélange réactif de formation de polyuréthanne à un seul composant.

10. Procédé suivant les revendications 1 à 5 et 7, caractérisé en ce qu'on utilise comme composés polyaminés de poids moléculaire élevé, des composés polyaminés aromatiques du type de ceux que l'on obtient par hydrolyse de NCO-prépolymères correspondants à base de composés polyhydroxyliques de poids moléculaire élevé et de diisocyanates aromatiques en excès, par hydrolyse de préférence basique.

11. Procédé suivant les revendications 3, 4, 5 et 7, caractérisé en ce qu'on fait réagir les polyisocyanates solides en suspension à basses températures, de préférence à la température ambiante, dans des polyamines aliphatiques de poids moléculaire élevé comme agents stabilisants aminés et milieu liquide.

12. Polyisocyanates en fines particules solides, stabilises, à enveloppe formée d'un produit de polyaddition, à réactivité retardée suivant les revendications de procédé 1 à 11, en suspension dans des polyols de bas poids moléculaire et/ou de poids moléculaire élevé et/ou dans des polyamines aromatiques de bas poids moléculaire et/ou de poids moléculaire élevé et/ou dans des polyamines aliphatiques de poids moléculaire élevé, avec une teneur résiduelle en groupes NCO d'au moins 75 % et de moins de 99,9 % des groupes NCO présents initialement dans les polyisocyanates non stabilisés, et à une température d'épaississement de cette suspension au-dessus de 55°C.

13. Polyisocyanates à enveloppppe formée de produit de polyaddition suivant la revendication 12, en

suspension dans un milieu constitue de polyols de poids moléculaire élevé et/ou de polyamines aromatiques et/ou aliphatiques de poids moléculaire élevé, avec des poids moleculaires de 400 à 6000, et de polyols de bas poids moléculaire et/ou de polyamines aromatiques avec des poids moléculaires de 60 à 399.

14. Polyisocyanates à enveloppe formée de produit de polyaddition suivant la revendication 12, en suspension dans des polyamines aromatiques et/ou aliphatiques de poids moléculaire élevé, ayant des poids moléculaires de 400 à 6000, le cas échéant avec addition de polyols de bas poids moléculaire et/ou de polyamines aromatiques d'un poids moléculaire de 62 à 399, avec, le cas échéant, de faibles quantités, par rapport aux polyamines de poids moléculaire élevé, de polyols de poids moléculaire élevé.

15. Polyisocyanates solides en fines particules, stabilisés, à enveloppe formée de produit de polyaddition, à réactivité retardée, préparés suivant la revendication 2, en suspension dans des polyols de bas poids moléculaire et/ou de poids moléculaire élevé et/ou dans des polyamines aromatiques de bas poids moléculaire et/ou de poids moléculaire élevé et/ou dans des polyamines aliphatiques de poids moléculaire élevé avec une teneur résiduelle en groupes NCO d'au moins 75 % et de moins de 99,9 % des groupes NCO initialement présents des polyisocyanates non stabilisés, et à une température d'épaississement de la suspension au-dessus de 55° C.

16. Polyisocyanates à enveloppe formée d'un produit de polyaddition suivant les revendications 12 à 15, caractérisés en ce qu'ils présentent une teneur en matières solides, exprimée en polyisocyanates à enveloppe formée de produit de polyaddition, de 3 à 70 % en poids dans la suspension.

17. Polyisocyanates à enveloppe formée de produit de polyaddition suivant les revendications 12, 13, 15 et 16, caractérisés en ce qu'ils sont en suspension dans des polyols de poids moléculaire élevé, ayant des poids moléculaires de 400 à 6000 avec addition de diamines aromatiques de bas poids moléculaire et, le cas échéant, de polyols de bas poids moléculaire, dont les poids moléculaires vont jusqu' à 399.

18. Polyisocyanates solides en fines particules, stabilisés, à enveloppe formée de produit de polyaddition suivant les revendications 12 et 13, caractérisés en ce que les polyisocyanates solides sont produits d'après les procédés 1 à 7 et 8 avec des composés difonctionnels ou polyfonctionnels de bas poids moléculaire et/ou de poids moléculaire élevé portant des groupes amino à liaison aliphatique et ayant des poids moléculaires de 32 à 6000 comme "agents stabilisants aminés" dans un milieu liquide constitué de polyols de poids moléculaire élevé et de polyamines aromatiques de bas poids moléculaire comme agents d'allongement de chaîne avec addition, le cas échéant, de 0 à 90 moles % de polyols de bas poids moléculaire comme agents d'allongement de chaîne, ou bien on prépare les polyisocyanates à enveloppe formée de produit de polyaddition par réaction des polyisocyanates solides avec les "agents stabilisants aminés" dans un milieu liquide constitué de polyols de poids moléculaire élevé et on ajoute ensuite les polyamines aromatiques de bas poids moléculaire et, le cas échéant, les polyols additionnels de bas poids moléculaire comme agents d'allongement de chaîne.

19. Utilisation de polyisocyanates à enveloppe formée de produit de polyaddition, destinés à l'obtention de polyuréthannes à partir

A) de polyisocyanates

B) de composés polyhydroxyliques et/ou polyaminés de poids moléculaire élevé, éventuellement

C) d'agents d'allongement de chaîne de bas poids moléculaire, le cas échéant,

D) de catalyseurs pour polyuréthannes et, le cas échéant

E) d'adjuvants et d'additifs classiques,

caractérisée en ce qu'on utilise les polyisocyanates en suspension, à enveloppe formée de produit de polyaddition, obtenus conformément aux revendications 1 à 11, comme composant polyisocyanate A), composant de poids moléculaire élevé B) et, le cas échéant, composant d'allongement de chaîne de type polyol et/ou polyamine de bag poids moléculmire C), le cas échéant avec addition d'autres polyisocyanates A) non stabilisés, d'autres composés B) de poids moléculaire élevé et d'autres agents d'allongement de chaîne C), éventuellement avec addition de catalyseurs du type d'amines tertiaires et/ou de catalyseurs métalliques D) et, éventuellement, d'adjuvants B) pour la préparation de systèmes réactif s pour polyuréthanne à un composant, pouvant s'écouler ou fondant aisément, avec une température d'épaississement supérieure ou égale à 55° C, et on les fait durcir par la chaleur, par des forces de cisaillement et/ou par des solvants polaires en polyuréthannes compacts ou cellulaires.

20. Utilisation suivant la revendication 19, caractérisée en ce qu'on utilise les polyisocyanates stabilisés par formation d'une enveloppe de produit de polyaddition comme composant A), B) et, le cas échéant, C) obtenus par réaction de polyisocyanates solides sous la forme de fines particules avec l'hydrazine, des alkylhydrazines, des N,N'-dialkylhydrazines (portant des groupes alkyle en $C_1$ à $C_6$) comme N,N'-diméthylhydrazine et/ou avec des composés difonctionnels ou polyfonctionnels, de bas poids moléculaire et/ou de poids moléculaire élevé, portant des groupes $-CO.NH.NH_2$ terminaux et ayant un poids moléculaire de 32 à 6000, ou leurs mélanges, comme "agents stabilisants aminés", en une quantité de 0,1 à 25 % d'équivalents de groupes amino, par rapport aux groupes NCO, se présentant en suspension dans des polyols de poids moléculaire élevé et/ou des polyamines aromatiques et/ou aliphatiques de poids moléculaire élevé, avec des poids moléculaires de 400 à 6000, avec addition éventuelle de polyols et/ou de polyamines aromatiques de bas poids moléculaire, en utilisant, le cas échéant, d'autres polyisocyanates A) non stabilisés, composés B) de poids moléculaire élevé et agents C) d'allongement de chaîne, le cas échéant avec addition de catalyseurs D) du type d'amines tertiaires et/ou métalliques et de substances auxiliaires E), pour la production de systèmes réactifs à un constituant pour polyuréthanne à une température d'épaississement supérieure ou égale à 55°C et on les fait durcir par la chaleur, par des forces de cisaillement et/ou par des solvants polaires en polyuréthannes compacts ou

cellulaires.

21. Utilisation suivant la revendication 19 caractérisée en ce qu'on utilise des polyisocyanates stabilisés par formation d'une enveloppe de produit d'addition, obtenus par réaction de polyisocyanates libres sous une forme finement divisée à 0,1-25 % d' équivalents de groupes amino, par rapport aux groupes NCO, avec des composés difonctionnels ou polyfonctionnels de bas poids moléculaire et/ou de poids moléculaire élevé portant des groupes amino primaires et/ou secondaires à liaison aliphatique et/ou des groupes terminaux $-CO.NH.NH_2$ et/ou une ou plusieurs hydrazines, des hydrazines à substituants alkyle et/ou des N,N'-dialkylhydrazines telles que la N,N'-diméthylhydrazine, d'un poids moléculaire de 32 à 6000, ou leurs mélanges, en tant que "agents stabilisants aminés", se présentant en suspension dans des polyamines aromatiques et/ou aliphatiques de poids moléculaire élevé ayant des poids moléculaires de 400 à 6000 et, le cas échéant, des polyols et/ou des polyamines aromatiques de bas poids moléculaire, d'un poids moléculaire de 62 à 399, et, le cas échéant, de faibles quantités, par rapport aux polyamines de poids moléculaire élevé, de polyols de poids moléculaire élevé, comme composant A), B) et, le cas échéant, C), éventuellement avec addition d'autres polyisocyanates A) non stabilisés, composés B) de poids moléculaire élevé et agents C) d'allongement de chaîne, avec, le cas échéant, addition de catalyseurs du type d'amines tertiaires et/ou de catalyseurs métalliques D) et de substances auxiliaires B), pour la production de systèmes réactifs à un constituant pour polyuréthannes, avec une température d' épaississement supérieure ou égale à 55°C, et on les fait durcir par la chaleur, par des forces de cisaillement et/ou par des solvants polaires en polyuréthannes compacts ou cellulaires.

22. Utilisation suivant la revendication 19, caractérisée en ce qu' on utilise comme composant polyisocyanate A) et composant B) le produit de réaction de polyisocyanates solides sous la forme de fines particules avec des composés difonctionnels ou polyfonctionnels de bas poids moléculaire et/ou de poids moléculaire élevé portant des groupes amino primaires et/ou secondaires à liaison aliphatique et ayant un poids moléculaire de 60 à 3000 (ou leurs mélanges), comme "agents stabilisants aminés" en une quantité de 0,1 à 25 % en poids de groupes amino, par rapport à NCO, en suspension dans des polyols de poids moléculaire élevé ayant des poids moléculaires de 400 à 6000, le cas échéant avec addition de polyamines aromatiques de bas poids moléculaire et/ou de polyols de bas poids moléculaire avec des poids moléculaires allant jusqu'à 399, et comme composant d'allongement de chaîne C), des polyamines aromatiques de bas poids moléculaire dérivées de la réaction de stabilisation ou en tant qu'agent d'allongement de chaîne C) ajouté, éventuellement avec addition d'autres polyisocyanates A) non stabilisés, composés B) de poids moléculaire élevé et agents C) d'allongement de chaîne, avec addition éventuelle de catalyseurs du type d'amines tertiaires et/ou de catalyseurs métalliques D) et de substances auxiliaires B), pour la production de systèmes réactifs à un constituant pour polyuréthanne, avec une température d'épaississement supérieure ou égale à 55°C et on les fait durcir par la chaleur, par des forces de cisaillement et/ou par des solvants polaires en polyuréthannes compacts ou cellulaires.

23. Utilisation suivant les revendications 19 à 22, caractérisée en ce que le rapport des équivalents $NCO/(NH_2+OH)$ de $A/(B+C)$ s'élève à 0,5 : 1 à 1,5 : 1 dans la formation du polyuréthanne.

24. Utilisation suivant les revendications 19 à 23, caractérisée en ce qu'on utilise par équivalent de $(NH_2+OH)$ en polyols et/ou polyamines (B) de poids moléculaire élevé, 0,3 à 10 équivalents de $(OH+NH_2)$ en polyols et/ou en polyamines (C) de bas poids moléculaire pour la formation de polyuréthanne.

25. Utilisation suivant les revendications 19 à 24, caractérisée en ce qu'on utilise comme catalyseurs D) 0,001 à 5 % en poids, par rapport à A+B, de composés organiques de plomb et/ou d' étain dans les systèmes à un constituant.

26. Utilisation suivant les revendications 19 à 25, caractérisée en ce qu'on utilise des composés organiques de plomb de la série

a) des sels organiques du plomb divalent avec des acides dicarboxyliques,

b) de dithiocarbamates du plomb divalent de formule

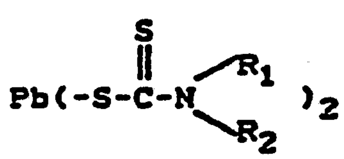

dans laquelle

$R_1$ et $R_2$ peuvent être différents et représentent un reste alkyle en $C_1$ à $C_{20}$,

c) des composés tétra-organiques du plomb-IV, le reste organique désignant alors un reste alkyle inférieur tel que, par exemple, méthyle ou éthyle et

d) des composés dérivés de composés 1,3-dicarbonyliques tels que, par exemple, l'acetylacétone, avec du plomb divalent, lorsqu'on utilise dans les systèmes à un constituant des polyéther-polyols (B) porteurs de groupes hydroxyle secondaires, et en ce qu'on ajoute des composés organiques d'étain du groupe des sels d'étain (II) d'acides carboxyliques ou des composés d' étain (IV) tels que l'oxyde de dibutylétain, le dichlorure de dibutylétain, le diacétate de dibutylétain, le dilaurate de dibutylétain, le maléate de dibutylétain ou des composés d' étain contenant du soufre de formule

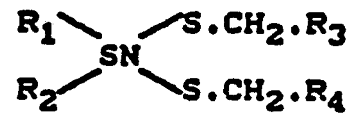

dans laquelle
$R_1$ et $R_2$ sont des restes alkyle ayant 1 à 10 atomes de carbone,
$R_3$ et $R_4$ représentent l'hydrogène et/ou des restes alkyle en $C_1$ à $C_{18}$ et/ou le reste $COOR_1$,
lorsqu'on utilise dans les systèmes à un constituant des polyols B) porteurs de groupes hydroxyle principalement primaires.

27. Utilisation suivant les revendications 19, 23 et 24, caractérisée en ce qu' on utilise des polyisocyanates à enveloppe formée d'un produit de polyaddition suivant la revendication 18.

**Revendications** pour les Etats contractants CH, LI

1. Procédé de production de polyisocyanates solides, stabilisés par formation d'une enveloppe polymérique, à réactivité retardée, caractérisé en ce qu'on fait réagir des polyisocyanates solides ayant des points de fusion au-dessus de 30°C, sous la forme finement divisée et avec une grosseur de particules de 0,5 à 200 µm, avec des composés difonctionnels ou polyfonctionnels, de bas poids moléculaire et/ou de poids moléculaire élevé, portant des groupes amino primaires et/ou secondaires à liaison aliphatique et/ou des groupes $-CO.NH.NH_2$ terminaux et/ou une ou plusieurs hydrazines, des hydrazines à substituants alkyle et/ou des N,N'-dialkylhydrazines telles que la N,N'-diméthylhydrazine, et d'un poids moléculaire de 32 à 6000, ou leurs mélanges, comme "agents stabilisants aminés" en une quantité de 0,1 à 25 % d'équivalents d'amine par groupe NCO, dans un milieu liquide formé de mono-ols et/ou de polyols de bas poids moléculaire et/ou de poids moléculaire élevé et/ou de polyamines aromatiques de bas poids moléculaire et/ou de poids moléculaire élevé et/ou de polyamines aliphatiques de poids moléculaire élevé et/ou de plastifiants et, le cas échéant, d'eau, éventuellement en présence de solvants non polaires ou peu polaires à des températures inférieures à la température de fusion des polyisocyanates, pour former une suspension de polyisocyanates stabilisés à enveloppe de produit de polyaddition dans le milieu liquide, et on isole éventuellement les polyisocyanates stabilisés des mono-alcools, des plastifiants, de l'eau et/ou des solvants et on les met en suspension dans les polyols et/ou dans les polyamines.

2. Procédé de production de polyisocyanates solides, stabilisés par formation d'une enveloppe polymérique, à réactivité retardée, caractérisé en ce qu'on fait réagir des polyisocyanates solides ayant des points de fusion au-dessus de 30°C sous la forme de fines particules d'un diamètre de 0,5 à 200 µm, avec l'hydrazine, des alkylhydrazines, des N,N'-dialkylhydrazines portant des groupes alkyle en $C_1$ à $C_6$ telles que la N,N'-diméthylhydrazine et/ou des composés difonctionnels ou polyfonctionnels de bas poids moléculaire et/ou de poids moléculaire élevé portant des groupes terminaux $-CO.NH.NH_2$ et ayant un poids moléculaire de 32 à 6000 ou leurs mélanges comme "agents stabilisants aminés" en une quantité de 0,1 à 25 % d' équivalents d'un groupe terminal hydrazino ou hydrazido par groupe NCO, dans un milieu liquide formé de mono-ols et/ou de polyols de bas poids moléculaire et/ou de poids moléculaire élevé et/ou de polyamines aromatiques de bas poids moléculaire et/ou de poids moléculaire élevé et/ou de polyamines aliphatiques de poids moléculaire élevé, avec des poids moléculaires de 60 à 6000, et/ ou de plastifiants et, le cas échéant, d'eau, éventuellement en présence de solvants non polaires ou peu polaires, à des températures en dessous du point de fusion des polyisocyanates pour former une suspension de polyisocyanates stabilisés, à enveloppe de produit de polyaddition, en milieu liquide et, le cas échéant, on isole les polyisocyanates stabilisés des mono-alcools, des plastifiants, de l'eau ou des solvants peu polaires et on les met ensuite en suspension dans les polyols et/ou dans des polyamines.

3. Procédé de production de polyisocyanates solides stabilisés par formation d'une enveloppe de polymère, à réactivité retardée, caractérisé en ce qu' on fait réagir des polyisocyanates solides ayant des points de fusion au-dessus de 30°C sous la forme de fines particules d'un diamètre de 0,5 à 200 µm avec des composés difonctionnels ou polyfonctionnels, de bas poids moléculaire et/ou de poids moléculaire élevé, portant des groupes amino primaires et/ou secondaires à liaison aliphatique et/ou des groupes $-CO.NH.NH_2$ terminaux et/ou une ou plusieurs hydrazines, des hydrazines à substituants alkyle et/ou des N,N'-dialkylhydrazines telles que la N,N'-diméthylhydrazine et avec un poids moléculaire de 32 à 6000, ou leurs mélanges, comme "agents stabilisants aminés" en une quantité de 0,1 à 25 % d' équivalents d'amine par NCO, dans un milieu liquide formé de polyamines aromatiques et/ou aliphatiques de poids moléculaire élevé, ayant des poids moléculaires de 400 à 6000, et, le cas échéant, de polyamines aromatiques de bas poids moléculaire ainsi que, le cas échéant, de polyols de bas poids moléculaire et/ou de poids moléculaire élevé et/ou éventuellement de plastifiants ou d'eau, éventuellement en présence de solvants non polaires ou peu polaires à des températures inférieures à la température de fusion des polyisocyanates, pour former une suspension de polyisocyanates stabilisés, enveloppés de produit de polyaddition, dans les polyamines de poids moléculaire élevé et, le cas échéant, on isole les polyisocyanates stabilisés des plastifiants, de l'eau et/ou de solvants peu polaires et on

les met ensuite en suspension dans les polyamines de poids moléculaire élevé.

4. Procédé suivant les revendications 1 à 3, caractérisé en ce qu'on utilise comme polyisocyanates solides des polyisocyanates ayant des points de fusion au-dessus de 80° C, de la série du 1,5-diisocyanatonaphtalène, du 1,4-phénylènediisocyanate, de la 2,3'-diisocyanato-4,4'-diméthyl-N,N'-diphénylurée, du 1-méthyl-2,4-diisocyanatobenzène dimère, du 4,4'-diisocyanatodiphénylméthane dimère ou du 3,3'-diméthyl-4,4'-diisocyanatodiphényle.

5. Procédé suivant les revendications 1 à 4, caractérisé en ce qu'on utilise 0,1 à 8 % d'équivalents des "agents stabilisants aminés" par groupe NCO.

6. Procédé suivant les revendications 1 à 5, caractérisé en ce qu'on conduit la réaction des polyisocyanates solides avec les "agents stabilisants aminés" directement dans des polyols de poids moléculaire élevé et/ou des polyamines aromatiques et/ou aliphatiques de poids moléculaire élevé, ayant des poids moléculaires de 400 à 6000, le cas échéant avec addition de polyols de bas poids moléculaire et/ou de polyamines aromatiques comme agents d'allongement de chaîne ayant des poids moléculaires de 60 à 399, avec formation de la suspension.

7. Procédé suivant les revendications 1 à 6, caractérisé en ce qu'on fait réagir les composants en des quantités qui correspondent à la formulation des systèmes réactifs de formation de polyuréthannes à un seul composant.

8. Procédé suivant les revendications 1 et 4 à 7, caractérisé en ce qu'on fait réagir les polyisocyanates solides avec des composés difonctionnels ou polyfonctionnels de bas poids moléculaire et/ou de poids moléculaire élevé portant des groupes amino à liaison aliphatique et ayant des poids moléculaires de 32 à 6000 comme "agents stabilisants aminés" dans un milieu liquide formé de polyols de poids moléculaire élevé et de polyamines aromatiques de bas poids moléculaire comme agents d'allongement de chaîne, additionnés, le cas échéant, de 0 à 90 moles % de polyols de bas poids moléculaire comme agents d'allongement de chaîne, pour former une suspension de polyisocyanates stabilisés à enveloppe formée de produit de polyaddition, dans le milieu liquide.

9. Procédé suivant la revendication 1, caractérisé en ce qu'on fait réagir les polyisocyanates solides avec des "agents stabilisants aminés" à base de polyamines aliphatiques de bas poids moléculaire dans de l'eau comme milieu liquide en présence d'un composant polyol de bas poids moléculaire et/ou de poids moléculaire élevé et/ou d'un composant polyamine de bas poids moléculaire ou de poids moléculaire élevé, de préférence 2 à 25 % en poids d'un composant polyol ou polyamine de poids moléculaire élevé, on sépare par filtration le polyisocyanate stabilisé, on le sèche avec précaution, le cas échéant, et on l'ajoute sous la forme de poudre fine isolée, aux polyols et/ou aux polyamines de poids moléculaire élevé et, le cas échéant, à d'autres composants de départ du mélange réactif de formation de polyuréthanne à un seul composant.

10. Procédé suivant les revendications 3 à 5 et 7, caractérisé en ce qu'on utilise comme composés polyaminés de poids moléculaire élevé, des composés polyaminés aromatiques du type de ceux que l'on obtient par hydrolyse de NCO-prépolymères correspondants à base de composés polyhydroxyliques de poids moléculaire élevé et de diisocyanates aromatiques en excès, par hydrolyse de préférence basique.

11. Procédé suivant les revendications 3 à 5 et 7, caractérisé en ce qu'on fait réagir les polyisocyanates solides en suspension à basses températures, de préférence à la température ambiante, dans des polyamines aliphatiques de poids moléculaire élevé comme agents stabilisants aminés et milieu liquide.

12. Polyisocyanates en fines particules solides, stabilisés, à enveloppe formée d'un produit de polyaddition, à réactivité retardée suivant les revendications de procédé 1 à 11, en suspension dans des polyols de bas poids moléculaire et/ou de poids moléculaire élevé et/ou dans des polyamines aromatiques de bas poids moléculaire et/ou de poids moléculaire élevé et/ou dans des polyamines aliphatiques de poids moléculaire élevé, avec une teneur résiduelle en groupes NCO d'au moins 75 % et de moins de 99,9 % des groupes NCO présents initialement dans les polyisocyanates non stabilisés, et à une température d' épaississement de cette suspension au-dessus de 55° C.

13. Polyisocyanates à envelopppe formée de produit de polyaddition suivant la revendication 12, en suspension dans un milieu constitué de polyols de poids moléculaire élevé et/ou de polyamines aromatiques et/ou aliphatiques de poids moléculaire élevé, avec des poids moléculaires de 400 à 6000, et de polyols de bas poids moléculaire et/ou de polyamines aromatiques avec des poids moléculaires de 60 à 399.

14. Polyisocyanates à enveloppe formée de produit de polyaddition suivant la revendication 12, en suspension dans des polyamines aromatiques et/ou aliphatiques de poids moléculaire élevé, ayant des poids moléculaires de 400 à 6000, le cas échéant avec addition de polyols de bas poids moléculaire et/ou de polyamines aromatiques d'un poids moléculaire de 62 à 399, avec, le cas échéant, de faibles quantités, par rapport aux polyamines de poids moléculaire élevé, de polyols de poids moléculaire élevé.

15. Polyisocyanates solides en fines particules, stabilisés, à enveloppe formée de produit de polyaddition suivant les revendications 12 et 13, caractérisés en ce que les polyisocyanates solides sont produits d'après les procédés 1 à 8 avec des composés difonctionnels ou polyfonctionnels de bas poids moléculaire et/ou de poids moléculaire élevé portant des groupes amino à liaison aliphatique et ayant des poids moléculaires de 32 à 6000 comme "agents stabilisants aminés" dans un milieu liquide constitué de polyols de poids moléculaire élevé et de polyamines aromatiques de bas poids moléculaire comme agents d'allongement de chaîne avec addition, le cas échéant, de 0 à 90 moles % de polyols de bas poids moléculaire comme agents d'allongement de chaîne, ou-bien on prépare les polyisocyanates à enveloppe formée de produit de polyaddition par réaction des polyisocyanates solides avec les "agents stabilisants aminés" dans un milieu liquide constitué de polyols de

poids moléculaire élevé et on ajoute ensuite les polyamines aromatiques de bas poids moléculaire et, le cas échéant, les polyols additionnels de bas poids moléculaire comme agents d'allongement de chaîne.

16. Polyisocyanates solides en fines particules, stabilisés, à enveloppe formée de produit de polyaddition, à réactivité retardée, préparés suivant la revendication 2, en suspension dans des polyols de bas poids moléculaire et/ou de poids moléculaire élevé et/ou dans des polyamines aromatiques de bas poids moléculaire et/ou de poids moléculaire élevé et/ou dans des polyamines aliphatiques de poids moléculaire élevé avec une teneur résiduelle en groupes NCO d'au moins 75 % et de moins de 99,9 % des groupes NCO initialement présents des polyisocyanates non stabilisés, et à une température d'épaississement de la suspension au-dessus de 55° C.

17. Polyisocyanates à enveloppe formée d'un produit de polyaddition suivant les revendications 12 à 14 et 16, caractérisés en ce qu'ils présentent une teneur en matières solides, exprimée en polyisocyanates à enveloppe formée de produit de polyaddition, de 3 à 70 % en poids dans la suspension.

18. Polyisocyanates à enveloppe formée de produit de polyaddition suivant les revendications 12, 13, 16 et 17, caractérisés en ce qu'ils sont en suspension dans des polyols de poids moléculaire élevé, ayant des poids moléculaires de 400 à 6000 avec addition de diamines aromatiques de bas poids moléculaire et, le cas échéant, de polyols de bas poids moléculaire, dont les poids moléculaires vont jusqu' à 399.

19. Utilisation de polyisocyanates à enveloppe formée de produit de polyaddition, destinés à l'obtention de polyuréthannes à partir

A) de polyisocyanates

B) de composés polyhydroxyliques et/ou polyaminés de poids moléculaire élevé, éventuellement

C) d'agents d'allongement de chaîne de bas poids moléculaire, le cas échéant,

D) de catalyseurs pour polyuréthannes et, le cas échéant

E) d'adjuvants et d'additifs classiques,

caractérisée en ce qu'on utilise les polyisocyanates en suspension, à enveloppe formée de produit de polyaddition, obtenus conformément aux revendications 1 à 7, comme composant polyisocyanate A), composant de poids moléculaire élevé B) et, le cas échéant, composant d'allongement de chaîne de type polyol et/ou polyamine de bas poids moléculaire C), le cas échéant avec addition d'autres polyisocyanates A) non stabilisés, d'autres composés B) de poids moléculaire élevé et d'autres agents d'allongement de chaîne C), éventuellement avec addition de catalyseurs du type d'amines tertiaires et/ou de catalyseurs métalliques D) et, éventuellement, d'adjuvants E) pour la préparation de systèmes réactifs pour polyuréthanne à un composant, pouvant s'écouler ou fondant aisément, avec une température d' épaississement supérieure ou égale à 55° C, et on les fait durcir par la chaleur, par des forces de cisaillement et/ou par des solvants polaires en polyuréthannes compacts ou cellulaires.

20. Utilisation suivant la revendication 19, caractérisée en ce qu'on utilise les polyisocyanates stabilisés par formation d'une enveloppe de produit de polyaddition comme composant A), B) et, le cas échéant, C) obtenus par réaction de polyisocyanates solides sous la forme de fines particules avec l'hydrazine, des alkylhydrazines, des N,N'-dialkylhydrazines (portant des groupes alkyle en $C_1$ à $C_6$) comme N,N'-diméthylhydrazine et/ou avec des composés difonctionnels ou polyfonctionnels, de bas poids moléculaire et/ou de poids moléculaire élevé, portant des groupes $-CO.NH.NH_2$ terminaux et ayant un poids moléculaire de 32 à 6000, ou leurs mélanges, comme "agents stabilisants aminés", en une quantité de 0,1 à 25 % d'équivalents de groupes amino, par rapport aux groupes NCO, se présentant en suspension dans des polyols de poids moléculaire élevé et/ou des polyamines aromatiques et/ou aliphatiques de poids moléculaire élevé, avec des poids moléculaires de 400 à 6000, avec addition éventuelle de polyols et/ou de polyamines aromatiques de bas poids moléculaire, en utilisant, le cas échéant, d'autres polyisocyanates A) non stabilisés, composés B) de poids moléculaire élevé et agents C) d'allongement de chaîne, le cas échéant avec addition de catalyseurs D) du type d'amines tertiaires et/ou métalliques et de substances auxiliaires E), pour la production de systèmes réactifs à un constituant pour polyuréthanne à une température d' épaississement supérieure ou égale à 55°C et on les fait durcir par la chaleur, par des forces de cisaillement et/ou par des solvants polaires en polyuréthannes compacts ou cellulaires.

21. Utilisation suivant la revendication 19, caractérisée en ce qu'on utilise des polyisocyanates stabilisés par formation d'une enveloppe de produit d'addition, obtenus par réaction de polyisocyanates libres sous une forme finement divisée à 0,1-25 % d'équivalents de groupes amino, par rapport aux groupes NCO, avec des composés difonctionnels ou polyfonctionnels de bas poids moléculaire et/ou de poids moléculaire élevé portant des groupes amino primaires et/ou secondaires à liaison aliphatique et/ou des groupes terminaux $-CO.NH.NH_2$ et/ou une ou plusieurs hydrazines, des hydrazines à substituants alkyle et/ou des N,N'-dialkylhydrazines telles que la N,N'-diméthylhydrazine, d'un poids moléculaire de 32 à 6000, ou leurs mélanges, en tant que "agents stabilisants aminés", se présentant en suspension dans des polyamines aromatiques et/ou aliphatiques de poids moléculaire élevé ayant des poids moléculaires de 400 à 6000 et, le cas échéant, des polyols et/ou des polyamines aromatiques de bas poids moléculaire, d'un poids moléculaire de 62 à 399, et, le cas échéant, de faibles quantités, par rapport aux polyamines de poids moléculaire élevé, de polyols de poids moléculaire élevé, comme composant A), B) et, le cas échéant, C), éventuellement avec addition d'autres polyisocyanates A) non stabilisés, composés B) de poids moléculaire élevé et agents C) d'allongement de chaîne, avec, le cas échéant, addition de catalyseurs du type d'amines tertiaires et/ou de catalyseurs métalliques D) et de substances auxiliaires E), pour la production de systèmes réactif s à un constituant pour polyuréthannes, avec une température d' épaississement supérieure ou égale à 55° C, et on les fait durcir par la

chaleur, par des forces de cisaillement et/ou par des solvants polaires en polyuréthannes compacts ou cellulaires.

22. Utilisation suivant la revendication 19, caractérisée en ce qu' on utilise comme composant polyisocyanate A) et composant B) le produit de réaction de polyisocyanates solides sous la forme de fines particules avec des composés difonctionnels ou polyfonctionnels de bas poids moléculaire et/ou de poids moléculaire élevé portant des groupes amino primaires et/ou secondaires à liaison aliphatique et ayant un poids moléculaire de 60 à 3000 (ou leurs mélanges), comme "agents stabilisants aminés" en une quantité de 0, 1 à 25 % en poids de groupes amino, par rapport à NCO, en suspension dans des polyols de poids moléculaire élevé ayant des poids moléculaires de 400 à 6000, le cas échéant avec addition de polyamines aromatiques de bas poids moléculaire et/ou de polyamines aromatiques de bas poids molèculaire et/ou de polyols de bas poids moléculaire avec des poids moléculaires allant jusqu'à 399, et comme composant d'allongement de chaîne C), des polyamines aromatiques de bas poids moléculaire qu'agent d'allongement de chaîne C) ajouté, éventuellement avec addition d'autres polyisocyanates A) non stabilisés, composés B) de poids moléculaire élevé et agents C) d'allongement de chaîne, avec addition éventuelle de catalyseurs du type d'amines tertiaires et/ou de catalyseurs métalliques D) et de substances auxiliaires B), pour la production de systèmes réactifs à un constituant pour polyuréthanne, avec une température d' épaississement supérieure ou égale à 55°C et on les fait durcir par la chaleur, par des forces de cisaillement et/ou par des solvants polaires en polyuréthannes compacts ou cellulaires.

23. Utilisation suivant les revendications 19 à 22, caractérisée en ce que le rapport des équivalents $NCO/(NH_2+OH)$ de $A/(B+C)$ s'élève à 0,5 : 1 à 1,5 : 1 dans la formation du polyuréthanne.

24. Utilisation suivant les revendications 19 à 23, caractérisée en ce qu'on utilise par équivalent de $(NH_2+OH)$ en polyols et/ou polyamines (B) de poids moléculaire élevé, 0,3 à 10 équivalents de $(OH+NH_2)$ en polyols et/ou en polyamines (C) de bas poids moléculaire pour la formation de polyuréthanne.

25. Utilisation suivant les revendications 19 à 24, caractérisée en ce qu' on utilise comme catalyseurs D) 0,001 à 5 % en poids, par rapport à A+B, de composés organiques de plomb et/ou d'étain dans les systèmes à un constituant.

26. Utilisation suivant les revendications 19 à 25, caractérisée en ce qu' on utilise des composés organiques de plomb de la série

a) des sels organiques du plomb divalent avec des acides dicarboxyliques,

b) de dithiocarbamates du plomb divalent de formule

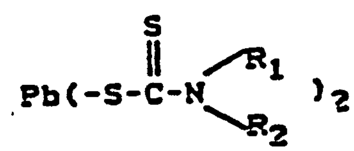

dans laquelle

$R_1$ et $R_2$ peuvent être différents et représentent un reste alkyle en $C_1$ à $C_{20}$,

c) des composés tétra-organiques du plomb-IV, le reste organique désignant alors un reste alkyle inférieur tel que, par exemple, méthyle ou éthyle et

d) des composés dérivés de composés 1,3-dicarbonyliques tels que, par exemple, l'acétylacétone, avec du plomb divalent, lorsqu'on utilise dans les systèmes à un constituant des polyéther-polyols (B) porteurs de groupes hydroxyle secondaires, et en ce qu'on ajoute des composés organiques d'étain du groupe des sels d'étain (II) d'acides carboxyliques ou des composés d' étain (IV) tels que l'oxyde de dibutylétain, le dichlorure de dibutylétain, le diacétate de dibutylétain, le dilaurate de dibutylétain, le maléate de dibutylétain ou des composés d' étain contenant du soufre de formule

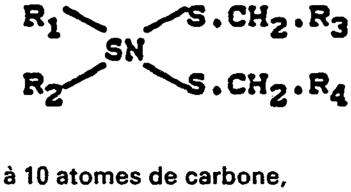

dans laquelle

$R_1$ et $R_2$ sont des restes alkyle ayant 1 à 10 atomes de carbone,

$R_3$ et $R_4$ représentent l'hydrogène et/ou des restes alkyle en $C_1$ à $C_{18}$ et/ou le reste $COOR_1$,

lorsqu'on utilise dans les systèmes à un constituant des polyols B) porteurs de groupes hydroxyle principalement primaires.

27. Utilisation suivant les revendications 19, 23 et 24, caractérisée en ce qu'on utilise des polyisocyanates à enveloppe formée d'un produit de polyaddition suivant la revendication 15.